(19) Europäisches Patentamt
European Patent Office
Office européen des brevets

(11) EP 4 346 818 B1

(12) EUROPEAN PATENT SPECIFICATION

(45) Date of publication and mention of the grant of the patent:
**03.12.2025 Bulletin 2025/49**

(21) Application number: **22741050.3**

(22) Date of filing: **03.06.2022**

(51) International Patent Classification (IPC):
***A61K 31/443*** *(2006.01)* ***A61P 23/00*** *(2006.01)*
***A61P 25/02*** *(2006.01)* ***A61P 29/00*** *(2006.01)*
***A61K 9/20*** *(2006.01)*

(52) Cooperative Patent Classification (CPC):
**A61K 9/2054; A61K 9/2013; A61K 31/443; A61P 23/00; A61P 25/02; A61P 29/00**

(86) International application number:
**PCT/US2022/032253**

(87) International publication number:
**WO 2022/256708 (08.12.2022 Gazette 2022/49)**

(54) **SOLID DOSAGE FORMS AND DOSING REGIMENS COMPRISING (2R,3S,4S,5R)-4-[[3-(3,4-DIFLUORO-2-METHOXY-PHENYL)-4,5-DIMETHYL-5-(TRIFLUOROMETHYL) TETRAHYDROFURAN-2-CARBONYL]AMINO]PYRIDINE-2-CARBOXAMIDE**

FESTE DARREICHUNGSFORMEN UND DOSIERUNGSSCHEMATA ENTHALTEND [[3-(3,4-DIFLUOR-2-METHOXY-PHENYL)-4,5-DIMETHYL-5-(TRIFLUORMETHYL) TETRAHYDROFURAN-2-CARBONYL]AMINO]PYRIDIN-2-CARBOXAMID

FORMES DE DOSAGE SOLIDES ET REGIMENS DE DOSAGE COMPRENANT LE [[3-(3,4-DIFLUORO-2-MÉTHOXY-PHÉNYL)-4,5-DIMÉTHYL-5-(TRIFLUOROMÉTHYL) TÉTRAHYDROFURANNE-2-CARBONYL]AMINO]PYRIDINE-2-CARBOXAMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**04.06.2021 US 202163196933 P**
**04.06.2021 US 202163196937 P**
**02.12.2021 US 202163285197 P**
**02.12.2021 US 202163285201 P**

(43) Date of publication of application:
**10.04.2024 Bulletin 2024/15**

(73) Proprietor: **Vertex Pharmaceuticals Incorporated**
**Boston, MA 02210 (US)**

(72) Inventors:
- **KARKARE, Radhika**
  **Boston, Massachusetts 02210 (US)**
- **CHU, Cathy**
  **Boston, MA 02210 (US)**
- **CIRINCIONE, Brenda**
  **Boston, MA 02210 (US)**
- **CORRELL, Darin J.**
  **Boston, MA 02210 (US)**
- **DELFF, Philip Kaj Harder**
  **Boston, MA 02210 (US)**
- **DINEHART, Kirk Raymond**
  **Boston, MA 02210 (US)**
- **BENITO GALLO, Paloma**
  **Boston, MA 02210 (US)**
- **HAY, Tanya Louise**
  **Boston, MA 02210 (US)**
- **JIANG, Licong**
  **Boston, MA 02210 (US)**
- **JONES, James B.**
  **Boston, MA 02210 (US)**
- **MCCARTY, Katie L.**
  **Boston, MA 02210 (US)**
- **METZLER, Catherine P.**
  **Boston, MA 02210 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MILLER, Jonathan M.**
  **Boston, MA 02210 (US)**
- **PETERSON, Mark C.**
  **Boston, MA 02210 (US)**
- **ROOPWANI, Rahul**
  **Boston, MA 02210 (US)**
- **STAROPOLI, John F.**
  **Boston, MA 02210 (US)**

(74) Representative: **Carpmaels & Ransford LLP**
**One Southampton Row**
**London WC1B 5HA (GB)**



(56) References cited:
**WO-A1-2019/030302** **WO-A1-2021/113627**
**US-A1- 2019 016 671**

- **M. F. JARVIS ET AL: "From the Cover: A-803467, a potent and selective Nav1.8 sodium channel blocker, attenuates neuropathic and inflammatory pain in the rat", PROCEEDINGS OF THE NATIONAL ACADEMY OF SCIENCES, vol. 104, no. 20, 15 May 2007 (2007-05-15), pages 8520 - 8525, XP055030736, ISSN: 0027-8424, DOI: 10.1073/pnas.0611364104**
- **BELL JACOB ET AL: "Vertex moves pain drug into mid-stage testing Dive Brief", DIVE BRIEF, 26 April 2021 (2021-04-26), pages 1 - 5, XP055962076, Retrieved from the Internet <URL:https://www.biopharmadive.com/news/vertex-pain-drug-phase-2/599025/> [retrieved on 20220919]**
- **ANONYMOUS: "History of Changes for Study: NCT05034952 A Study Evaluating Efficacy and Safety of VX-548 for Acute Pain After an Abdominoplasty", CLINICALTRIALS.GOV ARCHIVE, 27 August 2021 (2021-08-27), pages 1 - 5, XP055962070, Retrieved from the Internet <URL:https://www.clinicaltrials.gov/ct2/history/NCT05034952?V_1=View#StudyPageTop> [retrieved on 20220919]**
- **ANONYMOUS: "History of Changes for Study: NCT04977336 A Study Evaluating Efficacy and Safety of VX-548 for Acute Pain After a Bunionectomy", CLINICALTRIALS.GOV ARCHIVE, 15 July 2021 (2021-07-15), pages 1 - 6, XP055962084, Retrieved from the Internet <URL:https://clinicaltrials.gov/ct2/history/NCT04977336?V_1=View#StudyPageTop> [retrieved on 20220919]**

## Description

BACKGROUND

Pain is a protective mechanism that enables healthy persons and animals to avoid tissue damage and to prevent further damage to injured tissue. Managing pain in the clinical setting, in both acute and chronic clinical settings, remains a high unmet need. In addition to acute pain, there are many conditions where chronic pain persists beyond its protective role (neuropathic pain) where patients would benefit from inhibition of pain. Neuropathic pain is a form of chronic pain caused by an injury to the sensory nerves (Dieleman, J.P., et al., Incidence rates and treatment of neuropathic pain conditions in the general population. Pain, 2008. 137(3): p. 681-8). Neuropathic pain can be divided into two categories: pain caused by generalized metabolic damage to the nerve and pain caused by a discrete nerve injury. The metabolic neuropathies include post herpetic neuropathy, diabetic neuropathy, and drug-induced neuropathy. Discrete nerve injuries indications include post amputation pain, post-surgical nerve injury pain, and nerve entrapment injuries like neuropathic back pain. Neuropathic pain is a major cause or disability worldwide, negatively affecting patient's sleep, mood, and functionality. Clin. Ther. 2018 40(6): p. 828-49.

Current pain therapies suffer from poor efficacy and a high risk of adverse events (AEs). For example, lidocaine (a nonselective sodium channel blocker) may effectively reduce pain, but its utility is limited because of prominent side effects when given at dose levels required for pain relief. Opioid pain medications have a high abuse liability, leading to frequent deaths due to overdose. In addition, opioid-induced hyperalgesia also limits the long term use of opioids. Opioid-induced hyperalgesia is encountered regularly in clinical practice and creates significant challenges in pain management.

Antidepressants and anticonvulsants remain the first line treatment for neuropathic pain despite not being designated for such purpose. Their use is often limited by an arsenal of side effects or inadequate pain relief. Clinical development has exhibited a considerable lack of recent progress and innovation of new medications to treat both acute and chronic pain. Over the last decades, most approved analgesic drugs for the treatment of neuropathic pain either act on the serotonin-norepinephrine system (such as the serotonin-norepinephrine reuptake inhibitor duloxetine) or on voltage-gated calcium channels (such as the gabapentionid pregabalin). Given the limited treatment options for pain, combined with a growing awarenss of the risks and relative ineffectiveness of the current standards of care, the development of analgesics targeting specific pathophysiology mechanisms with improved efficacy and safety profiles is vital for better pain management and patient health outcomes.

Voltage-gated sodium channels ($Na_V$s) are involved in pain signaling. $Na_V$s are biological mediators of electrical signaling as they mediate the rapid upstroke of the action potential of many excitable cell types (e.g., neurons, skeletal myocytes, cardiac myocytes). Support for the assertion that Navs play a critical and central role in pain signaling arises from (1) evaluation of the role Navs plays in normal physiology, (2) pathological states arising from mutations in the Nav1.8 gene *(SCN10A).* (3) preclinical work in animal models, and (4) pharmacology of known Nav1.8-modulating agents. In addition, because Nav1.8 expression is restricted to peripheral neurons, particularly those that sense pain (e.g., the dorsal root ganglia), Nav1.8 inhibitors are less likely to be associated with the side effects commonly observed with other sodium channel modulators and the abuse liability associated with opioid therapies. Therefore, targeting the underlying biology of pain through selective Nav1.8 inhibition represents a novel approach to analgesic drug development that has the potential to address an urgent unmet need for safe and effective acute and chronic pain therapies (Rush, A.M. and T.R. Cummins, Painful Research: Identification of a Small-Molecule Inhibitor that Selectively Targets NaV1.8 Sodium Channels. Mol. Interv., 2007. 7(4): p. 192-5); England, S., Voltage-gated sodium channels: the search for subtype-selective analgesics. Expert Opin. Investig. Drugs 17 (12), p. 1849-64 (2008); Krafte, D. S. and Bannon, A. W., Sodium channels and nociception: recent concepts and therapeutic opportunities. Curr. Opin. Pharmacol. 8 (1), p. 50-56 (2008)). Because of the role $Na_V$s play in the initiation and propagation of neuronal signals, antagonists that reduce $Na_V$ currents can prevent or reduce neural signaling and $Na_V$ channels have been considered likely targets to reduce pain in conditions where hyper-excitability is observed (Chahine, M., Chatelier, A., Babich, O., and Krupp, J. J., Voltage-gated sodium channels in neurological disorders. CNS Neurol. Disord. Drug Targets 7 (2), p. 144-58 (2008)). Several clinically useful analgesics have been identified as inhibitors of $Na_V$ channels. The local anesthetic drugs such as lidocaine block pain by inhibiting $Na_V$ channels, and other compounds, such as carbamazepine, lamotrigine, and tricyclic antidepressants that have proven effective at reducing pain have also been suggested to act by sodium channel inhibition (Soderpalm, B., Anticonvulsants: aspects of their mechanisms of action. Eur. J. Pain 6 Suppl. A, p. 3-9 (2002); Wang, G. K., Mitchell, J., and Wang, S. Y., Block of persistent late Na+ currents by antidepressant sertraline and paroxetine. J. Membr. Biol. 222 (2), p. 79-90 (2008)).

The $Na_V$s form a subfamily of the voltage-gated ion channel super-family and comprises 9 isoforms, designated $Na_V$1.1 - $Na_V$1.9. The tissue localizations of the nine isoforms vary. $Na_V$1.4 is the primary sodium channel of skeletal muscle, and $Na_V$1.5 is the primary sodium channel of cardiac myocytes. $Na_V$ 1.7, Nav 1.8 and Nav 1.9 are primarily localized to the peripheral nervous system, while $Na_V$ 1.1, Nav 1.2, Nav 1.3, and Nav 1.6 are neuronal channels found in both the central and peripheral nervous systems. The functional behaviors of the nine isoforms are similar but distinct in the specifics of their voltage-dependent and kinetic behavior (Catterall, W. A., Goldin, A. L., and Waxman, S. G., International

Union of Pharmacology. XLVII. Nomenclature and structure-function relationships of voltage-gated sodium channels. Pharmacol. Rev. 57 (4), p. 397 (2005)).

Upon their discovery, $Na_V1.8$ channels were identified as likely targets for analgesia (Akopian, A.N., L. Sivilotti, and J.N. Wood, A tetrodotoxin-resistant voltage-gated sodium channel expressed by sensory neurons. Nature, 1996. 379(6562): p. 257-62). Since then, $Na_V1.8$ has been shown to be a carrier of the sodium current that maintains action potential firing in small DRG neurons, supporting its potential as a target for multiple indications or across multiple pain types (Blair, N.T. and B.P. Bean, Roles of tetrodotoxin (TTX)-sensitive Na+ current, TTX-resistant Na+ current, and Ca2+ current in the action potentials of nociceptive sensory neurons. J. Neurosci., 2002. 22(23): p. 10277-90). $Na_V1.8$ is involved in spontaneous firing in damaged neurons, like those that drive neuropathic pain (Roza, C., et al., The tetrodotoxin-resistant Na+ channel NaV1.8 is essential for the expression of spontaneous activity in damaged sensory axons of mice. J. Physiol., 2003. 550(Pt 3): p. 921-6; Jarvis, M.F., et al., A-803467, a potent and selective NaV1.8 sodium channel blocker, attenuates neuropathic and inflammatory pain in the rat. Proc. Natl. Acad. Sci. U S A, 2007. 104(20): p. 8520-5; Joshi, S.K., et al., Involvement of the TTX-resistant sodium channel NaV1.8 in inflammatory and neuropathic, but not post-operative, pain states. Pain, 2006. 123(1-2): pp. 75-82; Lai, J., et al., Inhibition of neuropathic pain by decreased expression of the tetrodotoxin-resistant sodium channel, NaV1.8. Pain, 2002. 95(1-2): p. 143-52; Dong, X.W., et al., Small interfering RNA-mediated selective knockdown of Na(V) 1.8 tetrodotoxin-resistant sodium channel reverses mechanical allodynia in neuropathic rats. Neuroscience, 2007. 146(2): p. 812-21; Huang, H.L., et al., Proteomic profiling of neuromas reveals alterations in protein composition and local protein synthesis in hyper-excitable nerves. Mol. Pain, 2008. 4: p. 33; Black, J.A., et al., Multiple sodium channel isoforms and mitogen-activated protein kinases are present in painful human neuromas. Ann. Neurol., 2008. 64(6): p. 644-53; Coward, K., et al., Immunolocalization of SNS/PN3 and NaN/SNS2 sodium channels in human pain states. Pain, 2000. 85(1-2): p. 41-50; Yiangou, Y., et al., SNS/PN3 and SNS2/NaN sodium channel-like immunoreactivity in human adult and neonate injured sensory nerves. FEBS Lett., 2000. 467(2-3): p. 249-52; Ruangsri, S., et al., Relationship of axonal voltage-gated sodium channel 1.8 (NaV1.8) mRNA accumulation to sciatic nerve injury-induced painful neuropathy in rats. J. Biol. Chem. 286(46): p. 39836-47). The small DRG neurons where $Na_V1.8$ is expressed include the nociceptors involved in pain signaling. $Na_V1.8$ mediates large amplitude action potentials in small neurons of the dorsal root ganglia (Blair, N.T. and B.P. Bean, Roles of tetrodotoxin (TTX)-sensitive Na+ current, TTX-resistant Na+ current, and Ca2+ current in the action potentials of nociceptive sensory neurons. J. Neurosci., 2002. 22(23): p. 10277-90). $Na_V1.8$ is necessary for rapid repetitive action potentials in nociceptors, and for spontaneous activity of damaged neurons (Choi, J.S. and S.G. Waxman, Physiological interactions between NaV1.7 and NaV1.8 sodium channels: a computer simulation study. J. Neurophysiol. 106(6): p. 3173-84; Renganathan, M., T.R. Cummins, and S.G. Waxman, Contribution of Na(V)1.8 sodium channels to action potential electrogenesis in DRG neurons. J. Neurophysiol., 2001. 86(2): p. 629-40; Roza, C., et al., The tetrodotoxin-resistant Na+ channel NaV1.8 is essential for the expression of spontaneous activity in damaged sensory axons of mice. J. Physiol., 2003. 550(Pt 3): p. 921-6). In depolarized or damaged DRG neurons, $Na_V1.8$ appears to be a driver of hyper-excitablility (Rush, A.M., et al., A single sodium channel mutation produces hyper- or hypoexcitability in different types of neurons. Proc. Natl. Acad. Sci. USA, 2006. 103(21): p. 8245-50). In some animal pain models, $Na_V1.8$ mRNA expression levels have been shown to increase in the DRG (Sun, W., et al., Reduced conduction failure of the main axon of polymodal nociceptive C-fibers contributes to painful diabetic neuropathy in rats. Brain, 135(Pt 2): p. 359-75; Strickland, I.T., et al., Changes in the expression of NaV1.7, NaV1.8 and NaV1.9 in a distinct population of dorsal root ganglia innervating the rat knee joint in a model of chronic inflammatory joint pain. Eur. J. Pain, 2008. 12(5): p. 564-72; Qiu, F., et al., Increased expression of tetrodotoxin-resistant sodium channels NaV1.8 and NaV1.9 within dorsal root ganglia in a rat model of bone cancer pain. Neurosci. Lett., 512(2): p. 61-6). Jarvis et al. disclose in Proc. Nat. Acad. Sci., 104(20), 2007, 8520, that A-803467 (which has a different structure than present Compound 1) is a potent and selective Nav1.8 sodium channel blocker, which attenuates neuropathic and inflammatory pain in the rat. US-A-2019/016671 discloses carboxamides which are inhibitors of voltage-gated sodium channels, such as Nav1.8, for use in treating various disorders, including pain.

## SUMMARY

The references to methods of treatment in this description are to be interpreted as references to the compounds, pharmaceutically acceptable salts, pharmaceutical compositions and medicaments of the present invention for use in a method for treatment of the human (or animal) body by therapy.

In one aspect, the invention relates to Compound 1 or a pharmaceutically acceptable salt thereof, for use in a method of treating or lessening the severity of pain in a subject, comprising administering to the subject a compound of formula

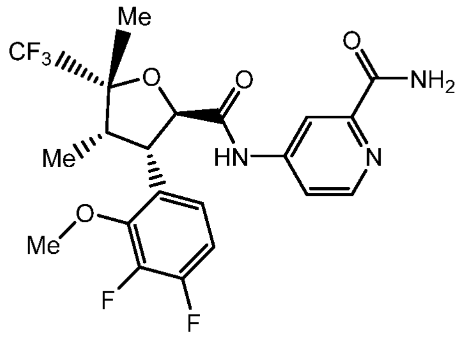

(Compound 1)

or a pharmaceutically acceptable salt thereof, in an amount of 10 mg to 300 mg per day.

In yet another aspect, the disclosure relates to a method of treating or lessening the severity in a subject of a variety of diseases, disorders, or conditions, including, but not limited to, chronic pain, gut pain, neuropathic pain, musculoskeletal pain, acute pain, inflammatory pain, cancer pain, idiopathic pain, postsurgical pain (e.g., bunionectomy pain, herniorrhaphy pain or abdominoplasty pain), visceral pain, multiple sclerosis, Charcot-Marie-Tooth syndrome, incontinence, pathological cough, and cardiac arrhythmia, by administering Compound 1, a pharmaceutically acceptable salt, or a pharmaceutical composition to the subject.

In one aspect, the invention relates to a solid dispersion, comprising Compound 1 or a pharmaceutically acceptable salt thereof, and at least one polymer. In one aspect, the invention relates to a pharmaceutical composition comprising said solid dispersion.

In one aspect, Compound 1 is substantially amorphous.

In one aspect, the disclosure relates to a pharmaceutical composition comprising Compound 1 and at least one polymer.

In another aspect, the pharmaceutical composition further comprises at least one polymer, at least one filler, at least one lubricant, and at least one disintegrant.

BRIEF DESCRIPTION OF THE DRAWINGS

Figure 1 depicts an XRPD pattern characteristic of Compound 1, Form A.
Figure 2 depicts TGA thermogram characteristic of Compound 1, Form A.
Figure 3 depicts DSC thermogram characteristic of Compound 1, Form A.
Figure 4 depicts an XRPD pattern characteristic of Compound 1, Form B.
Figure 5 depicts a solid state $^{13}C$ NMR spectrum characteristic of Compound 1, Form B.
Figure 6 depicts a solid state $^{19}F$ NMR spectrum characteristic of Compound 1, Form B.
Figure 7 depicts a TGA thermogram characteristic of Compound 1, Form B.
Figure 8 depicts a DSC thermogram characteristic of Compound 1, Form B.
Figure 9 depicts an IR spectrum characteristic of Compound 1, Form B.
Figure 10 depicts a thermal ellipsoid plot characteristic of Compound 1, Form B.
Figure 11 depicts an XRPD pattern of the spray-dried dispersion of Compound 1 of Example 3.
Figure 12A depicts an XRPD pattern of the SDD tablet composition of Example 3 over the range of about 3° to about 40°2θ.
Figure 12B depicts an XRPD pattern of the SDD tablet composition of Example 3 over the range of about 14° to about 16°2θ.
Figure 13A depicts a solid state $^{19}F$ NMR spectrum characteristic of the SDD tablet composition of Example 3.
Figure 13B depicts a solid state $^{13}C$ NMR spectrum characteristic of a powder of the SDD tablet composition of Example 3.

DETAILED DESCRIPTION

Definitions

The chemical elements are identified herein in accordance with the Periodic Table of the Elements, CAS version, Handbook of Chemistry and Physics, 75th Ed. Additionally, general principles of organic chemistry are described in "Organic Chemistry," Thomas Sorrell, University Science Books, Sausalito: 1999, and "March's Advanced Organic

Chemistry," 5th Ed., Ed.: Smith, M.B. and March, J., John Wiley & Sons, New York: 2001.

As used herein, the term "amorphous" refers to a solid material having no long range order in the position of its molecules. The moledules in an amorphous solid are generally arranged in a random manner with no well-defined arrangement. Amorphous solids are generally isotropic, i.e. exhibit similar properties in all directions and do not have definite melting points. For example, an amorphous material is a solid material having no sharp characteristic crystalline peak(s) in its X-ray power diffraction (XRPD) pattern (i.e., is not crystalline as determined by XRPD). Instead, one or several broad peaks (e.g., halos) appear in its XRPD pattern.

As used herein, the term "substantially amorphous" refers to a solid material having little or no long range order in the position of its molecules. For example, substantially amorphous materials have less than about 15% crystallinity (e.g., less than about 10% crystallinity or less than about 5% crystallinity). The term 'substantially amorphous' includes materials having no (0%) crystallinity.

As used herein, the term "dispersion" refers to a disperse system in which one substance, the dispersed phase, is distributed, in discrete units, throughout a second substance (the continuous phase or vehicle). The size of the dispersed phase can vary considerably (e.g. colloidal particles of nanometer dimension, to multiple microns in size). In general, the dispersed phases can be solids, liquids, or gases. In the case of a solid dispersion, the dispersed and continuous phases are both solids. In pharmaceutical applications, a solid dispersion can include a crystalline drug (dispersed phase) in an amorphous polymer (continuous phase); or alternatively, an amorphous drug (dispersed phase) in an amorphous polymer (continuous phase). In some embodiments, a solid dispersion includes the polymer constituting the dispersed phase, and the drug constitute the continuous phase. In other embodiments, a solid dispersion includes the drug constituting the dispersed phase, and the polymer constituting the continuous phase.

As used herein, the prefix *"rac-,"* when used in connection with a chiral compound, refers to a racemic mixture of the compound. In a compound bearing the *"rac-"* prefix, the (R)- and (S)-designators in the chemical name reflect the relative stereochemistry of the compound.

As used herein, the prefix *"rel-,"* when used in connection with a chiral compound, refers to a single enantiomer of unknown absolute configuration. In a compound bearing the *"rel-"* prefix, the (*R*)- and (*S*)- designators in the chemical name reflect the relative stereochemistry of the compound, but do not necessarily reflect the absolute stereochemistry of the compound. Where the relative stereochemistry of a given stereocenter is unknown, no stereochemical designator is provided. In some instances, the absolute configuration of some stereocenters is known, while only the relative configuration of the other stereocenters is known. In these instances, the stereochemical designators associated with the stereocenters of known absolute configuration are marked with an asterisk (*), e.g., (*R**)- and (*S**)-, while the stereochemical designators associated with stereocenters of unknown absolute configuration are not so marked. The unmarked stereochemical designators associated with the stereocenters of unknown absolute configuration reflect the relative stereochemistry of those stereocenters with respect to other stereocenters of unknown absolute configuration, but do not necessarily reflect the relative stereochemistry with respect to the stereocenters of known absolute configuration.

As used herein, the term "Compound 1," and the structure and chemical name corresponding to the "Compound 1," refer to a collection of molecules having identical chemical structures, namely the structure corresponding to the "Compound 1," except that there may be isotopic variation among the constituent atoms of the molecules. The term "Compound 1" includes such a collection of molecules without regard to the purity of a given sample containing the collection of molecules. Thus, the term "Compound 1" includes such a collection of molecules in pure form or in a mixture (e.g., solution, suspension, or colloid) with one or more other substances.

In the specification and claims, unless otherwise specified, any atom not specifically designated as a particular isotope in Compound **1** is meant to represent any stable isotope of the specified element. In the Examples, where an atom is not specifically designated as a particular isotope, no effort was made to enrich that atom in a particular isotope, and therefore a person of ordinary skill in the art would understand that such atom likely was present at approximately the natural abundance isotopic composition of the specified element.

As used herein, the term "Compound **1a"** referes to the compound with the chemical name: 2-carbamoyl-4-((2R,3S,4S,5R)-3-(3,4-difluoro-2-methoxyphenyl)-4,5-dimethyl-5-(trifluoromethyl)tetrahydrofuran-2-carboxamido)pyridine 1-oxide.

As used herein, the term "stable," when referring to an isotope, means that the isotope is not known to undergo spontaneous radioactive decay. Stable isotopes include, but are not limited to, the isotopes for which no decay mode is identified in V.S. Shirley & C.M. Lederer, Isotopes Project, Nuclear Science Division, Lawrence Berkeley Laboratory, Table of Nuclides (January 1980).

As used herein, "H" refers to hydrogen and includes any stable isotope of hydrogen, namely $^{1}H$ and D. In the Examples, where an atom is designated as "H," no effort was made to enrich that atom in a particular isotope of hydrogen and, therefore, a person of ordinary skill in the art would understand that such hydrogen atom likely was present at approximately the natural abundance isotopic composition of hydrogen.

In some embodiments, Compound **1,** or a pharmaceutically acceptable salt thereof, includes each constituent atom at approximately the natural abundance isotopic composition of the specified element.

In some embodiments, Compound **1,** or a pharmaceutically acceptable salt thereof, includes one or more atoms having an atomic mass or mass number which differs from the atomic mass or mass number of the most abundant isotope of the specified element ("isotope-labelled" compound or salt). Examples of stable isotopes which are commercially available and suitable for the invention include without limitation isotopes of hydrogen, carbon, nitrogen, oxygen, and phosphorus, for example $^{2}H$, $^{13}C$, $^{15}N$, $^{18}O$, $^{17}O$, and $^{31}P$, respectively.

The terms "Compound **1"** and "pharmaceutically acceptable salt thereof" include the Compound 1 and any pharmaceutically acceptable salt thereof in any form, including any solid form thereof (including any amorphous or crystalline form thereof), any solvate, hydrate, or cocrystal form thereof, and any solution or suspension thereof.

As used herein, the term "about" includes the value of a specified amount or a range encompassing that specified amount that is recognized by one of ordinary skill in the art to provide a pharmacological effect equivalent to that obtained from the specified amount. The term "about" may refer to an acceptable error for a particular value as determined by one of skill in the art, which depends in part on how the value is measured or determined. In some embodiments, the term "about" means within 20%, 15%, 10%, 5%, 4%, 3%, 2%, 1% or 0.5% of a given value or range. In some embodiments, the term "about" means within 20% of a given value or range. In some embodiments, the term "about" means within 15% of a given value or range. In some embodiments, the term "about" means within 10% of a given value or range. In some embodiments, the term "about" means within 5% of a given value or range. In some embodiments, the term "about" means within 1% of a given value or range. In some embodiments, the term "about" means within 0.5% of a given value or range.

As used herein, the term "subject" or "patient" means an animal, preferably a mammal, and most preferably a human.

As used herein, the term "amount," when referring to an amount of Compound 1, or a pharmaceutically acceptable salt thereof, administered to a subject, refers to the mass of an equimolar amount of (2R,3S,4S,5R)-4-[[3-(3,4-difluoro-2-methoxy-phenyl)-4,5-dimethyl-5-(trifluoromethyl) tetrahydrofuran-2-carbonyl]amino]pyridine-2-carboxamide, regardless of the actual mass of any salt, solvate, hydrate, or cocrystal form that may be administered.

In certain embodiments, an "effective amount" of Compound **1,** a pharmaceutically acceptable salt thereof, or a pharmaceutical composition thereof is that amount effective for treating or lessening the severity of one or more of the conditions recited herein.

As used herein, and unless otherwise specified, the terms "per day" and "total daily dose," when referring to an amount of Compound **1,** or a pharmaceutically acceptable salt thereof, administered to a subject, refers to the amount of Compound **1,** or a pharmaceutically acceptable salt thereof, administered to the subject on at least one 24 hour period during a course of treatment. Unless otherwise specified, it will be understood that Compound **1,** or a pharmaceutically acceptable salt thereof, may be administered to the subject in a different amount on one or more other day(s) during the course of treatment.

As used herein, the term "first day" refers to the first 24 hour period in which Compound **1,** or a pharmaceutically acceptable salt thereof, is administered during a course of treatment.

As used herein, the term "course of treatment," when referring to Compound **1,** or a pharmaceutically acceptable salt thereof, refers to the administration of one or more doses of the compound or salt during a period of time that is separate from any earlier or later administration of the compound or salt. Typically, Compound **1** and any metabolites thereof are substantially eliminated from a subject's systemic circulation between courses of treatment.

As used herein, the term "dose," when referring to the administration of Compound 1, or a pharmaceutically acceptable salt thereof, refers to an amount of the compound or salt administered in a discrete period of time, separate from other amounts of the compound or salt that may be administered at other times during the same day or course of treatment. When a dose is administered orally, the dose may be administered in a single tablet, capsule, or other oral dosage form, or in multiple such dosage forms.

As used herein, the term "first dose" refers to the first dose of Compound **1,** or a pharmaceutically acceptable salt thereof, that is administered on a given day or in a given course of treatment, as the context dictates.

As used herein, the term "subsequent dose" refers to any dose of Compound **1,** or a pharmaceutically acceptable salt thereof, that is administered after the first dose on a given day or in a given course of treatment, as the context dictates.

As used herein, the term "baseline pain score" refers to a subject's pain score, such as a score on the 11-point Numeric Pain Rating Scale or Verbal Categorical Rating Scale, prior to beginning a course of treatment with Compound **1,** or a pharmaceutically acceptable salt thereof.

As used herein, the term "11-point Numeric Pain Rating Scale" refers to a pain rating scale on which a subject rates his or her pain intensity on a scale of 0 to 10, where a score of 0 denotes no pain, and a score of 10 denotes the worst pain intensity imaginable.

As used herein, the term "Verbal Categorical Rating Scale" refers to a pain rating scale on which a subject rates his or her pain intensity as none, mild, moderate, or severe.

As used herein, the term "adverse event" is defined as any untoward medical occurrence in a subject during a

study; the event does not necessarily have a causal relationship with the treatment. This includes any newly occurring event or worsening of a pre-existing condition (e.g., increase in its severity or frequency).

As used herein, an abnormal study assessment is considered "clinically significant" if the subject has 1 or more of the following: concomitant signs or symptoms related to the abnormal study assessment, further diagnostic testing or medical/surgical intervention, a change in the dose of study drug or discontinuation from the study. The determination of whether the study assessment results are clinically significant will be made by the investigator.

Medical Uses of Compound 1 or a Pharmaceutically Acceptable Salt Thereof

In one aspect, the invention relates to Compound 1 or a pharmaceutically acceptable salt thereof, for use in a method of treating or lessening the severity of pain in a subject, comprising administering to the subject Compound 1 or a pharmaceutically acceptable salt thereof in an amount of 10 mg to 300 mg per day.

In another aspect, the disclosure relates to a use of Compound 1, or a pharmaceutically acceptable salt thereof, in a method of treating or lessening the severity of pain in a subject, comprising administering to the subject Compound 1, or the pharmaceutically acceptable salt thereof.

In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject.

*Dosing Schedules*

Compound 1, or a pharmaceutically acceptable salt thereof, may be administered in any amount appropriate to treat or lessen the severity of pain in the subject. According to the invention, Compound 1 or a pharmaceutically acceptable salt thereof is administered to the subject in an amount of 10 mg to 300 mg per day. Any amounts described herein which fall outside this range are for illustration only. In some embodiments.
Compound **1,** or a pharmaceutically acceptable salt thereof is administered in an amount of 20 mg to 5000 mg per day, or 20 mg to 4500 mg per day, or 20 mg to 4000 mg per day, or 20 mg to 3500 mg per day, or 20 mg to 3000 mg per day, or 20 mg to 2500 mg per day, or 20 mg to 2000 mg per day, or 20 mg to 1500 mg per day, or 20 mg to 1000 mg per day, or 20 mg to 800 mg per day, or 20 mg to 700 mg per day, or 20 mg to 600 mg per day, or 20 mg to 500 mg per day, or 20 mg to 450 mg per day, or 20 mg to 400 mg per day, or 20 mg to 350 mg per day, or 20 mg to 300 mg per day, or 20 mg to 250 mg per day, or 20 mg to 200 mg per day, or 20 mg to 150 mg per day, or 20 mg to 30 mg per day, or 90 mg to 120 mg per day, or 100 mg to 5000 mg per day, or 100 mg to 4500 mg per day, or 100 mg to 4000 mg per day, or 100 mg to 3500 mg per day, or 100 mg to 3000 mg per day, or 100 mg to 2500 mg per day, or 100 mg to 2000 mg per day, or 100 mg to 1500 mg per day, or 100 mg to 1000 mg per day, or 100 mg to 800 mg per day, or 100 mg to 600 mg per day, or 100 mg to 500 mg per day, or 100 mg to 400 mg per day, or 100 mg to 300 mg per day, or 100 mg to 200 mg per day, or 100 mg to 150 mg per day, or 60 mg to 2500 mg per day, or 60 mg to 2400 mg per day, or 60 mg to 2300 mg per day, or 60 mg to 2200 mg per day, or 60 mg to 2100 mg per day, or 60 mg to 2000 mg per day, or 60 mg to 1900 mg per day, or 60 mg to 1800 mg per day, or 60 mg to 1700 mg per day, or 60 mg to 1200 mg per day, or 60 mg to 800 mg per day, or about 60 mg per day, or 60 mg to 700 mg per day, or 60 mg to 600 mg per day, or 60 mg to 500 mg per day, or 60 mg to 300 mg per day, or 60 mg to 200 mg per day, or 60 mg to 150 mg per day, or 60 mg to 90 mg per day, or about 10 mg per day, or about 20 mg per day, or about 23 mg per day, or about 30 mg per day, or about 46 mg per day, or about 50 mg per day, or about 60 mg per day, or about 69 mg perday, or about 70 mg per day, or about 90 mg per day, or about 100 mg per day, or about 150 mg per day, or about 200 mg per day.

Compound **1,** or a pharmaceutically acceptable salt thereof, when administered for multiple days, may be administered in the same or different amounts each day.

In some embodiments, Compound 1, or a pharmaceutically acceptable salt thereof, is administered in different amounts on the first day and after the first day. In some embodiments, Compound 1, or a pharmaceutically acceptable salt thereof, is administered in an amount of 10 mg to 3000 mg, or 10 mg to 2000 mg, or 10 mg to 1000 mg, or 10 mg to 500 mg, or 10 mg to 400 mg, or 10 mg to 350 mg, or 10 mg to 300 mg, or 10 mg to 250 mg, or 10 mg to 200 mg, or about 100 mg, or about 150 mg, or about 120 mg, or about 90 mg, or about 30 mg on a first day and in an amount of 10 mg to 2000 mg per day, or 10 mg to 1500 mg per day, or 10 mg to 1000 mg per day, or 10 mg to 500 mg per day, or 10 mg to 400 mg per day, or 10 mg to 300 mg per day, or 20 mg to 100 mg per day, or about 20 mg per day, or about 60 mg per day, or about 100 mg per day, or about 30 mg per day, or about 20 mg per day, or about 50 mg per day after the first day.

In some embodiments, Compound **1,** or a pharmaceutically acceptable salt thereof, is administered in an amount of 100 mg, or 150 mg, or 20 mg, or about 30 mg, or about 60 mg, or about 90 mg on the first day.

In some embodiments, Compound **1,** or a pharmaceutically acceptable salt thereof, is administered in an amount of 100 mg per day, or 150 mg per day, or 50 mg per day, or 30 mg per day, or 60 mg per day, or about 10 mg per day, or about 20 mg per day after the first day.

Compound **1,** or a pharmaceutically acceptable salt thereof, may be administered in any number of doses per

day. In some embodiments, Compound **1,** or a pharmaceutically acceptable salt thereof, is administered in one dose per day. In some embodiments, Compound **1,** or a pharmaceutically acceptable salt thereof, is administered in one dose of 10 mg to 500 mg, or 10 mg to 400 mg, or 10 mg to 300 mg, or 10 mg to 260 mg, or 10 mg to 200 mg, or 10 mg to 150 mg, or 10 mg to 100 mg, or 10 mg, or 20 mg, or 30 mg per day, or 60 mg per day, or 90 mg per day, or 100 mg per day, or 120 mg per day, or 150 mg per day.

In some embodiments, Compound **1,** or a pharmaceutically acceptable salt thereof, is administered in two doses per day.

Compound **1,** or a pharmaceutically acceptable salt thereof, when administered for multiple days, may be administered in the same or different numbers of doses each day. In some embodiments, Compound **1,** or a pharmaceutically acceptable salt thereof, is administered in the same number of doses on the first day and after the first day.

In some embodiments, Compound **1,** or a pharmaceutically acceptable salt thereof, is administered in two doses on the first day (i.e., a first dose and a subsequent dose). The quantity of the first dose and the subsequent dose may be the same or different.

In some embodiments, the first dose and the subsequent dose are the same. In some embodiments, Compound **1,** or a pharmaceutically acceptable salt thereof, is administered in two doses of 10 mg to 1000 mg, or 10 mg to 500 mg, or 10 mg to 400 mg, or 10 mg to 300 mg, or 10 mg to 200 mg, or about 100 mg per day.

In some embodiments, the first dose is larger than the subsequent dose on the first day. In some embodiments, the first dose is between 5 mg and 2000 mg, or between 10 mg and 1000 mg, or between 10 mg and 200 mg, or between 10 mg and 150 mg, or between 10 mg and 100 mg, or between 20 mg and 150 mg, or between 20 mg and 100 mg, or about 250 mg, or about 200 mg, or about 150 mg, or about 100 mg, or about 90 mg, or about 60 mg, or about 30 mg, or about 20 mg, or about 10 mg.

In some embodiments, Compound **1,** or a pharmaceutically acceptable salt thereof, is administered in two doses per day after the first day (i.e., a first dose and a subsequent dose). The quantity of the first dose and the subsequent dose may be the same or different. In some embodiments, the first dose and the subsequent dose are the same after the first day.

In some embodiments, Compound 1, or a pharmaceutically acceptable salt thereof, is administered in two doses of 10 to 100 mg, or in two doses of 10 mg to 200 mg, or in two doses of 10 mg to 250 mg, or in two doses of 10 mg to 300 mg, or in two doses of 10 mg to 350 mg, or in two doses of 10 mg, or in two doses of 30 mg, or in two doses of 50 mg per day after the first day.

In some embodiments, Compound **1,** or a pharmaceutically acceptable salt thereof, is administered every 12-30 hours in a dose of about 10 mg.

In some embodiments, Compound **1,** or a pharmaceutically acceptable salt thereof, is administered every 12-30 hours in a dose of about 23 mg.

In some embodiments, Compound **1,** or a pharmaceutically acceptable salt thereof, is administered every 12-30 hours in a dose of about 30 mg.

In some embodiments, Compound **1,** or a pharmaceutically acceptable salt thereof, is administered every 12-30 hours in a dose of about 46 mg.

In some embodiments, Compound **1,** or a pharmaceutically acceptable salt thereof, is administered every 12-30 hours in a dose of about 50 mg.

In some embodiments, Compound **1,** or a pharmaceutically acceptable salt thereof, is administered every 12-30 hours in a dose of about 69 mg.

In some embodiments, Compound **1,** or a pharmaceutically acceptable salt thereof, is administered every 12-30 hours in a dose of about 70 mg.

In some embodiments, Compound **1,** or a pharmaceutically acceptable salt thereof, is administered every 24 hours in a dose of about 20 mg.

In some embodiments, Compound **1,** or a pharmaceutically acceptable salt thereof, is administered every 24 hours in a dose of about 23 mg.

In some embodiments, Compound **1,** or a pharmaceutically acceptable salt thereof, is administered every 24 hours in a dose of about 46 mg.

In some embodiments, Compound **1,** or a pharmaceutically acceptable salt thereof, is administered every 24 hours in a dose of about 60 mg.

In some embodiments, Compound **1,** or a pharmaceutically acceptable salt thereof, is administered every 24 hours in a dose of about 69 mg.

In some embodiments, Compound **1,** or a pharmaceutically acceptable salt thereof, is administered every 24 hours in a dose of about 70 mg.

In some embodiments, Compound **1,** or a pharmaceutically acceptable salt thereof, is administered every 24 hours in a dose of about 100 mg.

In some embodiments, Compound **1,** or a pharmaceutically acceptable salt thereof, is administered every 24 hours in one or more doses totaling about 100 mg.

In some embodiments, Compound **1,** or a pharmaceutically acceptable salt thereof, is administered once per day in a dose of about 10 mg.

In some embodiments, about 50 mg of Compound **1,** or a pharmaceutically acceptable salt thereof, is administered two times per day (b.i.d.).

In some embodiments, about 50 mg of Compound **1,** or a pharmaceutically acceptable salt thereof, is administered every 12 hours (q12h).

In some embodiments, Compound **1,** or a pharmaceutically acceptable salt thereof, is administered every 18-30 hours in a dose of about 100 mg.

In some embodiments, Compound **1,** or a pharmaceutically acceptable salt thereof, is administered every 21-27 hours in a dose of about 20 mg to about 150 mg.

In some embodiments, Compound **1,** or a pharmaceutically acceptable salt thereof, is administered every 21-27 hours in a dose of about 20 mg to about 100 mg.

In some embodiments, Compound **1,** or a pharmaceutically acceptable salt thereof, is administered every 21-27 hours in a dose of 23 mg, 46 mg, 50 mg, 60 mg, 69 mg, 70 mg, 75 mg, 80 mg, 85 mg, 90 mg, 95 mg, 100 mg, 105 mg, 110 mg, 115 mg, 120 mg, or 125 mg.

In some embodiments, Compound **1,** or a pharmaceutically acceptable salt thereof, is administered every 21-27 hours in a dose of 75 mg to 125 mg.

In some embodiments, Compound **1,** or a pharmaceutically acceptable salt thereof, is administered every 21-27 hours in a dose of 80 mg to 100 mg.

In some embodiments, Compound **1,** or a pharmaceutically acceptable salt thereof, is administered twice per day in a dose of about 10 mg (20 mg per day).

In some embodiments, Compound **1,** or a pharmaceutically acceptable salt thereof, is administered every 6-18 hours in a dose of about 10 mg.

In some embodiments, Compound **1,** or a pharmaceutically acceptable salt thereof, is administered every 9-15 hours in a dose of about 10 mg.

In some embodiments, Compound **1,** or a pharmaceutically acceptable salt thereof, is administered every 12 hours in a dose of about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, or about 75 mg.

In some embodiments, Compound **1,** or a pharmaceutically acceptable salt thereof, is administered every 12 hours in a dose of 10 mg, 20 mg, 30 mg, 40 mg, 50 mg, 60 mg, or 70 mg. In some embodiments, Compound 1, or a pharmaceutically acceptable salt thereof, is administered every 12 hours in a dose of 10 mg. In some embodiments, Compound **1,** or a pharmaceutically acceptable salt thereof, is administered every 12 hours in a dose of 20 mg. In some embodiments, Compound **1,** or a pharmaceutically acceptable salt thereof, is administered every 12 hours in a dose of 30 mg. In some embodiments, Compound **1,** or a pharmaceutically acceptable salt thereof, is administered every 12 hours in a dose of 40 mg. In some embodiments, Compound **1,** or a pharmaceutically acceptable salt thereof, is administered every 12 hours in a dose of 50 mg. In some embodiments, Compound **1,** or a pharmaceutically acceptable salt thereof, is administered every 12 hours in a dose of 60 mg. In some embodiments, Compound **1,** or a pharmaceutically acceptable salt thereof, is administered every 12 hours in a dose of 70 mg.

In some embodiments, Compound **1** is administered every 12 hours in a dose of about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, or about 75 mg.

In some embodiments, Compound **1** is administered every 12 hours in a dose of 10 mg, 20 mg, 30 mg, 40 mg, 50 mg, 60 mg, or 70 mg. In some embodiments, Compound **1** is administered every 12 hours in a dose of 10 mg. In some embodiments, Compound **1** is administered every 12 hours in a dose of 20 mg. In some embodiments, Compound **1** is administered every 12 hours in a dose of 30 mg. In some embodiments, Compound **1** is administered every 12 hours in a dose of 40 mg. In some embodiments, Compound **1** is administered every 12 hours in a dose of 50 mg. In some embodiments, Compound **1** is administered every 12 hours in a dose of 60 mg. In some embodiments, Compound **1** is administered every 12 hours in a dose of 70 mg.

In some embodiments, Compound **1,** or a pharmaceutically acceptable salt thereof, is administered every 12 hours in a dose of about 10 mg.

In some embodiments, Compound **1,** or a pharmaceutically acceptable salt thereof, is administered twice per day in a dose of about 30 mg (60 mg per day).

In some embodiments, Compound **1,** or a pharmaceutically acceptable salt thereof, is administered every 6-18 hours in a dose of about 30 mg.

In some embodiments, Compound **1,** or a pharmaceutically acceptable salt thereof, is administered every 9-15 hours in a dose of about 30 mg.

In some embodiments, Compound **1,** or a pharmaceutically acceptable salt thereof, is administered every 12 hours (q12h) in a dose of about 50 mg.

In some embodiments, Compound **1,** or a pharmaceutically acceptable salt thereof, is administered every 6-18 hours after a first dose of about 20 mg and a subsequent dose of about 10 mg.

In some embodiments, Compound **1,** or a pharmaceutically acceptable salt thereof, is administered every 9-15 hours after a first dose of about 20 mg and a subsequent dose of about 10 mg.

In some embodiments, Compound **1,** or a pharmaceutically acceptable salt thereof, is administered every 12 hours after a first dose of about 20 mg and a subsequent dose of about 10 mg.

In some embodiments, Compound **1,** or a pharmaceutically acceptable salt thereof, is administered in a dose of about 10 mg every 12 hours after a first dose of about 20 mg and a subsequent dose of about 10 mg.

In some embodiments, Compound **1,** or a pharmaceutically acceptable salt thereof, is administered in a dose of 10 mg every 12 hours after a first dose of 20 mg and a subsequent dose of 10 mg.

In some embodiments, Compound **1** is administered in a dose of about 10 mg every 12 hours after a first dose of about 20 mg and a subsequent dose of about 10 mg.

In some embodiments, Compound **1** is administered in a dose of 10 mg every 12 hours after a first dose of 20 mg and a subsequent dose of 10 mg.

In some embodiments, Compound **1,** or a pharmaceutically acceptable salt thereof, is administered in a dose of about 10 mg every 12 hours after a first dose of about 20 mg and a subsequent dose of about 10 mg, wherein the subsequent dose is administered 12 hours after the first dose.

In some embodiments, Compound **1,** or a pharmaceutically acceptable salt thereof, is administered in a dose of 10 mg every 12 hours after a first dose of 20 mg and a subsequent dose of 10 mg, wherein the subsequent dose is administered 12 hours after the first dose.

In some embodiments, Compound **1** is administered in a dose of 10 mg every 12 hours after a first dose of about 20 mg and a subsequent dose of about 10 mg, wherein the subsequent dose is administered 12 hours after the first dose.

In some embodiments, Compound **1** is administered in a dose of 10 mg every 12 hours after a first dose of 20 mg and a subsequent dose of 10 mg, wherein the subsequent dose is administered 12 hours after the first dose.

In some embodiments, Compound **1,** or a pharmaceutically acceptable salt thereof, is administered in a dose of about 20 mg every 12 hours after a first dose of about 40 mg and a subsequent dose of about 20 mg.

In some embodiments, Compound **1,** or a pharmaceutically acceptable salt thereof, is administered in a dose of 20 mg every 12 hours after a first dose of 40 mg and a subsequent dose of 20 mg.

In some embodiments, Compound **1** is administered in a dose of about 20 mg every 12 hours after a first dose of about 40 mg and a subsequent dose of about 20 mg.

In some embodiments, Compound 1 is administered in a dose of 20 mg every 12 hours after a first dose of 40 mg and a subsequent dose of 20 mg.

In some embodiments, Compound **1,** or a pharmaceutically acceptable salt thereof, is administered in a dose of about 20 mg every 12 hours after a first dose of about 40 mg and a subsequent dose of about 20 mg, wherein the subsequent dose is administered 12 hours after the first dose.

In some embodiments, Compound **1,** or a pharmaceutically acceptable salt thereof, is administered in a dose of 20 mg every 12 hours after a first dose of 40 mg and a subsequent dose of 20 mg, wherein the subsequent dose is administered 12 hours after the first dose.

In some embodiments, Compound **1** is administered in a dose of about 20 mg every 12 hours after a first dose of about 40 mg and a subsequent dose of about 20 mg, wherein the subsequent dose is administered 12 hours after the first dose.

In some embodiments, Compound **1** is administered in a dose of 20 mg every 12 hours after a first dose of 40 mg and a subsequent dose of 20 mg, wherein the subsequent dose is administered 12 hours after the first dose.

In some embodiments, Compound **1,** or a pharmaceutically acceptable salt thereof, is administered every 6-18 hours after a first dose of about 60 mg and a subsequent dose of about 30 mg.

In some embodiments, Compound **1,** or a pharmaceutically acceptable salt thereof, is administered every 9-15 hours after a first dose of about 60 mg and a subsequent dose of about 30 mg.

In some embodiments, Compound **1,** or a pharmaceutically acceptable salt thereof, is administered every 12 hours after a first dose of about 60 mg and a subsequent dose of about 30 mg.

In some embodiments, Compound **1,** or a pharmaceutically acceptable salt thereof, is administered in a dose of about 30 mg every 12 hours after a first dose of about 60 mg and a subsequent dose of about 30 mg.

In some embodiments, Compound **1,** or a pharmaceutically acceptable salt thereof, is administered in a dose of 30 mg every 12 hours after a first dose of 60 mg and a subsequent dose of 30 mg.

In some embodiments, Compound **1** is administered in a dose of about 30 mg every 12 hours after a first dose of about 60 mg and a subsequent dose of about 30 mg.

In some embodiments, Compound **1** is administered in a dose of 30 mg every 12 hours after a first dose of 60 mg and a subsequent dose of 30 mg.

In some embodiments, Compound **1,** or a pharmaceutically acceptable salt thereof, is administered in a dose of

about 30 mg every 12 hours after a first dose of about 60 mg and a subsequent dose of about 30 mg, wherein the subsequent dose is administered 12 hours after the first dose.

In some embodiments, Compound **1,** or a pharmaceutically acceptable salt thereof, is administered in a dose of 30 mg every 12 hours after a first dose of 60 mg and a subsequent dose of 30 mg, wherein the subsequent dose is administered 12 hours after the first dose.

In some embodiments, Compound **1** is administered in a dose of about 30 mg every 12 hours after a first dose of about 60 mg and a subsequent dose of about 30 mg, wherein the subsequent dose is administered 12 hours after the first dose.

In some embodiments, Compound **1** is administered in a dose of 30 mg every 12 hours after a first dose of 60 mg and a subsequent dose of 30 mg, wherein the subsequent dose is administered 12 hours after the first dose.

In some embodiments, Compound **1,** or a pharmaceutically acceptable salt thereof, is administered in a dose of about 40 mg every 12 hours after a first dose of about 80 mg and a subsequent dose of about 40 mg.

In some embodiments, Compound **1,** or a pharmaceutically acceptable salt thereof, is administered in a dose of 40 mg every 12 hours after a first dose of 80 mg and a subsequent dose of 40 mg.

In some embodiments, Compound **1** is administered in a dose of about 40 mg every 12 hours after a first dose of about 80 mg and a subsequent dose of about 40 mg.

In some embodiments, Compound **1** is administered in a dose of 40 mg every 12 hours after a first dose of 80 mg and a subsequent dose of 40 mg.

In some embodiments, Compound **1,** or a pharmaceutically acceptable salt thereof, is administered in a dose of about 40 mg every 12 hours after a first dose of about 80 mg and a subsequent dose of about 40 mg, wherein the subsequent dose is administered 12 hours after the first dose.

In some embodiments, Compound **1,** or a pharmaceutically acceptable salt thereof, is administered in a dose of 40 mg every 12 hours after a first dose of 80 mg and a subsequent dose of 40 mg, wherein the subsequent dose is administered 12 hours after the first dose.

In some embodiments, Compound **1** is administered in a dose of about 40 mg every 12 hours after a first dose of about 80 mg and a subsequent dose of about 40 mg, wherein the subsequent dose is administered 12 hours after the first dose.

In some embodiments, Compound **1** is administered in a dose of 40 mg every 12 hours after a first dose of 80 mg and a subsequent dose of 40 mg, wherein the subsequent dose is administered 12 hours after the first dose.

In some embodiments, Compound **1,** or a pharmaceutically acceptable salt thereof, is administered every 6-18 hours after a first dose of about 100 mg and a subsequent dose of about 50 mg.

In some embodiments, Compound **1,** or a pharmaceutically acceptable salt thereof, is administered every 9-15 hours after a first dose of about 100 mg and a subsequent dose of about 50 mg.

In some embodiments, Compound **1,** or a pharmaceutically acceptable salt thereof, is administered every 12 hours after a first dose of about 100 mg and a subsequent dose of about 50 mg.

In some embodiments, Compound **1,** or a pharmaceutically acceptable salt thereof, is administered in a dose of about 50 mg every 12 hours after a first dose of about 100 mg and a subsequent dose of about 50 mg.

In some embodiments, Compound **1,** or a pharmaceutically acceptable salt thereof, is administered in a dose of 50 mg every 12 hours after a first dose of 100 mg and a subsequent dose of 50 mg.

In some embodiments, Compound **1** is administered in a dose of about 50 mg every 12 hours after a first dose of about 100 mg and a subsequent dose of about 50 mg.

In some embodiments, Compound **1** is administered in a dose of 50 mg every 12 hours after a first dose of 100 mg and a subsequent dose of 50 mg.

In some embodiments, Compound **1,** or a pharmaceutically acceptable salt thereof, is administered in a dose of about 50 mg every 12 hours after a first dose of about 100 mg and a subsequent dose of about 50 mg, wherein the subsequent dose is administered 12 hours after the first dose.

In some embodiments, Compound **1,** or a pharmaceutically acceptable salt thereof, is administered in a dose of 50 mg every 12 hours after a first dose of 100 mg and a subsequent dose of 50 mg, wherein the subsequent dose is administered 12 hours after the first dose.

In some embodiments, Compound **1** is administered in a dose of about 50 mg every 12 hours after a first dose of about 100 mg and a subsequent dose of about 50 mg, wherein the subsequent dose is administered 12 hours after the first dose.

In some embodiments, Compound **1** is administered in a dose of 50 mg every 12 hours after a first dose of 100 mg and a subsequent dose of 50 mg, wherein the subsequent dose is administered 12 hours after the first dose.

In some embodiments, Compound **1,** or a pharmaceutically acceptable salt thereof, is administered in a dose of about 60 mg every 12 hours after a first dose of about 120 mg and a subsequent dose of about 60 mg.

In some embodiments, Compound **1,** or a pharmaceutically acceptable salt thereof, is administered in a dose of 60 mg every 12 hours after a first dose of 120 mg and a subsequent dose of 60 mg.

In some embodiments, Compound **1** is administered in a dose of about 60 mg every 12 hours after a first dose of about 120 mg and a subsequent dose of about 60 mg.

In some embodiments, Compound **1** is administered in a dose of 60 mg every 12 hours after a first dose of 120 mg and a subsequent dose of 60 mg.

In some embodiments, Compound **1,** or a pharmaceutically acceptable salt thereof, is administered in a dose of about 60 mg every 12 hours after a first dose of about 120 mg and a subsequent dose of about 60 mg, wherein the subsequent dose is administered 12 hours after the first dose.

In some embodiments, Compound **1,** or a pharmaceutically acceptable salt thereof, is administered in a dose of 60 mg every 12 hours after a first dose of 120 mg and a subsequent dose of 60 mg, wherein the subsequent dose is administered 12 hours after the first dose.

In some embodiments, Compound **1** is administered in a dose of about 60 mg every 12 hours after a first dose of about 120 mg and a subsequent dose of about 60 mg, wherein the subsequent dose is administered 12 hours after the first dose.

In some embodiments, Compound **1** is administered in a dose of 60 mg every 12 hours after a first dose of 120 mg and a subsequent dose of 60 mg, wherein the subsequent dose is administered 12 hours after the first dose.

In some embodiments, Compound **1,** or a pharmaceutically acceptable salt thereof, is administered in a dose of about 70 mg every 12 hours after a first dose of about 140 mg and a subsequent dose of about 70 mg.

In some embodiments, Compound **1,** or a pharmaceutically acceptable salt thereof, is administered in a dose of 70 mg every 12 hours after a first dose of 140 mg and a subsequent dose of 70 mg.

In some embodiments, Compound **1** is administered in a dose of about 70 mg every 12 hours after a first dose of about 140 mg and a subsequent dose of about 70 mg.

In some embodiments, Compound **1** is administered in a dose of 70 mg every 12 hours after a first dose of 140 mg and a subsequent dose of 70 mg.

In some embodiments, Compound **1,** or a pharmaceutically acceptable salt thereof, is administered in a dose of about 70 mg every 12 hours after a first dose of about 140 mg and a subsequent dose of about 70 mg, wherein the subsequent dose is administered 12 hours after the first dose.

In some embodiments, Compound **1,** or a pharmaceutically acceptable salt thereof, is administered in a dose of 70 mg every 12 hours after a first dose of 140 mg and a subsequent dose of 70 mg, wherein the subsequent dose is administered 12 hours after the first dose.

In some embodiments, Compound **1** is administered in a dose of about 70 mg every 12 hours after a first dose of about 140 mg and a subsequent dose of about 70 mg, wherein the subsequent dose is administered 12 hours after the first dose.

In some embodiments, Compound **1** is administered in a dose of 70 mg every 12 hours after a first dose of 140 mg and a subsequent dose of 70 mg, wherein the subsequent dose is administered 12 hours after the first dose.

In some embodiments, Compound **1,** or a pharmaceutically acceptable salt thereof, is administered once per day in a dose of about 10 mg to about 100 mg, or at least one week, at least two weeks, at least three weeks, at least four weeks, at least five weeks, or at least six weeks, or from one to six weeks.

In some embodiments, Compound **1,** or a pharmaceutically acceptable salt thereof, is administered every 18-30 hours in a dose of about 20 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 18-30 hours in a dose of about 23 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 18-30 hours in a dose of about 46 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 18-30 hours in a dose of about 50 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 18-30 hours in a dose of about 69 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 18-30 hours in a dose of about 70 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 21-27 hours in a dose of about 100 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 24 hours in a dose of about 100 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered once per day in a dose of about 50 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 18-30 hours in a dose of about 50 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered every 21-27 hours in a dose of about 50 mg, for at least one week, at least two weeks, at least three weeks, at least four weeks, at least five weeks, or at least six weeks, or from one to six weeks.

In some embodiments, Compound **1,** or a pharmaceutically acceptable salt thereof, is administered in a dose of 50 mg twice a day after a first dose of 100 mg and a subsequent dose of 50 mg, wherein the subsequent dose is administered about 12 hours after the first dose.

In some embodiments, Compound **1,** or a pharmaceutically acceptable salt thereof, is administered in a dose of 30 mg twice a day after a first dose of 60 mg and a subsequent dose of 30 mg, wherein the subsequent dose is administered about 12 hours after the first dose.

In some embodiments, Compound **1,** or a pharmaceutically acceptable salt thereof, is administered in a dose of 10 mg twice a day after a first dose of 20 mg and a subsequent dose of 10 mg, wherein the subsequent dose is administered about 12 hours after the first dose.

In some embodiments, Compound **1,** or a pharmaceutically acceptable salt thereof, is administered in a dose of 30 mg twice a day after a first dose of 90 mg and a subsequent dose of 30 mg, wherein the subsequent dose is administered about 12 hours after the first dose.

In some embodiments, Compound **1,** or a pharmaceutically acceptable salt thereof, is administered every 24 hours (q24h) in a dose of about 10 mg, about 15 mg, about 20 mg, about 23 mg, about 25 mg, about 30 mg, about 35 mg, about 40 mg, about 43 mg, about 45 mg, about 50 mg, about 55 mg, about 60 mg, about 65 mg, about 69 mg, about 70 mg, about 75 mg, or about 80 mg. In some embodiments, a dose of about 10 mg, about 15 mg, about 20 mg, about 23 mg, about 25 mg, about 30 mg, about 35 mg, about 40 mg, about 43 mg, about 45 mg, about 50 mg, about 55 mg, about 60 mg, about 65 mg, about 69 mg, about 70 mg, about 75 mg, or about 80 mg of Compound **1,** or a pharmaceutically acceptable salt thereof, is administered once per day (qd). In some embodiments, Compound **1,** or a pharmaceutically acceptable salt thereof, is administered every 24 hours (q24h) in a dose of 10 mg, 15 mg, 20 mg, 23 mg, 25 mg, 30 mg, 35 mg, 40 mg, 43 mg, 45 mg, 50 mg, 55 mg, 60 mg, 65 mg, 69 mg, 70 mg, 75 mg, or 80 mg. In some embodiments, a dose of 10 mg, 15 mg, 20 mg, 23 mg, 25 mg, 30 mg, 35 mg, 40 mg, 43 mg, 45 mg, 50 mg, 55 mg, 60 mg, 65 mg, 69 mg, 70 mg, 75 mg, or 80 mg of Compound **1,** or a pharmaceutically acceptable salt thereof, is administered once per day (qd).

In some embodiments, Compound **1** is administered every 24 hours (q24h) in a dose of about 10 mg, about 15 mg, about 20 mg, about 23 mg, about 25 mg, about 30 mg, about 35 mg, about 40 mg, about 43 mg, about 45 mg, about 50 mg, about 55 mg, about 60 mg, about 65 mg, about 69 mg, about 70 mg, about 75 mg, or about 80 mg. In some embodiments, a dose of about 10 mg, about 15 mg, about 20 mg, about 23 mg, about 25 mg, about 30 mg, about 35 mg, about 40 mg, about 43 mg, about 45 mg, about 50 mg, about 55 mg, about 60 mg, about 65 mg, about 69 mg, about 70 mg, about 75 mg, or about 80 mg of Compound 1 is administered once per day (qd). In some embodiments, Compound 1 is administered every 24 hours (q24h) in a dose of 10 mg, 15 mg, 20 mg, 23 mg, 25 mg, 30 mg, 35 mg, 40 mg, 43 mg, 45 mg, 50 mg, 55 mg, 60 mg, 65 mg, 69 mg, 70 mg, 75 mg, or 80 mg. In some embodiments, a dose of 10 mg, 15 mg, 20 mg, 23 mg, 25 mg, 30 mg, 35 mg, 40 mg, 43 mg, 45 mg, 50 mg, 55 mg, 60 mg, 65 mg, 69 mg, 70 mg, 75 mg, or 80 mg of Compound 1 is administered once per day (qd).

Compound 1, or a pharmaceutically acceptable salt thereof, may be administered in any form, including any solid form (including any amorphous or crystalline form), any solvate, hydrate, or cocrystal form, or any solution or suspension of the compound, or a pharmaceutically acceptable salt thereof. In some embodiments, Compound 1 is administered in Form B. In some embodiments, Compound 1 is administered in a pharmaceutical composition prepared by mixing Form B with a pharmaceutically acceptable carrier, adjuvant, or vehicle. In some embodiments, Form B is characterized by an X-ray powder diffraction pattern (XRPD) comprising at least three approximate peak positions (degrees 2 theta + 0.2) when measured using Cu $K_\alpha$ radiation, selected from the group consisting of 4.4, 15.2, 16.4, 18.0, 19.1, 19.3, 19.9, 20.2, 20.5, 21.0, 22.2, 23.5 24.2, 24.8, 26.3, 29.6, 30.1 and 31.3, when the XRPD is collected from about 4 to about 40 degrees 2 theta (2 $\theta$). In some embodiments, Form B is characterized by an X-ray powder diffraction pattern (XRPD) comprising at least three approximate peak positions (degrees 2 theta $\pm$ 0.2) when measured using Cu $K_\alpha$ radiation, selected from the group consisting of 19.3, 22.2, 23.5, 26.3 and 30.1, when the XRPD is collected from about 4 to about 40 degrees 2 theta (2 $\theta$). In some embodiments, Form B is characterized by an X-ray powder diffraction pattern (XRPD), measured using Cu $K_\alpha$ radiation, substantially similar to Figure 1.

In some embodiments, the method comprises administering to the subject Compound 1 in non-salt form (i.e., as the free acid (2R,3S,4S,5R)-4-[[3-(3,4-difluoro-2-methoxy-phenyl)-4,5-dimethyl-5-(trifluoromethyl)tetrahydrofuran-2-carbonyl]amino]pyridine-2-carboxamide).

Compound 1, or a pharmaceutically acceptable salt thereof, may be administered by any route known in the art. In some embodiments, Compound 1, or a pharmaceutically acceptable salt thereof, is administered orally.

Compound 1, or a pharmaceutically acceptable salt thereof, may be administered for any number of days necessary or desirable to treat or lessen the severity of the subject's pain, which may depend on the type of pain experienced by the subject. In some embodiments, Compound 1, or a pharmaceutically acceptable salt thereof, is administered for at least two days. In some embodiments, Compound 1, or a pharmaceutically acceptable salt thereof, is administered for at least one week, at least two weeks, at least three weeks, at least four weeks, at least five weeks, or at least six weeks, or from one to six weeks.

In another aspect, the disclosure relates to a method of treating or lessening the severity of pain in a subject comprising administering Compound 1, or a pharmaceutically acceptable salt thereof, to the subject once per day in a dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a method of treating or lessening the severity of pain in a subject comprising administering Compound 1, or a pharmaceutically acceptable salt thereof, to the subject once per day in a dose of about 23 mg. In another aspect, the disclosure relates to a method of treating or lessening the severity of pain in a subject comprising administering Compound 1, or a pharmaceutically acceptable salt thereof, to the subject once per day in a dose of about 45 mg. In another aspect, the disclosure relates to a method of treating or lessening the severity of pain in a subject comprising administering Compound 1, or a pharmaceutically acceptable salt thereof, to the subject once per day in a dose of about 46 mg. In another aspect, the disclosure relates to a method of treating or lessening the severity of pain in a subject comprising administering Compound 1, or a pharmaceutically acceptable salt thereof, to the subject once per day in a dose of about 50 mg. In another aspect, the disclosure relates to a method of treating or lessening the severity of pain in a subject comprising administering Compound 1, or a pharmaceutically acceptable salt thereof, to the subject once per day in a dose of about 60 mg. In another aspect, the disclosure relates to a method of treating or lessening the severity of pain in a subject comprising administering Compound 1, or a pharmaceutically acceptable salt thereof, to the subject once per day in a dose of about 69 mg. In another aspect, the disclosure relates to a method of treating or lessening the severity of pain in a subject comprising administering Compound 1, or a pharmaceutically acceptable salt thereof, to the subject once per day in a dose of about 70 mg. In another aspect, the disclosure relates to a method of treating or lessening the severity of pain in a subject comprising administering Compound 1, or a pharmaceutically acceptable salt thereof, to the subject once per day in a dose of about 75 mg. In another aspect, the disclosure relates to a method of treating or lessening the severity of pain in a subject comprising administering Compound 1, or a pharmaceutically acceptable salt thereof, to the subject once per day in a dose of about 90 mg. In another aspect, the disclosure relates to a method of treating or lessening the severity of pain in a subject comprising administering Compound 1, or a pharmaceutically acceptable salt thereof, to the subject once per day in a dose of about 100 mg. In another aspect, the disclosure relates to a method of treating or lessening the severity of pain in a subject comprising administering Compound 1, or a pharmaceutically acceptable salt thereof, to the subject once per day in a dose of about 110 mg. In another aspect, the disclosure relates to a method of treating or lessening the severity of pain in a subject comprising administering Compound 1, or a pharmaceutically acceptable salt thereof, to the subject once per day in a dose of about 125 mg.

In another aspect, the disclosure relates to a method of treating or lessening the severity of pain in a subject comprising administering Compound 1, or a pharmaceutically acceptable salt thereof, to the subject once per day in a dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a method of treating or lessening the severity of pain in a subject comprising administering Compound 1, or a pharmaceutically acceptable salt thereof, to the subject once per day in a dose of 23 mg. In another aspect, the disclosure relates to a method of treating or lessening the severity of pain in a subject comprising administering Compound 1, or a pharmaceutically acceptable salt thereof, to the subject once per day in a dose of 45 mg. In another aspect, the disclosure relates to a method of treating or lessening the severity of pain in a subject comprising administering Compound 1, or a pharmaceutically acceptable salt thereof, to the subject once per day in a dose of 46 mg. In another aspect, the disclosure relates to a method of treating or lessening the severity of pain in a subject comprising administering Compound 1, or a pharmaceutically acceptable salt thereof, to the subject once per day in a dose of 50 mg. In another aspect, the disclosure relates to a method of treating or lessening the severity of pain in a subject comprising administering Compound 1, or a pharmaceutically acceptable salt thereof, to the subject once per day in a dose of 60 mg. In another aspect, the disclosure relates to a method of treating or lessening the severity of pain in a subject comprising administering Compound 1, or a pharmaceutically acceptable salt thereof, to the subject once per day in a dose of 69 mg. In another aspect, the disclosure relates to a method of treating or lessening the severity of pain in a subject comprising administering Compound 1, or a

pharmaceutically acceptable salt thereof, to the subject once per day in a dose of 70 mg. In another aspect, the disclosure relates to a method of treating or lessening the severity of pain in a subject comprising administering Compound 1, or a pharmaceutically acceptable salt thereof, to the subject once per day in a dose of 75 mg. In another aspect, the disclosure relates to a method of treating or lessening the severity of pain in a subject comprising administering Compound 1, or a pharmaceutically acceptable salt thereof, to the subject once per day in a dose of 90 mg. In another aspect, the disclosure relates to a method of treating or lessening the severity of pain in a subject comprising administering Compound 1, or a pharmaceutically acceptable salt thereof, to the subject once per day in a dose of 100 mg. In another aspect, the disclosure relates to a method of treating or lessening the severity of pain in a subject comprising administering Compound 1, or a pharmaceutically acceptable salt thereof, to the subject once per day in a dose of 110 mg. In another aspect, the disclosure relates to a method of treating or lessening the severity of pain in a subject comprising administering Compound 1, or a pharmaceutically acceptable salt thereof, to the subject once per day in a dose of 125 mg.

In another aspect, the disclosure relates to a method of treating or lessening the severity of pain in a subject comprising administering Compound 1 to the subject once per day in a dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a method of treating or lessening the severity of pain in a subject comprising administering Compound 1 to the subject once per day in a dose of about 23 mg. In another aspect, the disclosure relates to a method of treating or lessening the severity of pain in a subject comprising administering Compound 1 to the subject once per day in a dose of about 45 mg. In another aspect, the disclosure relates to a method of treating or lessening the severity of pain in a subject comprising administering Compound 1 to the subject once per day in a dose of about 46 mg. In another aspect, the disclosure relates to a method of treating or lessening the severity of pain in a subject comprising administering Compound 1 to the subject once per day in a dose of about 50 mg. In another aspect, the disclosure relates to a method of treating or lessening the severity of pain in a subject comprising administering Compound 1 to the subject once per day in a dose of about 60 mg. In another aspect, the disclosure relates to a method of treating or lessening the severity of pain in a subject comprising administering Compound 1 to the subject once per day in a dose of about 69 mg. In another aspect, the disclosure relates to a method of treating or lessening the severity of pain in a subject comprising administering Compound 1 to the subject once per day in a dose of about 70 mg. In another aspect, the disclosure relates to a method of treating or lessening the severity of pain in a subject comprising administering Compound 1 to the subject once per day in a dose of about 75 mg. In another aspect, the disclosure relates to a method of treating or lessening the severity of pain in a subject comprising administering Compound 1 to the subject once per day in a dose of about 90 mg In another aspect, the disclosure relates to a method of treating or lessening the severity of pain in a subject comprising administering Compound 1 to the subject once per day in a dose of about 100 mg. In another aspect, the disclosure relates to a method of treating or lessening the severity of pain in a subject comprising administering Compound 1 to the subject once per day in a dose of about 110 mg. In another aspect, the disclosure relates to a method of treating or lessening the severity of pain in a subject comprising administering Compound 1 to the subject once per day in a dose of about 125 mg.

In another aspect, the disclosure relates to a method of treating or lessening the severity of pain in a subject comprising administering Compound 1 to the subject once per day in a dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a method of treating or lessening the severity of pain in a subject comprising administering Compound **1** to the subject once per day in a dose of 23 mg. In another aspect, the disclosure relates to a method of treating or lessening the severity of pain in a subject comprising administering Compound **1** to the subject once per day in a dose of 45 mg. In another aspect, the disclosure relates to a method of treating or lessening the severity of pain in a subject comprising administering Compound **1** to the subject once per day in a dose of 46 mg. In another aspect, the disclosure relates to a method of treating or lessening the severity of pain in a subject comprising administering Compound **1** to the subject once per day in a dose of 50 mg. In another aspect, the disclosure relates to a method of treating or lessening the severity of pain in a subject comprising administering Compound **1** to the subject once per day in a dose of 60 mg. In another aspect, the disclosure relates to a method of treating or lessening the severity of pain in a subject comprising administering Compound **1** to the subject once per day in a dose of 69 mg. In another aspect, the disclosure relates to a method of treating or lessening the severity of pain in a subject comprising administering Compound **1** to the subject once per day in a dose of 70 mg. In another aspect, the disclosure relates to a method of treating or lessening the severity of pain in a subject comprising administering Compound **1** to the subject once per day in a dose of 75 mg. In another aspect, the disclosure relates to a method of treating or lessening the severity of pain in a subject comprising administering Compound **1** to the subject once per day in a dose of 90 mg. In another aspect, the disclosure relates to a method of treating or lessening the severity of pain in a subject comprising administering Compound **1** to the subject once per day in a dose of 100 mg. In another aspect, the disclosure relates to a method of treating or lessening the severity of pain in a subject comprising administering Compound **1** to the subject once per day in a dose of 110 mg. In another aspect, the disclosure relates to a method of treating or lessening the severity of pain in a subject

comprising administering Compound **1** to the subject once per day in a dose of 125 mg.

In another aspect, the disclosure relates to a method of treating or lessening the severity of pain in a subject comprising administering Compound **1,** or a pharmaceutically acceptable salt thereof, to the subject in a total daily dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a method of treating or lessening the severity of pain in a subject comprising administering Compound **1,** or a pharmaceutically acceptable salt thereof, to the subject in a total daily dose of about 23 mg. In another aspect, the disclosure relates to a method of treating or lessening the severity of pain in a subject comprising administering Compound 1, or a pharmaceutically acceptable salt thereof, to the subject in a total daily dose of about 45 mg. In another aspect, the disclosure relates to a method of treating or lessening the severity of pain in a subject comprising administering Compound **1,** or a pharmaceutically acceptable salt thereof, to the subject in a total daily dose of about 46 mg. In another aspect, the disclosure relates to a method of treating or lessening the severity of pain in a subject comprising administering Compound **1,** or a pharmaceutically acceptable salt thereof, to the subject in a total daily dose of about 50 mg. In another aspect, the disclosure relates to a method of treating or lessening the severity of pain in a subject comprising administering Compound 1, or a pharmaceutically acceptable salt thereof, to the subject in a total daily dose of about 60 mg. In another aspect, the disclosure relates to a method of treating or lessening the severity of pain in a subject comprising administering Compound 1, or a pharmaceutically acceptable salt thereof, to the subject in a total daily dose of about 69 mg. In another aspect, the disclosure relates to a method of treating or lessening the severity of pain in a subject comprising administering Compound 1, or a pharmaceutically acceptable salt thereof, to the subject in a total daily dose of about 70 mg. In another aspect, the disclosure relates to a method of treating or lessening the severity of pain in a subject comprising administering Compound 1, or a pharmaceutically acceptable salt thereof, to the subject in a total daily dose of about 75 mg. In another aspect, the disclosure relates to a method of treating or lessening the severity of pain in a subject comprising administering Compound 1, or a pharmaceutically acceptable salt thereof, to the subject in a total daily dose of about 90 mg. In another aspect, the disclosure relates to a method of treating or lessening the severity of pain in a subject comprising administering Compound 1, or a pharmaceutically acceptable salt thereof, to the subject in a total daily dose of about 100 mg. In another aspect, the disclosure relates to a method of treating or lessening the severity of pain in a subject comprising administering Compound 1, or a pharmaceutically acceptable salt thereof, to the subject in a total daily dose of about 110 mg. In another aspect, the disclosure relates to a method of treating or lessening the severity of pain in a subject comprising administering Compound 1, or a pharmaceutically acceptable salt thereof, to the subject in a total daily dose of about 125 mg.

In another aspect, the disclosure relates to a method of treating or lessening the severity of pain in a subject comprising administering Compound 1, or a pharmaceutically acceptable salt thereof, to the subject in a total daily dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a method of treating or lessening the severity of pain in a subject comprising administering Compound 1, or a pharmaceutically acceptable salt thereof, to the subject in a total daily dose of 23 mg. In another aspect, the disclosure relates to a method of treating or lessening the severity of pain in a subject comprising administering Compound 1, or a pharmaceutically acceptable salt thereof, to the subject in a total daily dose of 45 mg. In another aspect, the disclosure relates to a method of treating or lessening the severity of pain in a subject comprising administering Compound 1, or a pharmaceutically acceptable salt thereof, to the subject in a total daily dose of 46 mg. In another aspect, the disclosure relates to a method of treating or lessening the severity of pain in a subject comprising administering Compound 1, or a pharmaceutically acceptable salt thereof, to the subject in a total daily dose of 50 mg. In another aspect, the disclosure relates to a method of treating or lessening the severity of pain in a subject comprising administering Compound 1, or a pharmaceutically acceptable salt thereof, to the subject in a total daily dose of 60 mg. In another aspect, the disclosure relates to a method of treating or lessening the severity of pain in a subject comprising administering Compound 1, or a pharmaceutically acceptable salt thereof, to the subject in a total daily dose of 69 mg. In another aspect, the disclosure relates to a method of treating or lessening the severity of pain in a subject comprising administering Compound 1, or a pharmaceutically acceptable salt thereof, to the subject in a total daily dose of 70 mg. In another aspect, the disclosure relates to a method of treating or lessening the severity of pain in a subject comprising administering Compound **1,** or a pharmaceutically acceptable salt thereof, to the subject in a total daily dose of 75 mg. In another aspect, the disclosure relates to a method of treating or lessening the severity of pain in a subject comprising administering Compound 1, or a pharmaceutically acceptable salt thereof, to the subject in a total daily dose of 90 mg. In another aspect, the disclosure relates to a method of treating or lessening the severity of pain in a subject comprising administering Compound 1, or a pharmaceutically acceptable salt thereof, to the subject in a total daily dose of 100 mg. In another aspect, the disclosure relates to a method of treating or lessening the severity of pain in a subject comprising administering Compound 1, or a pharmaceutically acceptable salt thereof, to the subject in a total daily dose of 110 mg. In another aspect, the disclosure relates to a method of treating or lessening the severity of pain in a subject comprising administering Compound 1, or a

pharmaceutically acceptable salt thereof, to the subject in a total daily dose of 125 mg.

In another aspect, the disclosure relates to a method of treating or lessening the severity of pain in a subject comprising administering Compound 1 to the subject in a total daily dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a method of treating or lessening the severity of pain in a subject comprising administering Compound 1 to the subject in a total daily dose of about 23 mg. In another aspect, the disclosure relates to a method of treating or lessening the severity of pain in a subject comprising administering Compound 1 to the subject in a total daily dose of about 45 mg. In another aspect, the disclosure relates to a method of treating or lessening the severity of pain in a subject comprising administering Compound 1 to the subject in a total daily dose of about 46 mg. In another aspect, the disclosure relates to a method of treating or lessening the severity of pain in a subject comprising administering Compound 1 to the subject in a total daily dose of about 50 mg. In another aspect, the disclosure relates to a method of treating or lessening the severity of pain in a subject comprising administering Compound 1 to the subject in a total daily dose of about 60 mg. In another aspect, the disclosure relates to a method of treating or lessening the severity of pain in a subject comprising administering Compound 1 to the subject in a total daily dose of about 69 mg. In another aspect, the disclosure relates to a method of treating or lessening the severity of pain in a subject comprising administering Compound 1 to the subject in a total daily dose of about 70 mg. In another aspect, the disclosure relates to a method of treating or lessening the severity of pain in a subject comprising administering Compound 1 to the subject in a total daily dose of about 75 mg. In another aspect, the disclosure relates to a method of treating or lessening the severity of pain in a subject comprising administering Compound 1 to the subject in a total daily dose of about 90 mg. In another aspect, the disclosure relates to a method of treating or lessening the severity of pain in a subject comprising administering Compound 1 to the subject in a total daily dose of about 100 mg. In another aspect, the disclosure relates to a method of treating or lessening the severity of pain in a subject comprising administering Compound 1 to the subject in a total daily dose of about 110 mg. In another aspect, the disclosure relates to a method of treating or lessening the severity of pain in a subject comprising administering Compound 1 to the subject in a total daily dose of about 125 mg.

In another aspect, the disclosure relates to a method of treating or lessening the severity of pain in a subject comprising administering Compound 1 to the subject in a total daily dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a method of treating or lessening the severity of pain in a subject comprising administering Compound 1 to the subject in a total daily dose of 23 mg. In another aspect, the disclosure relates to a method of treating or lessening the severity of pain in a subject comprising administering Compound 1 to the subject in a total daily dose of 45 mg. In another aspect, the disclosure relates to a method of treating or lessening the severity of pain in a subject comprising administering Compound 1 to the subject in a total daily dose of 46 mg. In another aspect, the disclosure relates to a method of treating or lessening the severity of pain in a subject comprising administering Compound 1 to the subject in a total daily dose of 50 mg. In another aspect, the disclosure relates to a method of treating or lessening the severity of pain in a subject comprising administering Compound 1 to the subject in a total daily dose of 60 mg. In another aspect, the disclosure relates to a method of treating or lessening the severity of pain in a subject comprising administering Compound 1 to the subject in a total daily dose of 69 mg. In another aspect, the disclosure relates to a method of treating or lessening the severity of pain in a subject comprising administering Compound 1 to the subject in a total daily dose of 70 mg. In another aspect, the disclosure relates to a method of treating or lessening the severity of pain in a subject comprising administering Compound 1 to the subject in a total daily dose of 75 mg. In another aspect, the disclosure relates to a method of treating or lessening the severity of pain in a subject comprising administering Compound 1 to the subject in a total daily dose of 90 mg. In another aspect, the disclosure relates to a method of treating or lessening the severity of pain in a subject comprising administering Compound 1 to the subject in a total daily dose of 100 mg. In another aspect, the disclosure relates to a method of treating or lessening the severity of pain in a subject comprising administering Compound 1 to the subject in a total daily dose of 110 mg. In another aspect, the disclosure relates to a method of treating or lessening the severity of pain in a subject comprising administering Compound 1 to the subject in a total daily dose of 125 mg.

*Indications*

According to the invention, Compound 1 or a pharmaceutically acceptable salt thereof, is for use in a method of treating or lessening the severity of pain in a subject, wherein Compound 1 or a pharmaceutically acceptable salt thereof is administered to said subject in an amount of 10 mg to 300 mg per day. Compound 1, or a pharmaceutically acceptable salt thereof, may be administered to a subject for treating or lessening the severity of any type of pain known in the art.

In some embodiments, the disclosure features a method of treating or lessening the severity in a subject of acute pain, sub-acute and chronic pain, nociceptive pain, neuropathic pain, inflammatory pain, nociplastic pain, arthritis,

migraine, cluster headaches, trigeminal neuralgia, herpetic neuralgia, general neuralgias, epilepsy, epilepsy conditions, neurodegenerative disorders, psychiatric disorders, anxiety, depression, bipolar disorder, myotonia, arrhythmia, movement disorders, neuroendocrine disorders, ataxia, central neuropathic pain of multiple sclerosis and irritable bowel syndrome, incontinence, pathological cough, visceral pain, osteoarthritis pain, postherpetic neuralgia, diabetic neuropathy, radicular pain, sciatica, back pain, unspecific chronic back pain, head pain, neck pain, moderate pain, severe pain, intractable pain, nociceptive pain, breakthrough pain, postsurgical pain (e.g., joint replacement pain, soft tissue surgery pain, herniorrhaphy pain, bunionectomy pain or abdominoplasty pain), cancer pain including chronic cancer pain and breakthrough cancer pain, stroke (e.g., post stroke central neuropathic pain), whiplash associated disorders, fragility fractures, spinal fractures, ankylosing spondylitis, pemphigus, Raynaud's Disease, scleroderma, systemic lupus erythematosus, Epidermolysis bullosa, gout, juvenile idiopathic arthritis, melorheostosis, polymyalgia reumatica, pyoderma gangrenosum, chronic widespread pain, diffuse idiopathic skeletal hyperostosis, disc degeneration/herniation pain, radiculopathy, facet joint syndrome, failed back surgery syndrome, burns, carpal tunnel syndrome, Paget's disease pain, spinal canal stenosis, spondylodyscitis, transverse myelitis, Ehlers-Danlos syndrome, Fabry's disease, mastocytocytosis, neurofibromatosis, ocular neuropathic pain, sarcoidosis, spondylolysis, spondylolisthesis, chemotherapy induced oral mucositis, Charcot neuropathic osteoarhropathy, temporo-mandibular joint disorder, painful joint arthroplasties, non-cardiac chest pain, pudendal, renal colic, biliary tract diseases, vascular leg ulcers, pain in Parkinson's disease, pain in Alzheimer's disease, cerebral ischemia, traumatic brain injury, amyotrophic lateral sclerosis, stress induced angina, exercise induced angina, palpitations, hypertension, or abnormal gastro-intestinal motility, comprising administering an effective amount of Compound 1, a pharmaceutically acceptable salt thereof or a pharmaceutical composition thereof.

In another embodiment, the disclosure features a method of treating or lessening the severity in a subject of femur cancer pain; non-malignant chronic bone pain; rheumatoid arthritis; osteoarthritis; spinal stenosis; neuropathic low back pain; myofascial pain syndrome; fibromyalgia; temporomandibular joint pain; chronic visceral pain, abdominal pain; pancreatic pain; IBS pain; chronic and acute headache pain; migraine; tension headache; cluster headaches; chronic and acute neuropathic pain, post-herpetic neuralgia; diabetic neuropathy; HIV-associated neuropathy; trigeminal neuralgia; Charcot-Marie-Tooth neuropathy; hereditary sensory neuropathy; peripheral nerve injury; painful neuromas; ectopic proximal and distal discharges; radiculopathy; chemotherapy induced neuropathic pain; radiotherapy-induced neuropathic pain; persistent/chronic post-surgical pain (e.g., post amputation, post-thoracotomy, post-cardiac surgery), post-mastectomy pain; central pain; spinal cord injury pain; post-stroke pain; thalamic pain; phantom pain (e.g., following removal of lower extremity, upper extremity, breast); intractable pain; acute pain, acute post-operative pain; acute musculoskeletal pain; joint pain; mechanical low back pain; neck pain; tendonitis; injury pain; exercise pain; acute visceral pain; pyelonephritis; appendicitis; cholecystitis; intestinal obstruction; hernias; chest pain, cardiac pain; pelvic pain, renal colic pain, acute obstetric pain, labor pain; cesarean section pain; acute inflammatory pain, burn pain, trauma pain; acute intermittent pain, endometriosis; acute herpes zoster pain; sickle cell anemia; acute pancreatitis; breakthrough pain; orofacial pain; sinusitis pain; dental pain; multiple sclerosis (MS) pain; pain in depression; leprosy pain; Behcet's disease pain; adiposis dolorosa; phlebitic pain; Guillain-Barre pain; painful legs and moving toes; Haglund syndrome; erythromelalgia pain; Fabry's disease pain; bladder and urogenital disease; urinary incontinence, pathological cough; hyperactive bladder; painful bladder syndrome; interstitial cystitis (IC); prostatitis; complex regional pain syndrome (CRPS), type I, complex regional pain syndrome (CRPS) type II; widespread pain, paroxysmal extreme pain, pruritus, tinnitus, or angina-induced pain, comprising administering an effective amount of Compound 1, a pharmaceutically acceptable salt thereof or a pharmaceutical composition thereof.

In yet another embodiment, the disclosure features a method of treating or lessening the severity in a subject of acute pain comprising administering an effective amount of Compound 1, a pharmaceutically acceptable salt thereof or a pharmaceutical composition thereof. In some embodiments, the acute pain comprises acute post-operative pain.

In yet another embodiment, the disclosure features a method of treating or lessening the severity in a subject of postsurgical pain (e.g., joint replacement pain, soft tissue surgery pain, herniorrhaphy pain, bunionectomy pain or abdominoplasty pain) comprising administering an effective amount of Compound 1, a pharmaceutically acceptable salt thereof or a pharmaceutical composition thereof.

In yet another embodiment, the disclosure features a method of treating or lessening the severity in a subject of bunionectomy pain comprising administering an effective amount of Compound 1, a pharmaceutically acceptable salt thereof or a pharmaceutical composition thereof.

In yet another embodiment, the disclosure features a method of treating or lessening the severity in a subject of herniorrhaphy pain comprising administering an effective amount of Compound 1, a pharmaceutically acceptable salt thereof or a pharmaceutical composition thereof.

In yet another embodiment, the disclosure features a method of treating or lessening the severity in a subject of abdominoplasty pain comprising administering an effective amount of Compound 1, a pharmaceutically acceptable salt thereof or a pharmaceutical composition thereof.

In yet another embodiment, the disclosure features a method of treating or lessening the severity in a subject of visceral pain comprising administering an effective amount of Compound 1, a pharmaceutically acceptable salt thereof or

a pharmaceutical composition thereof. In some aspects, the visceral pain comprises visceral pain from abdominoplasty.

In yet another embodiment, the disclosure features a method of treating or lessening the severity in a subject of a neurodegenerative disease comprising administering an effective amount of Compound 1, a pharmaceutically acceptable salt thereof or a pharmaceutical composition thereof. In some aspects, the neurodegenerative disease comprises multiple sclerosis. In some aspects, the neurodegenerative disease comprises Pitt Hopkins Syndrome (PTHS).

In another embodiment, the disclosure features a method of treating or lessening the severity in a subject of acute pain, chronic pain, neuropathic pain, inflammatory pain, arthritis, migraine, cluster headaches, trigeminal neuralgia, herpetic neuralgia, general neuralgias, epilepsy, epilepsy conditions, neurodegenerative disorders, psychiatric disorders, anxiety, depression, bipolar disorder, myotonia, arrhythmia, movement disorders, neuroendocrine disorders, ataxia, multiple sclerosis, irritable bowel syndrome, incontinence, pathological cough, visceral pain, osteoarthritis pain, post-herpetic neuralgia, diabetic neuropathy, radicular pain, sciatica, back pain, head pain, neck pain, severe pain, intractable pain, nociceptive pain, breakthrough pain, postsurgical pain (e.g., herniorrhaphy pain, bunionectomy pain or abdominoplasty pain), cancer pain, stroke, cerebral ischemia, traumatic brain injury, amyotrophic lateral sclerosis, stress induced angina, exercise induced angina, palpitations, hypertension, or abnormal gastro-intestinal motility, comprising administering an effective amount of Compound 1, a pharmaceutically acceptable salt thereof or a pharmaceutical composition thereof.

*Patient Populations*

Compound 1, or a pharmaceutically acceptable salt thereof, may be administered to a subject having pain of any severity for treating or lessening the severity of the pain.

In some embodiments, the subject has a baseline pain score of at least 4 on an 11-point Numeric Pain Rating Scale prior to administration of Compound 1, or a pharmaceutically acceptable salt thereof.

In some embodiments, the subject has a baseline pain level of moderate or severe on a Verbal Categorical Rating Scale prior to administration of Compound 1, or a pharmaceutically acceptable salt thereof.

Compounds, Pharmaceutically Acceptable Salts, and Compositions for Use

The invention relates to Compound 1 or a pharmaceutically acceptable salt thereof, for use in a method of treating or lessening the severity of pain in a subject, comprising administering to the subject Compound 1 or a pharmaceutically acceptable salt thereof in an amount of 10 mg to 300 mg per day.

In another aspect, the disclosure relates to Compound 1, or a pharmaceutically acceptable salt or pharmaceutical composition thereof, for use in a method of treating or lessening the severity of pain in a subject in accordance with the method described herein (including any embodiment thereof).

In another aspect, the disclosure relates to Compound 1, or a pharmaceutically acceptable salt thereof, for use in a method of treating or lessening the severity of pain in a subject, comprising administering to the subject Compound 1, or the pharmaceutically acceptable salt thereof, twice per day in a dose of about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, or about 75 mg.

In another aspect, the disclosure relates to Compound 1 for use in a method of treating or lessening the severity of pain in a subject, comprising administering to the subject Compound 1 twice per day in a dose of about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, or about 75 mg.

In another aspect, the disclosure relates to Compound 1 for use in a method of treating or lessening the severity of pain in a subject, comprising administering to the subject Compound 1 twice per day in a dose of 10 mg, 15 mg, 20 mg, 25 mg, 30 mg, 35 mg, 40 mg, 45 mg, 50 mg, 55 mg, 60 mg, 65 mg, 70 mg, or 75 mg.

In another aspect, the disclosure relates to Compound 1, or a pharmaceutically acceptable salt thereof, for use in a method of treating or lessening the severity of pain in a subject, comprising administering to the subject Compound 1, or the pharmaceutically acceptable salt thereof, in a daily dose of about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, or about 150 mg.

In another aspect, the disclosure relates to Compound 1 for use in a method of treating or lessening the severity of pain in a subject, comprising administering to the subject Compound 1, or the pharmaceutically acceptable salt thereof, in a daily dose of about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, or about 150 mg.

In another aspect, the disclosure relates to Compound 1 for use in a method of treating or lessening the severity of pain in a subject, comprising administering to the subject Compound 1, or the pharmaceutically acceptable salt thereof, in a daily dose of 20 mg, 30 mg, 40 mg, 50 mg, 60 mg, 70 mg, 80 mg, 90 mg, 100 mg, 110 mg, 120 mg, 130 mg, 140 mg, or 150

mg.

In another aspect, the disclosure relates to Compound 1, or a pharmaceutically acceptable salt thereof, for use in a method of treating or lessening the severity of pain in a subject, comprising administering to the subject Compound 1, or the pharmaceutically acceptable salt thereof, twice per day in a dose of about 10 mg, about 30 mg, or about 50 mg. In another aspect, the disclosure relates to Compound 1, or a pharmaceutically acceptable salt thereof, for use in a method of treating or lessening the severity of pain in a subject, comprising administering to the subject Compound 1, or the pharmaceutically acceptable salt thereof, twice per day in a dose of 10 mg, 30 mg, or 50 mg.

In another aspect, the disclosure relates to Compound 1, or a pharmaceutically acceptable salt thereof, for use in a method of treating or lessening the severity of pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of gut pain in a subject in accordance with the method described herein (including any embodiment thereof).

In another aspect, the disclosure relates to Compound 1, or a pharmaceutically acceptable salt thereof, for use in a method of treating or lessening the severity of gut pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of gut pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of gut pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of gut pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of neuropathic pain in a subject in accordance with the method described herein (including any embodiment thereof).

In another aspect, the disclosure relates to Compound 1, or a pharmaceutically acceptable salt thereof, for use in a method of treating or lessening the severity of neuropathic pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of neuropathic pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of neuropathic pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in

a method of treating or lessening the severity of neuropathic pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of idiopathic small fiber neuropathy in a subject in accordance with the method described herein (including any embodiment thereof).

In another aspect, the disclosure relates to Compound 1, or a pharmaceutically acceptable salt thereof, for use in a method of treating or lessening the severity of idiopathic small fiber neuropathy in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of idiopathic small fiber neuropathy in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of idiopathic small fiber neuropathy in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of idiopathic small fiber neuropathy in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of diabetic peripheral neuropathy in a subject in accordance with the method described herein (including any embodiment thereof).

In another aspect, the disclosure relates to Compound 1, or a pharmaceutically acceptable salt thereof, for use in a method of treating or lessening the severity of diabetic peripheral neuropathy in a subject, comprising administering to the subject Compound 1, or the pharmaceutically acceptable salt thereof, once per day in a dose of about 23 mg, about 46 mg, about 50 mg, about 69 mg, or about 70 mg.

In another aspect, the disclosure relates to Compound 1, or a pharmaceutically acceptable salt thereof, for use in a method of treating or lessening the severity of diabetic peripheral neuropathy in a subject, comprising administering to the subject Compound 1, or the pharmaceutically acceptable salt thereof, twice per day in a dose of about 10 mg, about 30 mg, or about 50 mg, or about 130 mg.

In another aspect, the disclosure relates to Compound 1, or a pharmaceutically acceptable salt thereof, for use in a method of treating or lessening the severity of diabetic peripheral neuropathy in a subject, comprising administering to the subject Compound 1, or the pharmaceutically acceptable salt thereof, twice per day in a dose of about 10 mg, about 30 mg, or about 50 mg, or about 130 mg for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of diabetic peripheral neuropathy in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of diabetic peripheral neuropathy in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of musculoskeletal pain in a subject in accordance with the method described herein (including any embodiment thereof).

In another aspect, the disclosure relates to Compound 1, or a pharmaceutically acceptable salt thereof, for use in a method of treating or lessening the severity of musculoskeletal pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in

a method of treating or lessening the severity of musculoskeletal pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of musculoskeletal pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of musculoskeletal pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of osteoarthritis pain in a subject in accordance with the method described herein (including any embodiment thereof).

In another aspect, the disclosure relates to Compound 1, or a pharmaceutically acceptable salt thereof, for use in a method of treating or lessening the severity of osteoarthritis pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of osteoarthritis pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of osteoarthritis pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of osteoarthritis pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of acute pain in a subject in accordance with the method described herein (including any embodiment thereof).

In another aspect, the disclosure relates to Compound 1, or a pharmaceutically acceptable salt thereof, for use in a method of treating or lessening the severity of acute pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of acute pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of acute pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of acute pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of inflammatory pain in a subject in accordance with the method described herein (including any embodiment thereof).

In another aspect, the disclosure relates to Compound 1, or a pharmaceutically acceptable salt thereof, for use in

a method of treating or lessening the severity of inflammatory pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of inflammatory pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of inflammatory pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of inflammatory pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of cancer pain in a subject in accordance with the method described herein (including any embodiment thereof).

In another aspect, the disclosure relates to Compound 1, or a pharmaceutically acceptable salt thereof, for use in a method of treating or lessening the severity of cancer pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of cancer pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of cancer pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of cancer pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of idiopathic pain in a subject in accordance with the method described herein (including any embodiment thereof).

In another aspect, the disclosure relates to Compound 1, or a pharmaceutically acceptable salt thereof, for use in a method of treating or lessening the severity of idiopathic pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of idiopathic pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of idiopathic pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of idiopathic pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about

30 mg, or about 50 mg per day each day after the first day.

In another aspect, the disclosure relates to Compound 1, or a pharmaceutically acceptable salt or pharmaceutical composition thereof, for use in a method of treating or lessening the severity of postsurgical pain in a subject in accordance with the method described herein (including any embodiment thereof).

In another aspect, the disclosure relates to Compound 1, or a pharmaceutically acceptable salt thereof, for use in a method of treating or lessening the severity of postsurgical pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of postsurgical pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of postsurgical pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of postsurgical pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to Compound 1, or a pharmaceutically acceptable salt or pharmaceutical composition thereof, for use in a method of treating or lessening the severity of visceral pain in a subject in accordance with the method described herein (including any embodiment thereof).

In another aspect, the disclosure relates to Compound 1, or a pharmaceutically acceptable salt thereof, for use in a method of treating or lessening the severity of visceral pain in a subject, comprising administering to the subject Compound 1, or the pharmaceutically acceptable salt thereof, twice per day in a dose of about 10 mg, about 30 mg, or about 50 mg.

In another aspect, the disclosure relates to Compound 1, or a pharmaceutically acceptable salt thereof, for use in a method of treating or lessening the severity of visceral pain in a subject, comprising administering to the subject Compound 1, or the pharmaceutically acceptable salt thereof, twice per day in a dose of about 10 mg, about 30 mg, or about 50 mg, for at least one week, at least two weeks, at least three weeks, at least four weeks, at least five weeks, or at least six weeks, or from one to six weeks.

In another aspect, the disclosure relates to Compound 1, or a pharmaceutically acceptable salt thereof, for use in a method of treating or lessening the severity of visceral pain in a subject, comprising administering to the subject Compound 1, or the pharmaceutically acceptable salt thereof, twice per day in a dose of about 10 mg (20 mg per day), about 30 mg (60 mg per day), or about 50 mg (100 mg per day).

In another aspect, the disclosure relates to Compound 1, or a pharmaceutically acceptable salt thereof, for use in a method of treating or lessening the severity of visceral pain in a subject, comprising administering to the subject Compound 1, or the pharmaceutically acceptable salt thereof, in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 23 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 45 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 46 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 50 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 60 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 70 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 75 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 90 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 100 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 110 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 125 mg.

In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 23 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 45 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 46 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 50 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 60 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 69 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 70 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 75 mg. In another aspect, the disclosure relates to a composition comprising Compound 1, or a pharmaceutically acceptable salt thereof, for use in a method of treating or lessening the severity of pain in a subject, wherein the composition is prepared for administration of

Compound 1, or the pharmaceutically acceptable salt thereof, to the subject once per day in a dose of 90 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 100 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 110 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of 125 mg.

In another aspect, the disclosure relates to a composition comprising Compound 1 for use in a method of treating or lessening the severity of pain in a subject, wherein the composition is prepared for administration of Compound 1 to the subject once per day in a dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a composition comprising Compound 1 for use in a method of treating or lessening the severity of pain in a subject, wherein the composition is prepared for administration of Compound 1 to the subject once per day in a dose of about 23 mg. In another aspect, the disclosure relates to a composition comprising Compound 1 for use in a method of treating or lessening the severity of pain in a subject, wherein the composition is prepared for administration of Compound 1 to the subject once per day in a dose of about 45 mg. In another aspect, the disclosure relates to a composition comprising Compound 1 for use in a method of treating or lessening the severity of pain in a subject, wherein the composition is prepared for administration of Compound 1 to the subject once per day in a dose of about 46 mg. In another aspect, the disclosure relates to a composition comprising Compound 1 for use in a method of treating or lessening the severity of pain in a subject, wherein the composition is prepared for administration of Compound 1 to the subject once per day in a dose of about 50 mg. In another aspect, the disclosure relates to a composition comprising Compound 1 for use in a method of treating or lessening the severity of pain in a subject, wherein the composition is prepared for administration of Compound 1 to the subject once per day in a dose of about 60 mg. In another aspect, the disclosure relates to a composition comprising Compound 1 for use in a method of treating or lessening the severity of pain in a subject, wherein the composition is prepared for administration of Compound 1 to the subject once per day in a dose of about 69 mg. In another aspect, the disclosure relates to a composition comprising Compound 1 for use in a method of treating or lessening the severity of pain in a subject, wherein the composition is prepared for administration of Compound 1 to the subject once per day in a dose of about 70 mg. In another aspect, the disclosure relates to a composition comprising Compound 1 for use in a method of treating or lessening the severity of pain in a subject, wherein the composition is prepared for administration of Compound 1 to the subject once per day in a dose of about 75 mg. In another aspect, the disclosure relates to a composition comprising Compound 1 for use in a method of treating or lessening the severity of pain in a subject, wherein the composition is prepared for administration of Compound 1 to the subject once per day in a dose of about 90 mg. In another aspect, the disclosure relates to a composition comprising Compound 1 for use in a method of treating or lessening the severity of pain in a subject, wherein the composition is prepared for administration of Compound 1 to the subject once per day in a dose of about 100 mg. In another aspect, the disclosure relates to a composition comprising Compound 1 for use in a method of treating or lessening the severity of pain in a subject, wherein the composition is prepared for administration of Compound 1 to the subject once per day in a dose of about 110 mg. In another aspect, the disclosure relates to a composition comprising Compound 1 for use in a method of treating or lessening the severity of pain in a subject, wherein the composition is prepared for administration of Compound 1 to the subject once per day in a dose of about 125 mg.

In another aspect, the disclosure relates to a composition comprising Compound 1 for use in a method of treating or lessening the severity of pain in a subject, wherein the composition is prepared for administration of Compound 1 to the subject once per day in a dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a composition comprising Compound 1 for use in a method of treating or lessening the severity of pain in a subject, wherein the composition is prepared for administration of Compound 1 to the subject once per day in a dose of 23 mg. In another aspect, the disclosure relates to a composition comprising Compound 1 for use in a method of treating or lessening the severity of pain in a subject, wherein the composition is prepared for administration of Compound 1 to the subject once per day in a dose of 45 mg. In another aspect, the disclosure relates to a composition comprising Compound 1 for use in a method of treating or lessening the severity of pain in a subject, wherein the composition is prepared for administration of Compound 1 to the subject once per day in a dose of 46 mg. In another aspect, the disclosure relates to a composition comprising Compound 1 for use in a method of treating or lessening the severity of pain in a subject, wherein the composition is prepared for administration of Compound 1 to the subject once per

day in a dose of 50 mg. In another aspect, the disclosure relates to a composition comprising Compound 1 for use in a method of treating or lessening the severity of pain in a subject, wherein the composition is prepared for administration of Compound 1 to the subject once per day in a dose of 60 mg. In another aspect, the disclosure relates to a composition comprising Compound 1 for use in a method of treating or lessening the severity of pain in a subject, wherein the composition is prepared for administration of Compound 1 to the subject once per day in a dose of 69 mg. In another aspect, the disclosure relates to a composition comprising Compound 1 for use in a method of treating or lessening the severity of pain in a subject, wherein the composition is prepared for administration of Compound 1 to the subject once per day in a dose of 70 mg. In another aspect, the disclosure relates to a composition comprising Compound 1 for use in a method of treating or lessening the severity of pain in a subject, wherein the composition is prepared for administration of Compound 1 to the subject once per day in a dose of 75 mg. In another aspect, the disclosure relates to a composition comprising Compound 1 for use in a method of treating or lessening the severity of pain in a subject, wherein the composition is prepared for administration of Compound 1 to the subject once per day in a dose of 90 mg. In another aspect, the disclosure relates to a composition comprising Compound 1 for use in a method of treating or lessening the severity of pain in a subject, wherein the composition is prepared for administration of Compound 1 to the subject once per day in a dose of 100 mg. In another aspect, the disclosure relates to a composition comprising Compound 1 for use in a method of treating or lessening the severity of pain in a subject, wherein the composition is prepared for administration of Compound 1 to the subject once per day in a dose of 110 mg. In another aspect, the disclosure relates to a composition comprising Compound 1 for use in a method of treating or lessening the severity of pain in a subject, wherein the composition is prepared for administration of Compound 1 to the subject once per day in a dose of 125 mg.

In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 23 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 45 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 46 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 50 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 60 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 69 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 70 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 75 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 90 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 100 mg. In another aspect, the disclosure relates to a composition comprising Compound 1, or a pharmaceutically acceptable salt thereof, for use in a method of treating or

lessening the severity of pain in a subject, wherein the composition is prepared for administration of Compound 1, or the pharmaceutically acceptable salt thereof, to the subject in a total daily dose of about 110 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of about 125 mg.

In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 23 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 45 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 46 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 50 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 60 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 69 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 70 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 75 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 90 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 100 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 110 mg. In another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a total daily dose of 125 mg.

In another aspect, the disclosure relates to a composition comprising Compound 1 for use in a method of treating or lessening the severity of pain in a subject, wherein the composition is prepared for administration of Compound 1 to the subject in a total daily dose of about 10 mg, about 15 mg, about 20 mg, about 23 mg, about 25 mg, about 30 mg, about 35 mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, the disclosure relates to a composition comprising Compound 1 for use in a method of treating or lessening the severity of pain in a subject, wherein the composition is prepared for administration of Compound 1 to the subject in a total daily dose of about 23 mg. In another aspect, the disclosure relates to a composition comprising Compound 1 for use in a method of treating or lessening the severity of pain in a subject, wherein the composition is prepared for administration of Compound 1 to the subject in a total daily dose of about 45 mg. In another aspect, the disclosure relates to a composition comprising Compound 1 for use in a method of treating or lessening the

severity of pain in a subject, wherein the composition is prepared for administration of Compound 1 to the subject in a total daily dose of about 46 mg. In another aspect, the disclosure relates to a composition comprising Compound 1 for use in a method of treating or lessening the severity of pain in a subject, wherein the composition is prepared for administration of Compound 1 to the subject in a total daily dose of about 50 mg. In another aspect, the disclosure relates to a composition comprising Compound 1 for use in a method of treating or lessening the severity of pain in a subject, wherein the composition is prepared for administration of Compound 1 to the subject in a total daily dose of about 60 mg. In another aspect, the disclosure relates to a composition comprising Compound 1 for use in a method of treating or lessening the severity of pain in a subject, wherein the composition is prepared for administration of Compound 1 to the subject in a total daily dose of about 69 mg. In another aspect, the disclosure relates to a composition comprising Compound 1 for use in a method of treating or lessening the severity of pain in a subject, wherein the composition is prepared for administration of Compound 1 to the subject in a total daily dose of about 70 mg. In another aspect, the disclosure relates to a composition comprising Compound 1 for use in a method of treating or lessening the severity of pain in a subject, wherein the composition is prepared for administration of Compound 1 to the subject in a total daily dose of about 75 mg. In another aspect, the disclosure relates to a composition comprising Compound 1 for use in a method of treating or lessening the severity of pain in a subject, wherein the composition is prepared for administration of Compound 1 to the subject in a total daily dose of about 90 mg. In another aspect, the disclosure relates to a composition comprising Compound 1 for use in a method of treating or lessening the severity of pain in a subject, wherein the composition is prepared for administration of Compound 1 to the subject in a total daily dose of about 100 mg. In another aspect, the disclosure relates to a composition comprising Compound 1 for use in a method of treating or lessening the severity of pain in a subject, wherein the composition is prepared for administration of Compound 1 to the subject in a total daily dose of about 110 mg. In another aspect, the disclosure relates to a composition comprising Compound 1 for use in a method of treating or lessening the severity of pain in a subject, wherein the composition is prepared for administration of Compound 1 to the subject in a total daily dose of about 125 mg.

In another aspect, the disclosure relates to a composition comprising Compound 1 for use in a method of treating or lessening the severity of pain in a subject, wherein the composition is prepared for administration of Compound 1 to the subject in a total daily dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg. In another aspect, the disclosure relates to a composition comprising Compound 1 for use in a method of treating or lessening the severity of pain in a subject, wherein the composition is prepared for administration of Compound 1 to the subject in a total daily dose of 23 mg. In another aspect, the disclosure relates to a composition comprising Compound 1 for use in a method of treating or lessening the severity of pain in a subject, wherein the composition is prepared for administration of Compound 1 to the subject in a total daily dose of 45 mg. In another aspect, the disclosure relates to a composition comprising Compound 1 for use in a method of treating or lessening the severity of pain in a subject, wherein the composition is prepared for administration of Compound 1 to the subject in a total daily dose of 46 mg. In another aspect, the disclosure relates to a composition comprising Compound 1 for use in a method of treating or lessening the severity of pain in a subject, wherein the composition is prepared for administration of Compound 1 to the subject in a total daily dose of 50 mg. In another aspect, the disclosure relates to a composition comprising Compound 1 for use in a method of treating or lessening the severity of pain in a subject, wherein the composition is prepared for administration of Compound 1 to the subject in a total daily dose of 60 mg. In another aspect, the disclosure relates to a composition comprising Compound 1 for use in a method of treating or lessening the severity of pain in a subject, wherein the composition is prepared for administration of Compound 1 to the subject in a total daily dose of 69 mg. In another aspect, the disclosure relates to a composition comprising Compound 1 for use in a method of treating or lessening the severity of pain in a subject, wherein the composition is prepared for administration of Compound 1 to the subject in a total daily dose of 70 mg. In another aspect, the disclosure relates to a composition comprising Compound 1 for use in a method of treating or lessening the severity of pain in a subject, wherein the composition is prepared for administration of Compound 1 to the subject in a total daily dose of 75 mg. In another aspect, the disclosure relates to a composition comprising Compound 1 for use in a method of treating or lessening the severity of pain in a subject, wherein the composition is prepared for administration of Compound 1 to the subject in a total daily dose of 90 mg. In another aspect, the disclosure relates to a composition comprising Compound 1 for use in a method of treating or lessening the severity of pain in a subject, wherein the composition is prepared for administration of Compound 1 to the subject in a total daily dose of 100 mg. In another aspect, the disclosure relates to a composition comprising Compound 1 for use in a method of treating or lessening the severity of pain in a subject, wherein the composition is prepared for administration of Compound 1 to the subject in a total daily dose of 110 mg. In another aspect, the disclosure relates to a composition comprising Compound 1 for use in a method of treating or lessening the severity of pain in a subject, wherein the composition is prepared for administration of Compound 1 to the subject in a total daily dose of 125 mg.

**In** another aspect, the disclosure relates to a composition comprising Compound 1 for use in a method of treating or lessening the severity of pain in a subject, wherein the composition is prepared for administration of Compound 1 to the subject once per day in a dose of about 10 mg, about 15 mg, about 20 mg, about 23 mg, about 25 mg, about 30 mg, about 35

mg, about 40 mg, about 45 mg, about 46 mg, about 50 mg, about 55 mg, about 60 mg, about 65 mg, about 69 mg, about 70 mg, about 75 mg, about 80 mg, about 85 mg, about 90 mg, about 95 mg, about 100 mg, about 110 mg, about 120 mg, about 125 mg, about 140 mg, or about 150 mg.

**In** another aspect, the disclosure relates to a composition comprising Compound 1 for use in a method of treating or lessening the severity of pain in a subject, wherein the composition is prepared for administration of Compound 1 to the subject once per day in a dose of 10 mg, 15 mg, 20 mg, 23 mg, 25 mg, 30 mg, 35 mg, 40 mg, 45 mg, 46 mg, 50 mg, 55 mg, 60 mg, 65 mg, 69 mg, 70 mg, 75 mg, 80 mg, 85 mg, 90 mg, 95 mg, 100 mg, 110 mg, 120 mg, 125 mg, 140 mg, or 150 mg.

**In** another aspect, the disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 110 mg, about 120 mg, about 125 mg, about 140 mg, or about 150 mg. In another aspect, disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 45 mg. In another aspect, the disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 50 mg. In another aspect, disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 60 mg. In another aspect, disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 70 mg. In another aspect, the disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 75 mg. In another aspect, the disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 90 mg. In another aspect, the disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 100 mg. In another aspect, the disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 110 mg. In another aspect, the disclosure relates to a composition comprising Compound 1, or a pharmaceutically acceptable salt thereof, for use in a method of treating or lessening the severity of chronic pain in a subject, wherein the composition is prepared for administration of Compound 1, or the pharmaceutically acceptable salt thereof, to the subject once per day in a dose of 125 mg.

**In** another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically

acceptable salt thereof, for use in a method of treating or lessening the severity of gut pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of gut pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of gut pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of gut pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of neuropathic pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of neuropathic pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of neuropathic pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of neuropathic pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of idiopathic small fiber neuropathy in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of idiopathic small fiber neuropathy in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of idiopathic small fiber neuropathy in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of idiopathic small fiber neuropathy in a subject, wherein the composition is prepared for administration of Compound 1, or the pharmaceutically acceptable salt

thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of musculoskeletal pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of musculoskeletal pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of musculoskeletal pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of musculoskeletal pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of osteoarthritis pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of osteoarthritis pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of osteoarthritis pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of osteoarthritis pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of acute pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of acute pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of acute pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of acute pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of inflammatory pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of inflammatory pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of inflammatory pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of inflammatory pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of cancer pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of cancer pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of cancer pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of cancer pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of idiopathic pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of idiopathic pain in a subject, wherein the

composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of idiopathic pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of idiopathic pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of postsurgical pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of postsurgical pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of postsurgical pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of postsurgical pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

**In** another aspect, the disclosure relates to a composition comprising Compound 1, or a pharmaceutically acceptable salt thereof, for use in a method of treating or lessening the severity of visceral pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg.

**In** another aspect, the disclosure relates to a composition comprising Compound 1, or a pharmaceutically acceptable salt thereof, for use in a method of treating or lessening the severity of visceral pain in a subject, wherein the composition is prepared for administration of Compound 1, or the pharmaceutically acceptable salt thereof, to the subject once per day in a dose of about 10 mg, about 20 mg, about 30 mg, about 50 mg, about 60 mg, about 90 mg, about 100 mg, or about 150 mg, for at least one week, at least two weeks, at least three weeks, at least four weeks, at least five weeks, or at least six weeks, or from one to six weeks.

**In** another aspect, the disclosure relates to a composition comprising Compound 1, or a pharmaceutically acceptable salt thereof, for use in a method of treating or lessening the severity of visceral pain in a subject, wherein the composition is prepared for administration of Compound 1, or the pharmaceutically acceptable salt thereof, to the subject twice per day in a dose of about 10 mg (20 mg per day), about 30 mg (60 mg per day), or about 50 mg (100 mg per day).

**In** another aspect, the disclosure relates to a composition comprising Compound 1, or a pharmaceutically acceptable salt thereof, for use in a method of treating or lessening the severity of visceral pain in a subject, wherein the composition is prepared for administration of Compound 1, or the pharmaceutically acceptable salt thereof, to the subject in a first dose of about 20 mg, about 60 mg, or about 100 mg and a subsequent dose of about 10 mg, about 30 mg, or about 50 mg on the first day, and in two doses of about 10 mg, about 30 mg, or about 50 mg per day each day after the first day.

Manufacture of Medicaments

**In** another aspect, the disclosure relates to the use of Compound 1, or a pharmaceutically acceptable salt or pharmaceutical composition thereof, for the manufacture of a medicament for treating or lessening the severity of pain in a subject in accordance with the method described herein (including any embodiment thereof).

**In** yet another aspect, the disclosure provides the use of Compound 1, a pharmaceutically acceptable salt thereof, or a pharmaceutical composition thereof for the manufacture of a medicament for use in treating or lessening the severity in a subject of chronic pain, gut pain, neuropathic pain, musculoskeletal pain, acute pain, inflammatory pain, cancer pain, idiopathic pain, postsurgical pain (e.g., herniorrhaphy pain, bunionectomy pain or abdominoplasty pain), visceral pain, multiple sclerosis, Charcot-Marie-Tooth syndrome, incontinence, pathological cough, or cardiac arrhythmia.

**In** yet another aspect, the disclosure provides the use of Compound 1, a pharmaceutically acceptable salt thereof, or a pharmaceutical composition thereof for the manufacture of a medicament for use in treating or lessening the severity in a subject of chronic pain, gut pain, neuropathic pain, musculoskeletal pain, acute pain, inflammatory pain, cancer pain, idiopathic pain, postsurgical pain, herniorrhaphy pain, bunionectomy pain, multiple sclerosis, Charcot-Marie-Tooth syndrome, incontinence, or cardiac arrhythmia.

**In** yet another aspect, the disclosure provides the use of Compound 1, pharmaceutically acceptable salt, or pharmaceutical composition described herein for the manufacture of a medicament for use in treating or lessening the severity in a subject of gut pain, wherein gut pain comprises inflammatory bowel disease pain, Crohn's disease pain or interstitial cystitis pain.

**In** yet another aspect, the disclosure provides Compound 1, a pharmaceutically acceptable salt thereof, or a pharmaceutical composition thereof for the manufacture of a medicament for use in treating or lessening the severity in a subject of neuropathic pain. In some aspects, the neuropathic pain comprises post-herpetic neuralgia, small fiber neuropathy, diabetic neuropathy, or idiopathic small-fiber neuropathy. In some aspects, the neuropathic pain comprises diabetic neuropathy (e.g., diabetic peripheral neuropathy).

**In** yet another aspect, the disclosure provides Compound 1, a pharmaceutically acceptable salt thereof, or a pharmaceutical composition thereof for the manufacture of a medicament for use in a treating or lessening the severity in a subject of neuropathic pain, wherein neuropathic pain comprises post-herpetic neuralgia, diabetic neuralgia, painful HIV-associated sensory neuropathy, trigeminal neuralgia, burning mouth syndrome, post-amputation pain, phantom pain, painful neuroma, traumatic neuroma, Morton's neuroma, nerve entrapment injury, spinal stenosis, carpal tunnel syndrome, radicular pain, sciatica pain, nerve avulsion injury, brachial plexus avulsion injury, complex regional pain syndrome, drug therapy induced neuralgia, cancer chemotherapy induced neuralgia, anti-retroviral therapy induced neuralgia, post spinal cord injury pain, small fiber neuropathy, idiopathic small-fiber neuropathy, idiopathic sensory neuropathy or trigeminal autonomic neuropathy.

**In** yet another aspect, the disclosure provides Compound 1, a pharmaceutically acceptable salt thereof, or a pharmaceutical composition thereof for the manufacture of a medicament for use in treating or lessening the severity in a subject of musculoskeletal pain. In some aspects the musculoskeletal pain comprises osteoarthritis pain.

**In** yet another aspect, the disclosure provides Compound 1, a pharmaceutically acceptable salt thereof, or a pharmaceutical composition thereof for the manufacture of a medicament for use in treating or lessening the severity in a subject of musculoskeletal pain, wherein musculoskeletal pain comprises osteoarthritis pain, back pain, cold pain, burn pain or dental pain.

**In** yet another aspect, the disclosure provides Compound 1, a pharmaceutically acceptable salt thereof, or a pharmaceutical composition thereof for the manufacture of a medicament for use in treating or lessening the severity in a subject of inflammatory pain, wherein inflammatory pain comprises rheumatoid arthritis pain or vulvodynia.

**In** yet another aspect, the disclosure provides Compound 1, a pharmaceutically acceptable salt thereof, or a pharmaceutical composition thereof for the manufacture of a medicament for use in treating or lessening the severity in a subject of inflammatory pain, wherein inflammatory pain comprises rheumatoid arthritis pain.

**In** yet another aspect, the disclosure provides Compound 1, a pharmaceutically acceptable salt thereof, or a pharmaceutical composition thereof for the manufacture of a medicament for use in treating or lessening the severity in a subject of idiopathic pain, wherein idiopathic pain comprises fibromyalgia pain.

**In** yet another aspect, the disclosure provides Compound 1, a pharmaceutically acceptable salt thereof, or a pharmaceutical composition thereof for the manufacture of a medicament for use in treating or lessening the severity in a subject of pathological cough.

**In** yet another aspect, the disclosure provides Compound 1, a pharmaceutically acceptable salt thereof, or a pharmaceutical composition thereof for the manufacture of a medicament for use in treating or lessening the severity in a subject of acute pain. In some aspects, the acute pain comprises acute post-operative pain.

**In** yet another aspect, the disclosure provides Compound 1, a pharmaceutically acceptable salt thereof, or a pharmaceutical composition thereof for the manufacture of a medicament for use in treating or lessening the severity in a subject of postsurgical pain (e.g., herniorrhaphy pain, bunionectomy pain or abdominoplasty pain).

**In** yet another aspect, the disclosure provides Compound 1, a pharmaceutically acceptable salt thereof, or a pharmaceutical composition thereof for the manufacture of a medicament for use in treating or lessening the severity in a subject of herniorrhaphy pain.

**In** yet another aspect, the disclosure provides Compound 1, a pharmaceutically acceptable salt thereof, or a pharmaceutical composition thereof for the manufacture of a medicament for use in treating or lessening the severity in a subject of bunionectomy pain.

**In** yet another aspect, the disclosure provides Compound 1, a pharmaceutically acceptable salt thereof, or a pharmaceutical composition thereof for the manufacture of a medicament for use in treating or lessening the severity in a subject of abdominoplasty pain.

**In** yet another aspect, the disclosure provides Compound 1, a pharmaceutically acceptable salt thereof, or a pharmaceutical composition thereof for the manufacture of a medicament for use in treating or lessening the severity in a subject of visceral pain. In some aspects, the visceral pain comprises visceral pain from abdominoplasty.

**In** another aspect, the disclosure features Compound 1, or a pharmaceutically acceptable salt or pharmaceutical composition thereof, for the manufacture of a medicament for use in treating or lessening the severity in a subject of a neurodegenerative disease. In some aspects, the neurodegenerative disease comprises multiple sclerosis. In some aspects, the neurodegenerative disease comprises Pitt Hopkins Syndrome (PTHS).

**In** yet another aspect, the disclosure provides Compound 1, a pharmaceutically acceptable salt thereof, or a pharmaceutical composition thereof for the manufacture of a medicament for use in combination with one or more additional therapeutic agents administered concurrently with, prior to, or subsequent to treatment with the compound or pharmaceutical composition. In some embodiments, the additional therapeutic agent is a sodium channel inhibitor.

**In** another aspect, the disclosure provides Compound 1, a pharmaceutically acceptable salt thereof, or a pharmaceutical composition thereof for the manufacture of a medicament for use in treating or lessening the severity of acute pain, chronic pain, neuropathic pain, inflammatory pain, arthritis, migraine, cluster headaches, trigeminal neuralgia, herpetic neuralgia, general neuralgias, epilepsy, epilepsy conditions, neurodegenerative disorders, psychiatric disorders, anxiety, depression, bipolar disorder, myotonia, arrhythmia, movement disorders, neuroendocrine disorders, ataxia, multiple sclerosis, irritable bowel syndrome, incontinence, pathological cough, visceral pain, osteoarthritis pain, post-herpetic neuralgia, diabetic neuropathy, radicular pain, sciatica, back pain, head pain, neck pain, severe pain, intractable pain, nociceptive pain, breakthrough pain, postsurgical pain (e.g., herniorrhaphy pain, bunionectomy pain or abdominoplasty pain), cancer pain, stroke, cerebral ischemia, traumatic brain injury, amyotrophic lateral sclerosis, stress induced angina, exercise induced angina, palpitations, hypertension, or abnormal gastro-intestinal motility.

In another aspect, the disclosure provides Compound 1, a pharmaceutically acceptable salt thereof, or a pharmaceutical composition thereof for the manufacture of a medicament for use in treating or lessening the severity of femur cancer pain, non-malignant chronic bone pain, rheumatoid arthritis, osteoarthritis, spinal stenosis, neuropathic low back pain, myofascial pain syndrome, fibromyalgia, temporomandibular joint pain, chronic visceral pain, abdominal pain, pancreatic pain, IBS pain, chronic and acute headache pain, migraine, tension headache, cluster headaches, chronic and acute neuropathic pain, post-herpetic neuralgia, diabetic neuropathy, HIV-associated neuropathy, trigeminal neuralgia, Charcot-Marie-Tooth neuropathy, hereditary sensory neuropathy, peripheral nerve injury, painful neuromas, ectopic proximal and distal discharges, radiculopathy, chemotherapy induced neuropathic pain, radiotherapy-induced neuropathic pain, post-mastectomy pain, central pain, spinal cord injury pain, post-stroke pain, thalamic pain, complex regional pain syndrome, phantom pain, intractable pain, acute pain, acute post-operative pain, acute musculoskeletal pain, joint pain, mechanical low back pain, neck pain, tendonitis, injury pain, exercise pain, acute visceral pain, pyelonephritis, appendicitis, cholecystitis, intestinal obstruction, hernias, chest pain, cardiac pain, pelvic pain, renal colic pain, acute obstetric pain, labor pain, cesarean section pain, acute inflammatory, burn pain, trauma pain, acute intermittent pain, endometriosis, acute herpes zoster pain, sickle cell anemia, acute pancreatitis, breakthrough pain, orofacial pain, sinusitis pain, dental pain, multiple sclerosis (MS) pain, pain in depression, leprosy pain, Behcet's disease pain, adiposis dolorosa, phlebitic pain, Guillain-Barre pain, painful legs and moving toes, Haglund syndrome, erythromelalgia pain, Fabry's disease pain, bladder and urogenital disease, urinary incontinence, pathological cough, hyperactive bladder, painful bladder syndrome, interstitial cystitis (IC), prostatitis, complex regional pain syndrome (CRPS) type **I,** complex regional pain syndrome (CRPS) type **II,** widespread pain, paroxysmal extreme pain, pruritus, tinnitus, or angina-induced pain.

In another aspect, the invention features a compound of the invention, or a pharmaceutically acceptable salt or pharmaceutical composition thereof, for use in a method of treating or lessening the severity in a subject of trigeminal neuralgia, migraines treated with botox, cervical radiculopathy, occipital neuralgia, axillary neuropathy, radial neuropathy, ulnar neuropathy, brachial plexopathy, thoracic radiculopathy, intercostal neuralgia, lumbrosacral radiculopathy, iliolingual neuralgia, pudendal neuralgia, femoral neuropathy, meralgia paresthetica, saphenous neuropathy, sciatic neuropathy, peroneal neuropathy, tibial neuropathy, lumbosacral plexopathy, traumatic neuroma stump pain or postamputation pain.

<u>Pharmaceutically Acceptable Salts, Pharmaceutical Compositions, Dosage Forms and Routes of Administration</u>

*Pharmaceutically Acceptable Salts*

The methods described and claimed herein comprise administering to a subject Compound 1, or a pharmaceutically acceptable salt thereof. As used herein, the term "pharmaceutically acceptable salt" refers to those salts which are, within the scope of sound medical judgement, suitable for use in contact with the tissues of humans and lower animals without undue toxicity, irritation, allergic response and the like, and are commensurate with a reasonable benefit/risk ratio. A "pharmaceutically acceptable salt" of Compound 1 includes any non-toxic salt that, upon administration to a recipient, is capable of providing, either directly or indirectly, Compound 1 or an inhibitorily active metabolite or residue thereof (e.g., the parent compound of a prodrug). As used herein, the term "inhibitorily active metabolite or residue thereof' means that a metabolite or residue thereof is also an inhibitor of a voltage-gated sodium channel.

Pharmaceutically acceptable salts are well known in the art. For example, S. M. Berge, et al. describe pharmaceutically acceptable salts in detail in J. Pharmaceutical Sciences, 1977, 66, 1-19. Pharmaceutically acceptable salts of Compound 1 include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, and ammonium salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

*Pharmaceutical Compositions*

**In** the methods described and claimed herein, Compound 1, or a pharmaceutically acceptable salt thereof, may be administered in the form of a pharmaceutical composition comprising Compound 1, or a pharmaceutically acceptable salt thereof, and a pharmaceutically acceptable carrier, adjuvant or vehicle.

The term "pharmaceutically acceptable carrier, adjuvant, or vehicle" includes any and all solvents, diluents, or other liquid vehicles, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with Compound 1, or a pharmaceutically acceptable salt thereof, such as by producing any undesirable biological effect or otherwise interacting in a deleterious manner with any other component(s) of the pharmaceutical composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose, starches such as corn starch and potato starch, cellulose and its derivatives such as sodium carboxymethyl cellulose, ethyl cellulose and cellulose acetate, powdered tragacanth, malt, gelatin, talc, excipients such as cocoa butter and suppository waxes, oils such as peanut oil, cottonseed oil, safflower oil, sesame oil, olive oil, corn oil and soybean oil, glycols, such a propylene glycol or polyethylene glycol, esters such as ethyl oleate and ethyl laurate, agar, buffering agents such as magnesium hydroxide and aluminum hydroxide, alginic acid, pyrogen-free water, isotonic saline, Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

*Dosage Forms and Routes of Administration*

The methods described and claimed herein may involve the administration of Compound 1, or a pharmaceutically acceptable salt thereof, by any route of administration effective for treating or lessening the severity of one or more of the pain diseases recited herein. Compound 1, or a pharmaceutically acceptable salt thereof, may be formulated in dosage unit form for ease of administration and uniformity of dosage. The term "dosage unit form," as used herein, refers to a physically discrete unit of agent appropriate for the subject to be treated.

Compound 1, or pharmaceutically acceptable salt thereof, can be administered to humans and other animals orally, rectally, parenterally, intracisternally, intravaginally, intraperitoneally, topically (as by powders, ointments, or drops), bucally, as an oral or nasal spray, or the like, depending on the condition being treated.

Liquid dosage forms for oral administration include, but are not limited to, pharmaceutically acceptable emulsions, microemulsions, solutions, suspensions, syrups and elixirs. In addition to Compound 1, or a pharmaceutically acceptable salt thereof, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

**In** order to prolong the therapeutic effect of Compound 1, it may be desirable to slow the absorption of the compound, or a pharmaceutically acceptable salt thereof, from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing Compound 1, or a pharmaceutically acceptable salt thereof, with suitable nonirritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, Compound 1, or a pharmaceutically acceptable salt thereof, is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or, in a certain part of the intestinal tract, optionally, in a delayed manner. Examples of embedding compositions that can be used include polymeric

substances and waxes.

The active compound or salt can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound or salt may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents.

Dosage forms for topical or transdermal administration of Compound 1, or a pharmaceutically acceptable salt thereof, include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the invention contemplates the use of transdermal patches, which have the added advantage of providing controlled delivery of a compound to the body. Such dosage forms are prepared by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

*Spray Dried Dispersion and Tablet*

Spray drying converts a liquid feed to a dried particulate form. Spray drying generally involves bringing into contact a highly dispersed liquid suspension or solution and a sufficient volume of hot air to promote drying of the liquid droplets. For example, a liquid solution containing Compound 1, or a salt thereof and at least one polymer can be sprayed into a current of warm filtered gas that evaporates the solvent and conveys the dried product to a collector. Evaporated solvent and spent gas are removed from the collector and can be sent to a condenser to capture the solvent. For example, commercial spray dryers are manufactured by Buchi Ltd. and Niro (e.g., the PSD line of spray driers manufactured by Niro) (see, US 2004/0105820, US 2003/0144257).

Techniques and methods for spray drying may be found in Perry's Chemical Engineering Handbook, 6th Ed., R. H. Perry, D. W. Green & J. O. Maloney, eds.), McGraw-Hill book co. (1984); and Marshall "Atomization and Spray-Drying" 50, Chem. Eng. Prog. Monogr. Series 2 (1954).

Additional drying steps may be required after spray-drying to ensure removal of the solvent. Other drying techniques include, but are not limited to, tray drying, fluid bed drying (e.g., from about room temperature to about 100° C.), vacuum drying, microwave drying, rotary drum drying or biconical vacuum drying (e.g., from about room temperature to about 200° C.).

In some embodiments, the solvent(s) used in spray-drying is a volatile solvent. A volatile solvent, for example, can have a boiling point less than 100° C. A mixture of volatile solvents may be used or a mixture of volatile and non-volatile solvents.

Exemplary solvents that could be tested include acetone, cyclohexane, dichloromethane, N,N-dimethylacetamide (DMA), N,N-dimethylformamide (DMF), 1,3-dimethyl-2-imidazolidinone (DMI), dimethyl sulfoxide (DMSO), dioxane, ethyl acetate, ethyl ether, glacial acetic acid (HOAc), methyl ethyl ketone (MEK), N-methyl-2-pyrrolidinone (NMP), methyl tert-butyl ether (MTBE), tetrahydrofuran (THF), pentane, acetonitrile, methanol, ethanol, isopropyl alcohol, isopropyl acetate, DCM, and toluene. Exemplary co-solvents include acetone/DMSO, acetone/DMF, acetone/water, MEK/water, THF/water, dioxane/water. In a two solvent system, the solvents can be present in of from about 0.1% to about 99.9%. In some embodiments, water is a co-solvent with acetone where water is present from about 0.1% to about 15%, for example about 9% to about 11%, e.g., about 10%. In some embodiments, water is a co-solvent with MEK where water is present from about 0.1% to about 15%, for example about 9% to about 11%, e.g., about 10%. In some embodiments the solvent system includes three solvents. In some embodiments where amorphous Compound 1 is a component of a solid amorphous dispersion, the solvent or solvents dissolve both Compound 1 and the at least one polymer. Suitable solvents include those described above, for example, DCM, water, methanol, IPA, and mixtures thereof. In some embodiments, the solvent comprises DCM and methanol.

In some embodiments, the at least one polymer is selected from: hydroxypropyl methylcellulose acetate succinate (HPMCAS), polyvinyl caprolactam-polyvinyl acetate-polyethylene glycol graft co-polymer, and any combination thereof.

In some embodiments, the at least one polymer is HPMCAS.

In some embodiments, the at least one polymer is polyvinyl caprolactam-polyvinyl acetate-polyethylene glycol graft co-polymer. Commercial examples of polyvinyl caprolactam-polyvinyl acetate-polyethylene glycol graft co-polymer include SOLUPLUS®.

In some embodiments, the at least one polymer is a compound of Formula I, where n is about 13, m is about 30,

and l is about 57. The weight averagle molecular weight determined by gel permeation chromatography is about 118,000 g/mol.

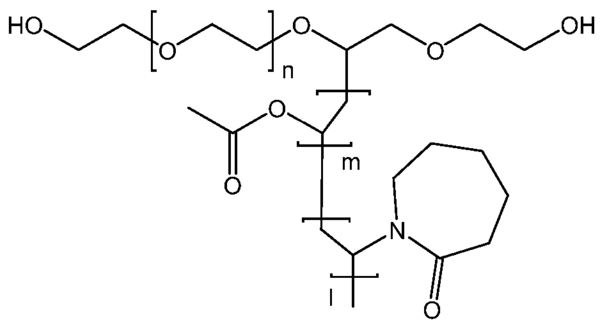

Formula I

In some embodiments, a solid dispersion disclosed herein comprises at least one polymer and Compound 1, or a pharmaceutically acceptable salt thereof. In some embodiments, the solid dispersion comprises about 20 wt % to about 50 wt % of Compound 1, or a pharmaceutically acceptable salt thereof. In some embodiments, the solid dispersion comprises about 20 wt % to about 45 wt %, or about 20 wt % to about 40 wt %, or about 20 wt % to about 35 wt %, or about 20 wt % to about 30 wt %, or about 22 wt % to about 28 wt %, or about 23 wt %, or about 24 wt %, or about 25 wt %, or about 26 wt %, or about 27 wt %, or about 28 wt %, or about 29 wt %, or about 30 wt % of Compound 1 or a pharmaceutically acceptable salt thereof.

In some embodiments, the solid dispersion comprises 5 wt % to 35 wt % of Compound 1, or a pharmaceutically acceptable salt thereof. In some embodiments, the solid dispersion comprises 5 wt % to 30 wt % of Compound 1, or a pharmaceutically acceptable salt thereof. In some embodiments, the solid dispersion comprises 10 wt % to 30 wt % of Compound 1, or a pharmaceutically acceptable salt thereof. In some embodiments, the solid dispersion comprises 15 wt % to 30 wt % of Compound 1, or a pharmaceutically acceptable salt thereof. In some embodiments, the solid dispersion comprises 20 wt % to 30 wt % of Compound 1, or a pharmaceutically acceptable salt thereof.

In some embodiments, the solid dispersion comprises 5 wt % to 35 wt % of Compound 1. In some embodiments, the solid dispersion comprises 5 wt % to 30 wt % of Compound 1. In some embodiments, the solid dispersion comprises 10 wt % to 30 wt % of Compound 1. In some embodiments, the solid dispersion comprises 15 wt % to 30 wt % of Compound 1. In some embodiments, the solid dispersion comprises 20 wt % to 30 wt % of Compound 1.

In some embodiments, the solid dispersion comprises about 5 wt %, about 6 wt %, about 7 wt %, about 8 wt %, about 9 wt %, about 10 wt %, about 11 wt %, about 12 wt %, about 13 wt %, about 14 wt %, about 15 wt %, about 16 wt %, about 17 wt %, about 18 wt %, about 19 wt %, about 20 wt %, about 21 wt %, about 22 wt %, about 23 wt %, about 24 wt %, about 25 wt %, about 26 wt %, about 27 wt %, about 28 wt %, about 29 wt %, about 30 wt %, about 31 wt %, about 32 wt %, about 33 wt %, about 34 wt %, or about 35 wt % of Compound 1, or a pharmaceutically acceptable salt thereof. In some embodiments, the solid dispersion comprises about 20 wt %, about 21 wt %, about 22 wt %, about 23 wt %, about 24 wt %, about 25 wt %, about 26 wt %, about 27 wt %, about 28 wt %, about 29 wt %, or about 30 wt % of Compound 1, or a pharmaceutically acceptable salt thereof. In some embodiments, the solid dispersion comprises about 20 wt % of Compound 1, or a pharmaceutically acceptable salt thereof. In some embodiments, the solid dispersion comprises about 25 wt % of Compound 1, or a pharmaceutically acceptable salt thereof. In some embodiments, the solid dispersion comprises about 30 wt % of Compound 1, or a pharmaceutically acceptable salt thereof. In some embodiments, the solid dispersion comprises about 35 wt % of Compound 1, or a pharmaceutically acceptable salt thereof.

In some embodiments, the solid dispersion comprises about 5 wt %, about 6 wt %, about 7 wt %, about 8 wt %, about 9 wt %, about 10 wt %, about 11 wt %, about 12 wt %, about 13 wt %, about 14 wt %, about 15 wt %, about 16 wt %, about 17 wt %, about 18 wt %, about 19 wt %, about 20 wt %, about 21 wt %, about 22 wt %, about 23 wt %, about 24 wt %, about 25 wt %, about 26 wt %, about 27 wt %, about 28 wt %, about 29 wt %, about 30 wt %, about 31 wt %, about 32 wt %, about 33 wt %, about 34 wt %, or about 35 wt % of Compound 1. In some embodiments, the solid dispersion comprises about 20 wt %, about 21 wt %, about 22 wt %, about 23 wt %, about 24 wt %, about 25 wt %, about 26 wt %, about 27 wt %, about 28 wt %, about 29 wt %, or about 30 wt % of Compound 1. In some embodiments, the solid dispersion comprises about 20 wt % of Compound 1. In some embodiments, the solid dispersion comprises about 25 wt % of Compound 1. In some embodiments, the solid dispersion comprises about 30 wt % of Compound 1. In some embodiments, the solid dispersion comprises about 35 wt % of Compound 1.

In some embodiments, the solid dispersion comprises 5 wt %, 6 wt %, 7 wt %, 8 wt %, 9 wt %, 10 wt %, 11 wt %, 12 wt %, 13 wt %, 14 wt %, 15 wt %, 16 wt %, 17 wt %, 18 wt %, 19 wt %, 20 wt %, 21 wt %, 22 wt %, 23 wt %, 24 wt %, 25 wt %, 26 wt %, 27 wt %, 28 wt %, 29 wt %, 30 wt %, 31 wt %, 32 wt %, 33 wt %, 34 wt %, or 35 wt % of Compound 1, or a

pharmaceutically acceptable salt thereof. In some embodiments, the solid dispersion comprises 20 wt %, 21 wt %, 22 wt %, 23 wt %, 24 wt %, 25 wt %, 26 wt %, 27 wt %, 28 wt %, 29 wt %, or 30 wt % of Compound 1, or a pharmaceutically acceptable salt thereof. In some embodiments, the solid dispersion comprises 20 wt % of Compound 1, or a pharmaceutically acceptable salt thereof. In some embodiments, the solid dispersion comprises 25 wt % of Compound 1, or a pharmaceutically acceptable salt thereof. In some embodiments, the solid dispersion comprises 30 wt % of Compound 1, or a pharmaceutically acceptable salt thereof. In some embodiments, the solid dispersion comprises 35 wt % of Compound 1, or a pharmaceutically acceptable salt thereof.

In some embodiments, the solid dispersion comprises 5 wt %, 6 wt %, 7 wt %, 8 wt %, 9 wt %, 10 wt %, 11 wt %, 12 wt %, 13 wt %, 14 wt %, 15 wt %, 16 wt %, 17 wt %, 18 wt %, 19 wt %, 20 wt %, 21 wt %, 22 wt %, 23 wt %, 24 wt %, 25 wt %, 26 wt %, 27 wt %, 28 wt %, 29 wt %, 30 wt %, 31 wt %, 32 wt %, 33 wt %, 34 wt %, or 35 wt % of Compound 1. In some embodiments, the solid dispersion comprises 20 wt %, 21 wt %, 22 wt %, 23 wt %, 24 wt %, 25 wt %, 26 wt %, 27 wt %, 28 wt %, 29 wt %, or 30 wt % of Compound 1. In some embodiments, the solid dispersion comprises 20 wt % of Compound 1. In some embodiments, the solid dispersion comprises 25 wt % of Compound 1. In some embodiments, the solid dispersion comprises 30 wt % of Compound 1. In some embodiments, the solid dispersion comprises 35 wt % of Compound 1.

In some embodiments, the solid dispersion comprises about 50 wt % to about 80 wt % of the polymer. In some embodiments, the solid dispersion comprises about 55 wt % to about 80 wt %, or about 60 wt % to about 80 wt %, or about 65 wt % to about 80 wt %, or about 70 wt % to about 80 wt %, or about 71 wt %, or about 72 wt %, or about 73 wt %, or about 74 wt %, or about 75 wt %, or about 76 wt %, or about 77 wt %, or about 78 wt %, or about 79 wt %, or about 80 wt % of the polymer.

In some embodiments, the solid dispersion comprises about 65 wt % to about 95 wt % of the at least one polymer. In some embodiments, the solid dispersion comprises about 70 wt % to about 95 wt % of the at least one polymer. In some embodiments, the solid dispersion comprises about 70 wt % to about 90 wt % of the at least one polymer. In some embodiments, the solid dispersion comprises about 70 wt % to about 85 wt % of the at least one polymer. In some embodiments, the solid dispersion comprises about 70 wt % to about 80 wt % of the at least one polymer.

In some embodiments, the solid dispersion comprises 65 wt % to 95 wt % of the at least one polymer. In some embodiments, the solid dispersion comprises 70 wt % to 95 wt % of the at least one polymer. In some embodiments, the solid dispersion comprises 70 wt % to 90 wt % of the at least one polymer. In some embodiments, the solid dispersion comprises 70 wt % to 85 wt % of the at least one polymer. In some embodiments, the solid dispersion comprises 70 wt % to 80 wt % of the at least one polymer.

In some embodiments, the solid dispersion comprises about 65 wt %, about 66 wt %, about 67 wt %, about 68 wt %, about 69 wt %, about 70 wt %, about 71 wt %, about 72 wt %, about 73 wt %, about 74 wt %, about 75 wt %, about 76 wt %, about 77 wt %, about 78 wt %, about 79 wt %, about 80 wt %, about 81 wt %, about 82 wt %, about 83 wt %, about 84 wt %, about 85 wt %, about 86 wt %, about 87 wt %, about 88 wt %, about 89 wt %, about 90 wt %, about 91 wt %, about 92 wt %, about 93 wt %, about 94 wt %, or about 95 wt % of the at least one polymer. In some embodiments, the solid dispersion comprises about 70 wt %, about 71 wt %, about 72 wt %, about 73 wt %, about 74 wt %, about 75 wt %, about 76 wt %, about 77 wt %, about 78 wt %, about 79 wt %, or about 80 wt % of the at least one polymer. In some embodiments, the solid dispersion comprises about 65 wt % of the at least one polymer. In some embodiments, the solid dispersion comprises about 70 wt % of the at least one polymer. In some embodiments, the solid dispersion comprises about 75 wt % of the at least one polymer. In some embodiments, the solid dispersion comprises about 80 wt % of the at least one polymer.

In some embodiments, the solid dispersion comprises 65 wt %, 66 wt %, 67 wt %, 68 wt %, 69 wt %, 70 wt %, 71 wt %, 72 wt %, 73 wt %, 74 wt %, 75 wt %, 76 wt %, 77 wt %, 78 wt %, 79 wt %, 80 wt %, 81 wt %, 82 wt %, 83 wt %, 84 wt %, 85 wt %, 86 wt %, 87 wt %, 88 wt %, 89 wt %, 90 wt %, 91 wt %, 92 wt %, 93 wt %, 94 wt %, or 95 wt % of the at least one polymer. In some embodiments, the solid dispersion comprises 70 wt %, 71 wt %, 72 wt %, 73 wt %, 74 wt %, 75 wt %, 76 wt %, 77 wt %, 78 wt %, 79 wt %, or 80 wt % of the at least one polymer. In some embodiments, the solid dispersion comprises 65 wt % of the at least one polymer. In some embodiments, the solid dispersion comprises 70 wt % of the at least one polymer. In some embodiments, the solid dispersion comprises 75 wt % of the at least one polymer. In some embodiments, the solid dispersion comprises 80 wt % of the at least one polymer.

In some embodiments, the solid dispersion comprises about 65 wt % to about 95 wt % of the at least one polymer and about 5 wt % to about 35 wt % of Compound **1,** or a pharmaceutically acceptable salt thereof. In some embodiments, the solid dispersion comprises about 70 wt % to about 95 wt % of the at least one polymer and about 5 wt % to about 30 wt % of Compound 1, or a pharmaceutically acceptable salt thereof. In some embodiments, the solid dispersion comprises about 70 wt % to about 90 wt % of the at least one polymer and about 10 wt % to about 30 wt % of Compound 1, or a pharmaceutically acceptable salt thereof. In some embodiments, the solid dispersion comprises about 70 wt % to about 85 wt % of the at least one polymer and about 15 wt % to about 30 wt % of Compound 1, or a pharmaceutically acceptable salt thereof. In some embodiments, the solid dispersion comprises about 70 wt % to about 80 wt % of the at least one polymer and about 20 wt % to about 30 wt % of Compound 1, or a pharmaceutically acceptable salt thereof.

In some embodiments, the solid dispersion comprises about 65 wt % to about 95 wt % of the at least one polymer and about 5 wt % to about 35 wt % of Compound 1. In some embodiments, the solid dispersion comprises about 70 wt % to

about 95 wt % of the at least one polymer and about 5 wt % to about 30 wt % of Compound 1. In some embodiments, the solid dispersion comprises about 70 wt % to about 90 wt % of the at least one polymer and about 10 wt % to about 30 wt % of Compound 1. In some embodiments, the solid dispersion comprises about 70 wt % to about 85 wt % of the at least one polymer and about 15 wt % to about 30 wt % of Compound 1. In some embodiments, the solid dispersion comprises about 70 wt % to about 80 wt % of the at least one polymer and about 20 wt % to about 30 wt % of Compound 1.

In some embodiments, the solid dispersion comprises 65 wt % to 95 wt % of the at least one polymer and 5 wt % to 35 wt % of Compound 1, or a pharmaceutically acceptable salt thereof. In some embodiments, the solid dispersion comprises 70 wt % to 95 wt % of the at least one polymer and 5 wt % to 30 wt % of Compound 1, or a pharmaceutically acceptable salt thereof. In some embodiments, the solid dispersion comprises 70 wt % to 90 wt % of the at least one polymer and 10 wt % to 30 wt % of Compound 1, or a pharmaceutically acceptable salt thereof. In some embodiments, the solid dispersion comprises 70 wt % to 85 wt % of the at least one polymer and 15 wt % to 30 wt % of Compound 1, or a pharmaceutically acceptable salt thereof. In some embodiments, the solid dispersion comprises 70 wt % to 80 wt % of the at least one polymer and 20 wt % to 30 wt % of Compound 1, or a pharmaceutically acceptable salt thereof.

In some embodiments, the solid dispersion comprises 65 wt % to 95 wt % of the at least one polymer and 5 wt % to 35 wt % of Compound 1. In some embodiments, the solid dispersion comprises 70 wt % to 95 wt % of the at least one polymer and 5 wt % to 30 wt % of Compound 1. In some embodiments, the solid dispersion comprises 70 wt % to 90 wt % of the at least one polymer and 10 wt % to 30 wt % of Compound 1. In some embodiments, the solid dispersion comprises 70 wt % to 85 wt % of the at least one polymer and 15 wt % to 30 wt % of Compound 1. In some embodiments, the solid dispersion comprises 70 wt % to 80 wt % of the at least one polymer and 20 wt % to 30 wt % of Compound 1.

In some embodiments, the solid dispersion comprises about 65 wt % to about 95 wt % of the at least one polymer and about 5 wt % to about 35 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises about 70 wt % to about 95 wt % of the at least one polymer and about 5 wt % to about 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises about 70 wt % to about 90 wt % of the at least one polymer and about 10 wt % to about 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises about 70 wt % to about 85 wt % of the at least one polymer and about 15 wt % to about 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises about 70 wt % to about 80 wt % of the at least one polymer and about 20 wt % to about 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous.

In some embodiments, the solid dispersion comprises about 65 wt % to about 95 wt % of the at least one polymer and about 5 wt % to about 35 wt % of Compound 1, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises about 70 wt % to about 95 wt % of the at least one polymer and about 5 wt % to about 30 wt % of Compound 1, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises about 70 wt % to about 90 wt % of the at least one polymer and about 10 wt % to about 30 wt % of Compound **1,** wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises about 70 wt % to about 85 wt % of the at least one polymer and about 15 wt % to about 30 wt % of Compound 1, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises about 70 wt % to about 80 wt % of the at least one polymer and about 20 wt % to about 30 wt % of Compound 1, wherein the at least one polymer is selected from HPMCAS and polyvinyl caprolactam-polyvinyl acetate-polyethylene glycol graft co-polymer, and wherein the Compound 1 is substantially amorphous.

In some embodiments, the solid dispersion comprises 65 wt % to 95 wt % of the at least one polymer and 5 wt % to 35 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises

70 wt % to 95 wt % of the at least one polymer and 5 wt % to 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises 70 wt % to 90 wt % of the at least one polymer and 10 wt % to 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises 70 wt % to 85 wt % of the at least one polymer and 15 wt % to 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises 70 wt % to 80 wt % of the at least one polymer and 20 wt % to 30 wt % of Compound 1, or a pharmaceutically acceptable salt thereof, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous.

In some embodiments, the solid dispersion comprises 65 wt % to 95 wt % of the at least one polymer and 5 wt % to 35 wt % of Compound 1, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises 70 wt % to 95 wt % of the at least one polymer and 5 wt % to 30 wt % of Compound 1, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises 70 wt % to 90 wt % of the at least one polymer and 10 wt % to 30 wt % of Compound 1, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises 70 wt % to 85 wt % of the at least one polymer and 15 wt % to 30 wt % of Compound 1, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises 70 wt % to 80 wt % of the at least one polymer and 20 wt % to 30 wt % of Compound 1, wherein the at least one polymer is selected from HPMCAS and polyvinyl caprolactam-polyvinyl acetate-polyethylene glycol graft co-polymer, and wherein the Compound 1 is substantially amorphous.

In some embodiments, the solid dispersion comprises about 25 wt % of Compound 1, or a pharmaceutically acceptable thereof, and about 75 wt % of the at least one polymer. In some embodiments, the solid dispersion comprises about 25 wt % of Compound 1 and about 75 wt % of the at least one polymer. In some embodiments, the solid dispersion comprises 25 wt % of Compound 1, or a pharmaceutically acceptable thereof, and 75 wt % of the at least one polymer. In some embodiments, the solid dispersion comprises 25 wt % of Compound 1 and 75 wt % of the at least one polymer.

In some embodiments, the solid dispersion comprises about 25 wt % of Compound 1, or a pharmaceutically acceptable thereof, and about 75 wt % of the at least one polymer, wherein the at least one polymer is selected from HPMCAS and polyvinyl caprolactam-polyvinyl acetate-polyethylene glycol graft co-polymer, and wherein the Compound 1, or pharmaceutically acceptable salt thereof, is substantially amorphous. In some embodiments, the solid dispersion comprises about 25 wt % of Compound 1 and about 75 wt % of the at least one polymer, wherein the at least one polymer is selected from HPMCAS and polyvinyl caprolactam-polyvinyl acetate-polyethylene glycol graft co-polymer, and wherein the Compound 1 is substantially amorphous. In some embodiments, the solid dispersion comprises 25 wt % of Compound 1, or a pharmaceutically acceptable thereof, and 75 wt % of the at least one polymer, wherein the at least one polymer is selected from HPMCAS and polyvinyl caprolactam-polyvinyl acetate-polyethylene glycol graft co-polymer, and wherein the Compound 1, or pharmaceutically acceptable salt, is substantially amorphous. In some embodiments, the solid dispersion comprises 25 wt % of Compound 1 and 75 wt % of the at least one polymer, wherein the at least one polymer is selected from HPMCAS and polyvinyl caprolactam-polyvinyl acetate-polyethylene glycol graft co-polymer, and wherein the Compound 1 is substantially amorphous.

In some embodiments, Compound 1 in the solid dispersion is substantially amorphous. In some embodiments, Compound 1 in the solid dispersion is amorphous.

A method of preparing a spray dried dispersion comprising Compound 1, or a pharmaceutically acceptable salt thereof, is provided. The method includes mixing Compound 1, or a pharmaceutically acceptable salt thereof, in a solvent (or mixture of solvents) with at least one polymer.

The solvent can be any solvent as described above, for example, in some embodiments, the solvent comprises a mixture of DCM and methanol.

In one embodiment, the method further comprises filtering the mixture before it is forced through the nozzle. In one embodiment, the method further comprises applying heat to the mixture as it enters the nozzle. In one embodiment, the nozzle comprises an inlet and an outlet, and the inlet is heated to a temperature that is greater than the boiling point of the solvent. It is understood that in certain embodiments, the temperature may be below the boiling point of the solvent,

such as, for example under high pressure conditions.

In one embodiment, the spray dryer is heated to a temperature of from about 40 °C to about 150 °C. In one embodiment, the spray dryer is heated to a temperature of from about 40 °C to about 60 °C, or from about 45 °C to about 55 °C, or about 48 °C. In one embodiment, the mixture is forced through the nozzle by a pressurized gas. In one embodiment, the pressurized gas comprises molecular nitrogen. At the nozzle the gas flow can be about 4 kg/hour to about 5 kg/hour.

In some embodiments, provided herein is a pharmaceutical composition that includes the solid dispersion disclosed herein. In some embodiments, the pharmaceutical composition can include one or more excipients. Examples of excipients include, but are not limited to, fillers, disintegrants, and lubricants.

Examples of fillers include, but are not limited to, microcrystalline cellulose, lactose monohydrate, mannitol, and mixtures of the same. In some embodiments, the filler comprises microcrystalline cellulose. In some embodiments, the filler comprises lactose monohydrate. In some embodiments, the filler comprises mannitol. In some embodiments, the filler comprises a mixture of microcrystalline cellulose and lactose monohydrate. In some embodiments, the filler comprises microcrystalline cellulose, wherein the microcrystalline cellulose is Avicel PhH101. In some embodiments, the filler comprises microcrystalline cellulose, wherein the microcrystalline cellulose is Avicel PH102. In some embodiments, the filler comprises microcrystalline cellulose, wherein the microcrystalline cellulose is a combination of Avicel PH101 and Avicel PH102.

Examples of suitable disintegrants include, but are not limited to, croscarmellose sodium, crospovidone, and mixtures thereof. In some embodiments, the disintegrant comprises croscarmellose sodium. In some embodiments, the disintegrant comprises crospovidone.

Examples of suitable lubricants include, but are not limited to, sodium stearyl fumarate, magnesium stearate, and mixtures thereof. In some embodiments, the lubricant comprises sodium stearyl fumarate. In some embodiments, the lubricant comprises magnesium stearate.

In some embodiments, the pharmaceutical composition comprises Compound 1 or a salt thereof, at least one polymer, at least one filler, at least one lubricant, and at least one disintegrant. In some embodiments, the filler comprises microcrystalline cellulose and lactose monohydrate, the disintegrant comprises croscarmellose sodium, and the lubricant comprises sodium stearyl fumarate.

In some embodiments, disclosed herein is a pharmaceutical composition comprising about 72.5 wt % of at least one filler, about 4.5 wt % of at least one disintegrant, and about 3 wt % of at least one lubricant.

In some embodiments, the pharmaceutical composition comprises about 1 to about 50 mg of Compound 1. In some embodiments, the pharmaceutical composition comprises about 1 to about 45 mg, or about 1 to about 40 mg, or about 1 to about 35 mg, or about 5 to about 40 mg, or about 5 to about 35 mg, or about 5 to about 30 mg, or about 5 mg, or about 10 mg, or about 15 mg, or about 20 mg, or about 25 mg, or about 30 mg, or about 40 mg, or about 50 mg of Compound 1. In some embodiments, the pharmaceutical composition comprises about 10 mg of Compound 1.

**In** some embodiments, disclosed herein is a pharmaceutical composition comprising about 40 to about 60 wt % of Compound 1 spray-dried dispersion, about 35 to about 55 wt % of at least one filler, about 1 to about 6 wt % of at least one disintegrant, and about 0.5 to about 2 wt % of at least one lubricant. In some embodiments, disclosed herein is a pharmaceutical composition comprising about 40 to about 60 wt % of Compound 1 spray-dried dispersion, about 35 to about 55 wt % of at least one filler, about 1 to about 6 wt % of at least one disintegrant, and about 0.5 to about 2 wt % of at least one lubricant, wherein the Compound 1 spray-dried dispersion comprises at least one polymer and about 20 to about 50 wt % Compound 1. In some embodiments, disclosed herein is a pharmaceutical composition comprising about 40 to about 60 wt % of Compound 1 spray-dried dispersion, about 35 to about 55 wt % of at least one filler, about 1 to about 6 wt % of at least one disintegrant, about 0.5 to about 2 wt % of at least one lubricant, and about 2 to about 4.5 wt % of at least one coating. In some embodiments, disclosed herein is a pharmaceutical composition comprising about 40 to about 60 wt % of Compound 1 spray-dried dispersion, about 35 to about 55 wt % of at least one filler, about 1 to about 6 wt % of at least one disintegrant, about 0.5 to about 2 wt % of at least one lubricant, and about 2 to about 4.5 wt % of at least one coating, wherein the Compound 1 spray-dried dispersion comprises at least one polymer and about 20 to about 50 wt % Compound 1.

**In** some embodiments, disclosed herein is a pharmaceutical composition comprising 40 to 60 wt % of Compound 1 spray-dried dispersion, 35 to 55 wt % of at least one filler, 1 to 6 wt % of at least one disintegrant, and 0.5 to 2 wt % of at least one lubricant. In some embodiments, disclosed herein is a pharmaceutical composition comprising 40 to 60 wt % of Compound 1 spray-dried dispersion, 35 to 55 wt % of at least one filler, 1 to 6 wt % of at least one disintegrant, and 0.5 to 2 wt % of at least one lubricant, wherein the Compound 1 spray-dried dispersion comprises at least one polymer and about 20 to about 50 wt % Compound 1. In some embodiments, disclosed herein is a pharmaceutical composition comprising 40 to 60 wt % of Compound 1 spray-dried dispersion, 35 to 55 wt % of at least one filler, 1 to 6 wt % of at least one disintegrant, 0.5 to 2 wt % of at least one lubricant, and 2 to 4.5 wt % of at least one coating. In some embodiments, disclosed herein is a pharmaceutical composition comprising 40 to 60 wt % of Compound 1 spray-dried dispersion, 35 to 55 wt % of at least one filler, 1 to 6 wt % of at least one disintegrant, 0.5 to 2 wt % of at least one lubricant, and 2 to 4.5 wt % of at least one coating, wherein the Compound 1 spray-dried dispersion comprises at least one polymer and about 20 to about 50 wt % Compound

1.

**In** some embodiments, disclosed herein is a pharmaceutical composition comprising about 40 to about 60 wt % of Compound 1 spray-dried dispersion, about 35 to about 55 wt % of microcrystalline cellulose, about 1 to about 6 wt % of croscarmellose sodium, and about 0.5 to about 2 wt % of magnesium stearate. In some embodiments, disclosed herein is a pharmaceutical composition comprising about 40 to about 60 wt % of Compound 1 spray-dried dispersion, about 35 to about 55 wt % of microcrystalline cellulose, about 1 to about 6 wt % of croscarmellose sodium, and about 0.5 to about 2 wt % of magnesium stearate, wherein the Compound 1 spray-dried dispersion comprises hydroxypropyl methylcellulose acetate succinate and about 20 to about 50 wt % Compound 1. In some embodiments, disclosed herein is a pharmaceutical composition comprising about 40 to about 60 wt % of Compound 1 spray-dried dispersion, about 35 to about 55 wt % of microcrystalline cellulose, about 1 to about 6 wt % of croscarmellose sodium, about 0.5 to about 2 wt % of magnesium stearate, and about 2 to about 4.5 wt % of Opadry blue. In some embodiments, disclosed herein is a pharmaceutical composition comprising about 40 to about 60 wt % of Compound 1 spray-dried dispersion, about 35 to about 55 wt % of microcrystalline cellulose, about 1 to about 6 wt % of croscarmellose sodium, about 0.5 to about 2 wt % of magnesium stearate, and about 2 to about 4.5 wt % of Opadry blue, wherein the Compound 1 spray-dried dispersion comprises hydroxypropyl methylcellulose acetate succinate and about 20 to about 50 wt % Compound 1.

**In** some embodiments, disclosed herein is a pharmaceutical composition comprising 40 to 60 wt % of Compound 1 spray-dried dispersion, 35 to 55 wt % of microcrystalline cellulose, 1 to 6 wt % of croscarmellose sodium, and 0.5 to 2 wt % of magnesium stearate. In some embodiments, disclosed herein is a pharmaceutical composition comprising 40 to 60 wt % of Compound 1 spray-dried dispersion, 35 to 55 wt % of microcrystalline cellulose, 1 to 6 wt % of croscarmellose sodium, and 0.5 to 2 wt % of magnesium stearate, wherein the Compound 1 spray-dried dispersion comprises hydroxypropyl methylcellulose acetate succinate and about 20 to about 50 wt % Compound 1. In some embodiments, disclosed herein is a pharmaceutical composition comprising 40 to 60 wt % of Compound 1 spray-dried dispersion, 35 to 55 wt % of microcrystalline cellulose, 1 to 6 wt % of croscarmellose sodium, 0.5 to 2 wt % of magnesium stearate, and 2 to 4.5 wt % of Opadry blue. In some embodiments, disclosed herein is a pharmaceutical composition comprising 40 to 60 wt % of Compound 1 spray-dried dispersion, 35 to 55 wt % of microcrystalline cellulose, 1 to 6 wt % of croscarmellose sodium, 0.5 to 2 wt % of magnesium stearate, and 2 to 4.5 wt % of Opadry blue, wherein the Compound 1 spray-dried dispersion comprises hydroxypropyl methylcellulose acetate succinate and about 20 to about 50 wt % Compound 1.

**In** some embodiments, disclosed herein is a pharmaceutical composition comprising about 45 to about 55 wt % of Compound 1 spray-dried dispersion, about 40 to about 50 wt % of at least one filler, about 1.5 to about 4 wt % of at least one disintegrant, and about 0.75 to about 1.5 wt % of at least one lubricant. In some embodiments, disclosed herein is a pharmaceutical composition comprising about 45 to about 55 wt % of Compound 1 spray-dried dispersion, about 40 to about 50 wt % of at least one filler, about 1.5 to about 4 wt % of at least one disintegrant, and about 0.75 to about 1.5 wt % of at least one lubricant, wherein the Compound 1 spray-dried dispersion comprises at least one polymer and about 20 to about 35 wt % Compound 1. In some embodiments, disclosed herein is a pharmaceutical composition comprising about 45 to about 55 wt % of Compound 1 spray-dried dispersion, about 40 to about 50 wt % of at least one filler, about 1.5 to about 4 wt % of at least one disintegrant, about 0.75 to about 1.5 wt % of at least one lubricant, and about 2.5 to about 3.5 wt % of at least one coating. In some embodiments, disclosed herein is a pharmaceutical composition comprising about 40 to about 55 wt % of Compound 1 spray-dried dispersion, about 40 to about 50 wt % of at least one filler, about 1.5 to about 4 wt % of at least one disintegrant, about 0.75 to about 1.5 wt % of at least one lubricant, and about 2.5 to about 3.5 wt % of at least one coating, wherein the Compound 1 spray-dried dispersion comprises at least one polymer and about 20 to about 35 wt % Compound 1.

**In** some embodiments, disclosed herein is a pharmaceutical composition comprising 45 to 55 wt % of Compound 1 spray-dried dispersion, 40 to 50 wt % of at least one filler, 1.5 to 4 wt % of at least one disintegrant, and 0.75 to 1.5 wt % of at least one lubricant. In some embodiments, disclosed herein is a pharmaceutical composition comprising 45 to 55 wt % of Compound 1 spray-dried dispersion, 40 to 50 wt % of at least one filler, 1.5 to 4 wt % of at least one disintegrant, and 0.75 to 1.5 wt % of at least one lubricant, wherein the Compound 1 spray-dried dispersion comprises at least one polymer and about 20 to about 35 wt % Compound 1. In some embodiments, disclosed herein is a pharmaceutical composition comprising 45 to 55 wt % of Compound 1 spray-dried dispersion, 40 to 50 wt % of at least one filler, 1.5 to 4 wt % of at least one disintegrant, 0.75 to 1.5 wt % of at least one lubricant, and 2.5 to 3.5 wt % of at least one coating. In some embodiments, disclosed herein is a pharmaceutical composition comprising 45 to 55 wt % of Compound 1 spray-dried dispersion, 40 to 50 wt % of at least one filler, 1.5 to 4 wt % of at least one disintegrant, 0.75 to 1.5 wt % of at least one lubricant, and 2.5 to 3.5 wt % of at least one coating, wherein the Compound 1 spray-dried dispersion comprises at least one polymer and about 20 to about 35 wt % Compound 1.

**In** some embodiments, disclosed herein is a pharmaceutical composition comprising about 45 to about 55 wt % of Compound 1 spray-dried dispersion, about 40 to about 50 wt % of microcrystalline cellulose, about 1.5 to about 4 wt % of croscarmellose sodium, and about 0.75 to about 1.5 wt % of magnesium stearate. In some embodiments, disclosed herein is a pharmaceutical composition comprising about 45 to about 55 wt % of Compound 1 spray-dried dispersion, about 40 to about 50 wt % of microcrystalline cellulose, about 1.5 to about 4 wt % of croscarmellose sodium, and about 0.75 to about

1.5 wt % of magnesium stearate, wherein the Compound 1 spray-dried dispersion comprises hydroxypropyl methylcellulose acetate succinate and about 20 to about 35 wt % Compound 1. In some embodiments, disclosed herein is a pharmaceutical composition comprising about 45 to about 55 wt % of Compound 1 spray-dried dispersion, about 40 to about 50 wt % of microcrystalline cellulose, about 1.5 to about 4 wt % of croscarmellose sodium, about 0.75 to about 1.5 wt % of magnesium stearate, and about 2.5 to about 3.5 wt % of Opadry blue. In some embodiments, disclosed herein is a pharmaceutical composition comprising about 45 to about 55 wt % of Compound 1 spray-dried dispersion, about 40 to about 50 wt % of microcrystalline cellulose, about 1.5 to about 4 wt % of croscarmellose sodium, about 0.75 to about 1.5 wt % of magnesium stearate, and about 2.5 to about 3.5 wt % of Opadry blue, wherein the Compound 1 spray-dried dispersion comprises hydroxypropyl methylcellulose acetate succinate and about 20 to about 35 wt % Compound 1.

**In** some embodiments, disclosed herein is a pharmaceutical composition comprising 45 to 55 wt % of Compound 1 spray-dried dispersion, 40 to 50 wt % of microcrystalline cellulose, 1.5 to 4 wt % of croscarmellose sodium, and 0.75 to 1.5 wt % of magnesium stearate. In some embodiments, disclosed herein is a pharmaceutical composition comprising 45 to 55 wt % of Compound 1 spray-dried dispersion, 40 to 50 wt % of microcrystalline cellulose, 1.5 to 4 wt % of croscarmellose sodium, and 0.75 to 1.5 wt % of magnesium stearate, wherein the Compound 1 spray-dried dispersion comprises hydroxypropyl methylcellulose acetate succinate and about 20 to about 35 wt % Compound 1. In some embodiments, disclosed herein is a pharmaceutical composition comprising 45 to 55 wt % of Compound 1 spray-dried dispersion, 40 to 50 wt % of microcrystalline cellulose, 1.5 to 4 wt % of croscarmellose sodium, 0.75 to 1.5 wt % of magnesium stearate, and 2.5 to 3.5 wt % of Opadry blue. In some embodiments, disclosed herein is a pharmaceutical composition comprising 45 to 55 wt % of Compound 1 spray-dried dispersion, 40 to 50 wt % of microcrystalline cellulose, 1.5 to 4 wt % of croscarmellose sodium, 0.75 to 1.5 wt % of magnesium stearate, and 2.5 to 3.5 wt % of Opadry blue, wherein the Compound 1 spray-dried dispersion comprises hydroxypropyl methylcellulose acetate succinate and about 20 to about 35 wt % Compound 1.

**In** some embodiments, disclosed herein is a pharmaceutical composition comprising about 47.5 to about 52.5 wt % of Compound 1 spray-dried dispersion, about 42.5 to about 47.5 wt % of at least one filler, about 2.5 to about 3.5 wt % of at least one disintegrant, and about 0.75 to about 1.25 wt % of at least one lubricant. In some embodiments, disclosed herein is a pharmaceutical composition comprising about 47.5 to about 52.5 wt % of Compound 1 spray-dried dispersion, about 42.5 to about 47.5 wt % of at least one filler, about 2.5 to about 3.5 wt % of at least one disintegrant, and about 0.75 to about 1.25 wt % of at least one lubricant, wherein the Compound 1 spray-dried dispersion comprises at least one polymer and about 20 to about 30 wt % Compound 1. In some embodiments, disclosed herein is a pharmaceutical composition comprising about 47.5 to about 52.5 wt % of Compound 1 spray-dried dispersion, about 42.5 to about 47.5 wt % of at least one filler, about 2.5 to about 3.5 wt % of at least one disintegrant, about 0.75 to about 1.25 wt % of at least one lubricant, and about 2.75 to about 3.25 wt % of at least one coating. In some embodiments, disclosed herein is a pharmaceutical composition comprising about 47.5 to about 52.5 wt % of Compound 1 spray-dried dispersion, about 42.5 to about 47.5 wt % of at least one filler, about 2.5 to about 3.5 wt % of at least one disintegrant, about 0.75 to about 1.25 wt % of at least one lubricant, and about 2.75 to about 3.25 wt % of at least one coating, wherein the Compound 1 spray-dried dispersion comprises at least one polymer and about 20 to about 30 wt % Compound 1.

**In** some embodiments, disclosed herein is a pharmaceutical composition comprising 47.5 to 52.5 wt % of Compound 1 spray-dried dispersion, 42.5 to 47.5 wt % of at least one filler, 2.5 to 3.5 wt % of at least one disintegrant, and 0.75 to 1.25 wt % of at least one lubricant. In some embodiments, disclosed herein is a pharmaceutical composition comprising 47.5 to 52.5 wt % of Compound 1 spray-dried dispersion, 42.5 to 47.5 wt % of at least one filler, 2.5 to 3.5 wt % of at least one disintegrant, and 0.75 to 1.25 wt % of at least one lubricant, wherein the Compound 1 spray-dried dispersion comprises at least one polymer and about 20 to about 30 wt % Compound 1. In some embodiments, disclosed herein is a pharmaceutical composition comprising 47.5 to 52.5 wt % of Compound 1 spray-dried dispersion, 42.5 to 47.5 wt % of at least one filler, 2.5 to 3.5 wt % of at least one disintegrant, 0.75 to 1.25 wt % of at least one lubricant, and 2.75 to 3.25 wt % of at least one coating. In some embodiments, disclosed herein is a pharmaceutical composition comprising 47.5 to 52.5 wt % of Compound 1 spray-dried dispersion, 42.5 to 47.5 wt % of at least one filler, 2.5 to 3.5 wt % of at least one disintegrant, 0.75 to 1.25 wt % of at least one lubricant, and 2.75 to 3.25 wt % of at least one coating, wherein the Compound 1 spray-dried dispersion comprises at least one polymer and about 20 to about 30 wt % Compound 1.

**In** some embodiments, disclosed herein is a pharmaceutical composition comprising about 47.5 to about 52.5 wt % of Compound 1 spray-dried dispersion, about 42.5 to about 47.5 wt % of microcrystalline cellulose, about 2.5 to about 3.5 wt % of croscarmellose sodium, and about 0.75 to about 1.25 wt % of magnesium stearate. In some embodiments, disclosed herein is a pharmaceutical composition comprising about 47.5 to about 52.5 wt % of Compound 1 spray-dried dispersion, about 42.5 to about 47.5 wt % of microcrystalline cellulose, about 2.5 to about 3.5 wt % of croscarmellose sodium, and about 0.75 to about 1.25 wt % of magnesium stearate, wherein the Compound 1 spray-dried dispersion comprises hydroxypropyl methylcellulose acetate succinate and about 20 to about 30 wt % Compound 1. In some embodiments, disclosed herein is a pharmaceutical composition comprising about 47.5 to about 52.5 wt % of Compound 1 spray-dried dispersion, about 42.5 to about 47.5 wt % of microcrystalline cellulose, about 2.5 to about 3.5 wt % of croscarmellose sodium, about 0.75 to about 1.25 wt % of magnesium stearate, and about 2.75 to about 3.25 wt % of Opadry blue. In some embodiments, disclosed herein is a pharmaceutical composition comprising about 47.5 to about

52.5 wt % of Compound 1 spray-dried dispersion, about 42.5 to about 47.5 wt % of microcrystalline cellulose, about 2.5 to about 3.5 wt % of croscarmellose sodium, about 0.75 to about 1.25 wt % of magnesium stearate, and about 2.75 to about 3.25 wt % of Opadry blue, wherein the Compound 1 spray-dried dispersion comprises hydroxypropyl methylcellulose acetate succinate and about 20 to about 30 wt % Compound 1.

In some embodiments, disclosed herein is a pharmaceutical composition comprising 47.5 to 52.5 wt % of Compound 1 spray-dried dispersion, 42.5 to 47.5 wt % of microcrystalline cellulose, 2.5 to 3.5 wt % of croscarmellose sodium, and 0.75 to 1.25 wt % of magnesium stearate. In some embodiments, disclosed herein is a pharmaceutical composition comprising 47.5 to 52.5 wt % of Compound 1 spray-dried dispersion, 42.5 to 47.5 wt % of microcrystalline cellulose, 2.5 to 3.5 wt % of croscarmellose sodium, and 0.75 to 1.25 wt % of magnesium stearate, wherein the Compound 1 spray-dried dispersion comprises hydroxypropyl methylcellulose acetate succinate and about 20 to about 30 wt % Compound 1. In some embodiments, disclosed herein is a pharmaceutical composition comprising 47.5 to 52.5 wt % of Compound 1 spray-dried dispersion, 42.5 to 52.5 wt % of microcrystalline cellulose, 2.5 to 3.5 wt % of croscarmellose sodium, 0.75 to 1.25 wt % of magnesium stearate, and 2.75 to 3.25 wt % of Opadry blue. In some embodiments, disclosed herein is a pharmaceutical composition comprising 47.5 to 52.5 wt % of Compound 1 spray-dried dispersion, 42.5 to 47.5 wt % of microcrystalline cellulose, 2.5 to 3.5 wt % of croscarmellose sodium, 0.75 to 1.25 wt % of magnesium stearate, and 2.75 to 3.25 wt % of Opadry blue, wherein the Compound 1 spray-dried dispersion comprises hydroxypropyl methylcellulose acetate succinate and about 20 to about 30 wt % Compound 1.

In some embodiments, disclosed herein is a pharmaceutical composition comprising about 48.5 wt % of Compound 1 spray-dried dispersion, about 44.7 wt % of at least one filler, about 2.9 wt % of at least one disintegrant, and about 1 wt % of at least one lubricant. In some embodiments, disclosed herein is a pharmaceutical composition comprising about 48.5 wt % of Compound 1 spray-dried dispersion, about 44.7 wt % of at least one filler, about 2.9 wt % of at least one disintegrant, and about 1 wt % of at least one lubricant, wherein the Compound 1 spray-dried dispersion comprises at least one polymer and about 25 wt % Compound 1. In some embodiments, disclosed herein is a pharmaceutical composition comprising about 48.5 wt % of Compound 1 spray-dried dispersion, about 44.7 wt % of at least one filler, about 2.9 wt % of at least one disintegrant, about 1 wt % of at least one lubricant, and about 2.9 wt % of at least one coating, wherein the Compound 1 spray-dried dispersion comprises at least one polymer and about 25 wt % Compound 1.

In some embodiments, disclosed herein is a pharmaceutical composition comprising 48.5 wt % of Compound 1 spray-dried dispersion, 44.7 wt % of at least one filler, 2.9 wt % of at least one disintegrant, and 1 wt % of at least one lubricant. In some embodiments, disclosed herein is a pharmaceutical composition comprising 48.5 wt % of Compound 1 spray-dried dispersion, 44.7 wt % of at least one filler, 2.9 wt % of at least one disintegrant, and 1 wt % of at least one lubricant, wherein the Compound 1 spray-dried dispersion comprises at least one polymer and 25 wt % Compound 1. In some embodiments, disclosed herein is a pharmaceutical composition comprising 48.5 wt % of Compound 1 spray-dried dispersion, 44.7 wt % of at least one filler, 2.9 wt % of at least one disintegrant, 1 wt % of at least one lubricant, and 2.9 wt % of at least one coating, wherein the Compound 1 spray-dried dispersion comprises at least one polymer and 25 wt % Compound 1.

**In** some embodiments, disclosed herein is a pharmaceutical composition comprising about 48.5 wt % of Compound 1 spray-dried dispersion, about 44.7 wt % of microcrystalline cellulose, about 2.9 wt % of croscarmellose sodium, and about 1 wt % of magnesium stearate. In some embodiments, disclosed herein is a pharmaceutical composition comprising about 48.5 wt % of Compound 1 spray-dried dispersion, about 44.7 wt % of microcrystalline cellulose, about 2.9 wt % of croscarmellose sodium, and about 1 wt % of magnesium stearate, wherein the Compound 1 spray-dried dispersion comprises hydroxypropyl methylcellulose acetate succinate and about 25 wt % Compound 1. In some embodiments, disclosed herein is a pharmaceutical composition comprising about 48.5 wt % of Compound 1 spray-dried dispersion, about 44.7 wt % of microcrystalline cellulose, about 2.9 wt % of croscarmellose sodium, about 1 wt % of magnesium stearate, and about 2.9 wt % of Opadry blue, wherein the Compound 1 spray-dried dispersion comprises hydroxypropyl methylcellulose acetate succinate and about 25 wt % Compound 1.

**In** some embodiments, disclosed herein is a pharmaceutical composition comprising 48.5 wt % of Compound 1 spray-dried dispersion, 44.7 wt % of microcrystalline cellulose, 2.9 wt % of croscarmellose sodium, and 1 wt % of magnesium stearate. In some embodiments, disclosed herein is a pharmaceutical composition comprising 48.5 wt % of Compound 1 spray-dried dispersion, 44.7 wt % of microcrystalline cellulose, 2.9 wt % of croscarmellose sodium, and 1 wt % of at least one magnesium stearate, wherein the Compound 1 spray-dried dispersion comprises hydroxypropyl methylcellulose acetate succinate and 25 wt % Compound 1. In some embodiments, disclosed herein is a pharmaceutical composition comprising 48.5 wt % of Compound 1 spray-dried dispersion, 44.7 wt % of microcrystalline cellulose, 2.9 wt % of croscarmellose sodium, 1 wt % of magnesium stearate, and 2.9 wt % of Opadry blue, wherein the Compound 1 spray-dried dispersion comprises hydroxypropyl methylcellulose acetate succinate and 25 wt % Compound 1.

**In** some embodiments, the pharmaceutical composition described herein can be made into tablets. In some embodiments, a tablet comprises Compound 1, or pharmaceutically acceptable salt thereof, a polymer, a filler, a lubricant, and a disintegrant.

**In** some embodiments, the tablet comprises a polymer selected from: hydroxypropyl methylcellulose acetate succinate (HPMCAS), polyvinyl caprolactam-polyvinyl acetate-polyethylene glycol graft co-polymer, and any combination thereof.

**In** some embodiments, the tablet comprises HPMCAS.

**In** some embodiments, the tablet comprises about 72.5 wt % of the at least one filler, about 4.5 wt % of the at least one disintegrant, and about 3 wt % of the at least one lubricant.

In some embodiments, the tablet comprises about 1 to about 50 mg of Compound 1. In some embodiments, the tablet comprises about 1 to about 45 mg, or about 1 to about 40 mg, or about 1 to about 35 mg, or about 5 to about 40 mg, or about 5 to about 35 mg, or about 5 to about 30 mg, or about 5 mg, or about 10 mg, or about 15 mg, or about 20 mg, or about 25 mg, or about 30 mg, or about 40 mg, or about 50 mg of Compound 1. In some embodiments, the tablet comprises about 10 mg of Compound 1.

*Additional Therapeutic Agents*

It will also be appreciated that Compound 1, salts, and pharmaceutically acceptable compositions of Compound 1 can be employed in combination therapies, that is, the compounds, salts, and pharmaceutically acceptable compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated." For example, exemplary additional therapeutic agents include, but are not limited to: non-opioid analgesics (indoles such as Etodolac, Indomethacin, Sulindac, Tolmetin, naphthylalkanones such as Nabumetone, oxicams such as Piroxicam, para-aminophenol derivatives, such as Acetaminophen, propionic acids such as Fenoprofen, Flurbiprofen, Ibuprofen, Ketoprofen, Naproxen, Naproxen sodium, Oxaprozin, salicylates such as Aspirin, Choline magnesium trisalicylate, Diflunisal, fenamates such as meclofenamic acid, Mefenamic acid, and pyrazoles such as Phenylbutazone), or opioid (narcotic) agonists (such as Codeine, Fentanyl, Hydromorphone, Levorphanol, Meperidine, Methadone, Morphine, Oxycodone, Oxymorphone, Propoxyphene, Buprenorphine, Butorphanol, Dezocine, Nalbuphine, and Pentazocine). Additionally, nondrug analgesic approaches may be utilized in conjunction with administration of one or more compounds of the invention. For example, anesthesiologic (intraspinal infusion, neural blockade), neurosurgical (neurolysis of CNS pathways), neurostimulatory (transcutaneous electrical nerve stimulation, dorsal column stimulation), physiatric (physical therapy, orthotic devices, diathermy), or psychologic (cognitive methods-hypnosis, biofeedback, or behavioral methods) approaches may also be utilized. Additional appropriate therapeutic agents or approaches are described generally in The Merck Manual, Nineteenth Edition, Ed. Robert S. Porter and Justin L. Kaplan, Merck Sharp &Dohme Corp., a subsidiary of Merck & Co., Inc., 2011, and the Food and Drug Administration website, www.fda.gov.

In another embodiment, additional appropriate therapeutic agents are selected from the following:

(1) an opioid analgesic, e.g. morphine, heroin, hydromorphone, oxymorphone, levorphanol, levallorphan, methadone, meperidine, fentanyl, cocaine, codeine, dihydrocodeine, oxycodone, hydrocodone, propoxyphene, nalmefene, nalorphine, naloxone, naltrexone, buprenorphine, butorphanol, nalbuphine, pentazocine, or difelikefalin;
(2) a nonsteroidal antiinflammatory drug (NSAID), e.g. aspirin, diclofenac, diflunisal, etodolac, fenbufen, fenoprofen, flufenisal, flurbiprofen, ibuprofen (including without limitation intravenous ibuprofen (e.g., Caldolor®)), indomethacin, ketoprofen, ketorolac (including without limitation ketorolac tromethamine (e.g., Toradol®)), meclofenamic acid, mefenamic acid, meloxicam, IV meloxicam (e.g., Anjeso®), nabumetone, naproxen, nimesulide, nitroflurbiprofen, olsalazine, oxaprozin, phenylbutazone, piroxicam, sulfasalazine, sulindac, tolmetin or zomepirac;
(3) a barbiturate sedative, e.g. amobarbital, aprobarbital, butabarbital, butalbital, mephobarbital, metharbital, methohexital, pentobarbital, phenobarbital, secobarbital, talbutal, thiamylal or thiopental;
(4) a benzodiazepine having a sedative action, e.g. chlordiazepoxide, clorazepate, diazepam, flurazepam, lorazepam, oxazepam, temazepam or triazolam;
(5) a histamine ($H_1$) antagonist having a sedative action, e.g. diphenhydramine, pyrilamine, promethazine, chlorpheniramine or chlorcyclizine;
(6) a sedative such as glutethimide, meprobamate, methaqualone or dichloralphenazone;
(7) a skeletal muscle relaxant, e.g. baclofen, carisoprodol, chlorzoxazone, cyclobenzaprine, methocarbamol or orphenadrine;
(8) an NMDA receptor antagonist, e.g. dextromethorphan ((+)-3-hydroxy-N-methylmorphinan) or its metabolite dextrorphan ((+)-3-hydroxy-N-methylmorphinan), ketamine, memantine, pyrroloquinoline quinine, cis-4-(phosphonomethyl)-2- piperidinecarboxylic acid, budipine, EN-3231 (MorphiDex®), a combination formulation of morphine and dextromethorphan), topiramate, neramexane or perzinfotel including an NR2B antagonist, e.g. ifenprodil, traxoprodil or (-)-(R)-6-{2-[4-(3-fluorophenyl)-4-hydroxy-l- piperidinyl]-l-hydroxyethyl-3,4-dihydro-2(lH)-quinolinone;
(9) an alpha-adrenergic, e.g. doxazosin, tamsulosin, clonidine, guanfacine, dexmedetomidine, modafinil, or 4-amino-6,7-dimethoxy-2-(5-methane-sulfonamido-l, 2,3,4- tetrahydroisoquinolin-2-yl)-5-(2-pyridyl) quinazoline;
(10) a tricyclic antidepressant, e.g. desipramine, imipramine, amitriptyline or nortriptyline;
(11) an anticonvulsant, e.g. carbamazepine (Tegretol®), lamotrigine, topiramate, lacosamide (Vimpat®) or valproate;
(12) a tachykinin (NK) antagonist, particularly an NK-3, NK-2 or NK-1 antagonist, e.g. (alphaR,9R)-7-[3,5-bis(trifluoromethyl)benzyl]-8,9,10,11 -tetrahydro-9-methyl-5-(4- methylphenyl)-7H-[l,4]diazocino[2,l-g][l,7]-naphthyridine-6-13-dione (TAK-637), 5- [[(2R,35)-2-[(lR)-l-[3,5-bis(trifluoromethyl)phenyl]ethoxy-3-(4-fluorophenyl)-4-morpholinyl]-methyl]-l,2-dihydro-3H-l,2,4-triazol-3-one (MK-869), aprepitant, lanepitant, dapitant or 3-[[2-methoxy-5-(trifluoromethoxy)phenyl]-methylamino]-2-phenylpiperidine (2S,3S);
(13) a muscarinic antagonist, e.g oxybutynin, tolterodine, propiverine, tropsium chloride, darifenacin, solifenacin, temiverine and ipratropium;
(14) a COX-2 selective inhibitor, e.g. celecoxib, rofecoxib, parecoxib, valdecoxib, deracoxib, etoricoxib, or lumiracoxib;
(15) a coal-tar analgesic, in particular paracetamol;
(16) a neuroleptic such as droperidol, chlorpromazine, haloperidol, perphenazine, thioridazine, mesoridazine, trifluoperazine, fluphenazine, clozapine, olanzapine, risperidone, ziprasidone, quetiapine, sertindole, aripiprazole, sonepiprazole, blonanserin, iloperidone, perospirone, raclopride, zotepine, bifeprunox, asenapine, lurasidone, amisulpride, balaperidone, palindore, eplivanserin, osanetant, rimonabant, meclinertant, Miraxion® or sarizotan;
(17) a vanilloid receptor agonist (e.g. resinferatoxin or civamide) or antagonist (e.g. capsazepine, GRC-15300);
(18) a beta-adrenergic such as propranolol;
(19) a local anesthetic such as mexiletine;
(20) a corticosteroid such as dexamethasone;
(21) a 5-HT receptor agonist or antagonist, particularly a 5-$HT_{IB/1D}$ agonist such as eletriptan, sumatriptan, naratriptan, zolmitriptan or rizatriptan;
(22) a 5-$HT_{2A}$ receptor antagonist such as R(+)-alpha-(2,3-dimethoxy-phenyl)-l-[2-(4-fluorophenylethyl)]-4-piperidinemethanol (MDL-100907);
(23) a cholinergic (nicotinic) analgesic, such as ispronicline (TC-1734), (E)-N-methyl-4-(3-pyridinyl)-3-buten-l-amine (RJR-2403), (R)-5-(2-azetidinylmethoxy)-2-chloropyridine (ABT-594) or nicotine;
(24) Tramadol®, Tramadol ER (Ultram ER®), IV Tramadol, Tapentadol ER (Nucynta®);
(25) a PDE5 inhibitor, such as 5-[2-ethoxy-5-(4-methyl-l-piperazinyl-sulphonyl)phenyl]-l-methyl-3-n-propyl-l,6-dihydro-7H-pyrazolo[4,3-d]pyrimidin-7-one (sildenafil), (6R,12aR)-2,3,6,7,12,12a-hexahydro-2-methyl-6-(3,4-methylenedioxyphenyl)-pyrazino[2',l':6,l]-pyrido[3,4-b]indole-l,4-dione (IC-351 or tadalafil), 2-[2-ethoxy-5-(4-ethyl-piperazin-l-yl-l-sulphonyl)-phenyl]-5-methyl-7-propyl-3H-imidazo|[5,l-f][l,2,4]triazin-4-one (vardenafil), 5-(5-acetyl-2-butoxy-3-pyridinyl)-3-ethyl-2-(l-ethyl-3-azetidinyl)-2,6-dihydro-7*H*- pyrazolo[4,3-*d*]pyrimidin-7-one, 5-(5-acetyl-2-propoxy-3-pyridinyl)-3-ethyl-2-(l-isopropyl-3-azetidinyl)-2,6-dihydro-7*H*-pyrazolo[4,3-d]pyrimidin-7-one, 5-[2-ethoxy-5-(4-ethylpiperazin-l-ylsulphonyl)pyridin-3-yl]-3-ethyl-2-[2-methoxyethyl]-2,6-dihydro-7H- pyrazolo[4,3-d] pyrimidin-7-one, 4-[(3-chloro-4-methoxybenzyl)amino]-2-[(2S)-2-(hydroxymethyl)pyrrolidin-l-yl]-N-(pyrimidin-2-ylmethyl)pyrimidine-5-carboxamide, 3-(l- methyl-7-oxo-3-propyl-6,7-dihydro-lH-pyrazolo[4,3-d]pyrimidin-5-yl)-N-[2-(l-methylpyrrolidin-2-yl)ethyl]-4-propoxybenzenesulfonamide;
(26) an alpha-2-delta ligand such as gabapentin (Neurontin®), gabapentin GR (Gralise®), gabapentin, enacarbil (Horizant®), pregabalin (Lyrica®), 3-methyl gabapentin, (l[alpha],3[alpha],5[alpha])(3-amino-methyl-bicyclo[3.2.0]hept-3-yl)-acetic acid, (3S,5R)-3-aminomethyl-5-methyl-heptanoic acid, (3S,5R)-3-amino-5-methyl-heptanoic acid, (3S,5R)-3-amino-5-methyl-octanoic acid, (2S,4S)-4-(3-chlorophenoxy)proline, (2S,4S)-4-(3-fluorobenzyl)-proline, [(lR,5R,6S)-6-(aminomethyl)bicyclo[3.2.0]hept-6-yl]acetic acid, 3-(l-aminomethyl-cyclohexylmethyl)-4H-[1,2,4]oxadiazol-5-one, C-[1-(1H-tetrazol-5-ylmethyl)-cycloheptyl]-methylamine, (3S,4S)-(l-aminomethyl-3,4-dimethyl-cyclopentyl)-acetic acid, (3S,5R)-3-aminomethyl-5-methyl-octanoic acid, (3S,5R)-3-amino-5-methyl-nonanoic acid, (3S,5R)-3-amino-5-methyl-octanoic acid, (3R,4R,5R)-3-amino-4,5-dimethyl-heptanoic acid and (3R,4R,5R)-3-amino-4,5-dimethyl-octanoic acid;
(27) a cannabinoid such as KHK-6188;
(28) metabotropic glutamate subtype 1 receptor (mGluRl) antagonist;
(29) a serotonin reuptake inhibitor such as sertraline, sertraline metabolite demethylsertraline, fluoxetine, norfluoxetine (fluoxetine desmethyl metabolite), fluvoxamine, paroxetine, citalopram, citalopram metabolite desmethylcitalopram, escitalopram, d,l-fenfluramine, femoxetine, ifoxetine, cyanodothiepin, litoxetine, dapoxetine, nefazodone, cericlamine and trazodone;
(30) a noradrenaline (norepinephrine) reuptake inhibitor, such as maprotiline, lofepramine, mirtazepine, oxaprotiline,

fezolamine, tomoxetine, mianserin, bupropion, bupropion metabolite hydroxybupropion, nomifensine and viloxazine (Vivalan®), especially a selective noradrenaline reuptake inhibitor such as reboxetine, in particular (S,S)-reboxetine;
(31) a dual serotonin-noradrenaline reuptake inhibitor, such as venlafaxine, venlafaxine metabolite O-desmethylvenlafaxine, clomipramine, clomipramine metabolite desmethylclomipramine, duloxetine (Cymbalta®), milnacipran and imipramine;
(32) an inducible nitric oxide synthase (iNOS) inhibitor such as S-[2-[(l-iminoethyl)amino]ethyl]-L-homocysteine, S-[2-[(l-iminoethyl)-amino]ethyl]-4,4-dioxo-L-cysteine, S-[2-[(l-iminoethyl)amino]ethyl]-2-methyl-L-cysteine, (2S,5Z)-2-amino-2-methyl-7-[(l-iminoethyl)amino]-5-heptenoic acid, 2-[[(lR,35)-3-amino-4-hydroxy-l-(5-thiazolyl)-butyl]thio]-S-chloro-S-pyridinecarbonitrile; 2-[[(lR,3S)-3-amino-4-hydroxy-l-(5- thiazolyl)butyl]thio]-4-chlorobenzonitrile, (2S,4R)-2-amino-4-[[2-chloro-5- (trifluoromethyl)phenyl]thio]-5-thiazolebutanol, 2-[[(lR,3S)-3-amino-4-hydroxy-l-(5-thiazolyl) butyl]thio]-6-(trifluoromethyl)-3-pyridinecarbonitrile, 2-[[(lR,3S)-3-amino-4-hydroxy-1-(5-thiazolyl)butyl]thio]-5-chlorobenzonitrile, N-[4-[2-(3-chlorobenzylamino)ethyl]phenyl]thiophene-2-carboxamidine, NXN-462, or guanidinoethyldisulfide;
(33) an acetylcholinesterase inhibitor such as donepezil;
(34) a prostaglandin E2 subtype 4 (EP4) antagonist such as N-[({2-[4-(2-ethyl-4,6- dimethyl-lH-imidazo[4,5-c]pyridin-l-yl)phenyl]ethyl}amino)-carbonyl]-4- methylbenzenesulfonamide or 4-[(15)-l-({[5-chloro-2-(3-fluorophenoxy)pyridin-3- yl]carbonyl}amino)ethyl]benzoic acid;
(35) a leukotriene B4 antagonist; such as l-(3-biphenyl-4-ylmethyl-4-hydroxy-chroman-7-yl)-cyclopentanecarboxylic acid (CP- 105696), 5-[2-(2-Carboxyethyl)-3-[6-(4-methoxyphenyl)-5E-hexenyl]oxyphenoxy]-valeric acid (ONO-4057) or DPC-11870;
(36) a 5-lipoxygenase inhibitor, such as zileuton, 6-[(3-fluoro-5-[4-methoxy-3,4,5,6-tetrahydro-2H-pyran-4-yl])phenoxy-methyl]-l-methyl-2-quinolone (ZD-2138), or 2,3,5- trimethyl-6-(3-pyridylmethyl)-l,4-benzoquinone (CV-6504);
(37) a sodium channel blocker, such as lidocaine, lidocaine plus tetracaine cream (ZRS-201) or eslicarbazepine acetate;
(38) a $Na_V$1.7 blocker, such as XEN-402, XEN403, TV-45070, PF-05089771, CNV1014802, GDC-0276, RG7893 BIIB-074 (Vixotrigine), BIIB-095, ASP-1807, DSP-3905, OLP-1002, RQ-00432979, FX-301, DWP-1706, DWP-17061, IMB-110, IMB-111, IMB-112 and such as those disclosed in WO2011/140425 (US2011/306607); WO2012/106499 (US2012/196869); WO2012/112743 (US2012245136); WO2012/125613 (US2012/264749), WO2012/116440 (US2014/187533), WO2011/026240 (US2012/220605), US8883840, US8466188, WO2013/109521 (US2015005304), WO2020/092667 (US2020/140411), or CN111217776;
(38a) a $Na_V$1.7 blocker such as (2-benzylspiro[3,4-dihydropyrrolo[1,2-a]pyrazine-1,4'-piperidine]-1'-yl)-(4-isopropoxy-3-methyl-phenyl)methanone, 2,2,2-trifluoro-1-[1'-[3-methoxy-4-[2-(trifluoromethoxy)ethoxy]benzoyl]-2,4-dimethyl-spiro[3,4-dihydropyrrolo[1,2-a]pyrazine-1,4'-piperidine]-6-yl]ethanone, [8-fluoro-2-methyl-6-(trifluoromethyl)spiro[3,4-dihydropyrrolo[1,2-a]pyrazine-1,4'-piperidine]-1'-yl]-(4-isobutoxy-3-methoxy-phenyl)methanone, 1-(4-benzhydrylpiperazin-1-yl)-3-[2-(3,4-dimethylphenoxy)ethoxy]propan-2-ol, (4-butoxy-3-methoxy-phenyl)-[2-methyl-6-(trifluoromethyl)spiro[3,4-dihydropyrrolo[1,2-a]pyrazine-1,4'-piperidine]-1'-yl]methanone, [8-fluoro-2-methyl-6-(trifluoromethyl)spiro[3,4-dihydropyrrolo[1,2-a]pyrazine-1,4'-piperidine]-1'-yl]-(5-isopropoxy-6-methyl-2-pyridyl)methanone, (4-isopropoxy-3-methyl-phenyl)-[2-methyl-6-(1,1,2,2,2-pentafluoroethyl)spiro[3,4-dihydropyrrolo[1,2-a]pyrazine-1,4'-piperidine]-1'-yl]methanone, 5-[2-methyl-4-[2-methyl-6-(2,2,2-trifluoroacetyl)spiro[3,4-dihydropyrrolo[1,2-a]pyrazine-1,4'-piperidine]-1'-carbonyl]phenyl]pyridine-2-carbonitrile, (4-isopropoxy-3-methyl-phenyl)-[6-(trifluoromethyl)spiro[3,4-dihydro-2H-pyrrolo[1,2-a]pyrazine-1,4'-piperidine]-1'-yl]methanone, 2,2,2-trifluoro-1-[1'-[3-methoxy-4-[2-(trifluoromethoxy)ethoxy]benzoy1]-2-methyl-spiro[3,4-dihydropyrrolo[1,2-a]pyrazine-l,4'-piperidine]-6-yl]ethanone, 2,2,2-trifluoro-1-[1'-(5-isopropoxy-6-methyl-pyridine-2-carbonyl)-3,3-dimethyl-spiro[2,4-dihydropyrrolo[1,2-a]pyrazine-1,4'-piperidine]-6-yl]ethanone, 2,2,2-trifluoro-1-[1'-(5-isopentyloxypyridine-2-carbonyl)-2-methyl-spiro[3,4-dihydropyrrolo[1,2-a]pyrazine-1,4'-piperidine]-6-yl]ethanone, (4-isopropoxy-3-methoxy-phenyl)-[2-methyl-6-(trifluoromethyl)spiro[3,4-dihydropyrrolo[1,2-a]pyrazine-1,4'-piperidine]-1'-yl]methanone, 2,2,2-trifluoro-1-[1'-(5-isopentyloxypyridine-2-carbonyl)-2,4-dimethyl-spiro[3,4-dihydropyrrolo[1,2-a]pyrazine-1,4'-piperidine]-6-yl]ethanone, 1-[(3S)-2,3-dimethyl-1'-[4-(3,3,3-trifluoropropoxymethyl)benzoyl]spiro[3,4-dihydropyrrolo[1,2-a]pyrazine-1,4'-piperidine]-6-yl]-2,2,2-trifluoro-ethanone, [8-fluoro-2-methyl-6-(trifluoromethyl)spiro[3,4-dihydropyrrolo[1,2-a]pyrazine-1,4'-piperidine]-1'-yl]-[3-methoxy-4-[(1R)-1-methylpropoxy]phenyl]methanone, 2,2,2-trifluoro-1-[1'-(5-isopropoxy-6-methyl-pyridine-2-carbonyl)-2,4-dimethyl-spiro[3,4-dihydropyrrolo[1,2-a]pyrazine-1,4'-piperidine]-6-yl]ethanone, 1-[1'-[4-methoxy-3-(trifluoromethyl)benzoyl]-2-methyl-spiro[3,4-dihydropyrrolo[1,2-a]pyrazine-1,4'-piperidine]-6-yl]-2,2-dimethyl-propan-1-one, (4-isopropoxy-3-methyl-phenyl)-[2-methyl-6-(trifluoromethyl)spiro[3,4-dihydropyrrolo[1,2-a]pyrazine-1,4'-piperidine]-1'-yl]methanone, [2-methyl-6-(1-methylcyclopropanecarbonyl)spiro[3,4-dihydropyrrolo[1,2-a]pyrazine-1,4'-piperidine]-1'-yl]-[4-(3,3,3-trifluoropropoxymethyl)phenyl]methanone, 4-bromo-N-(4-bromophenyl)-3-[(1-methyl-2-oxo-4-piperidyl)sulfamoyl]benzamide or (3-chloro-4-isopropoxy-phenyl)-[2-methyl-6-(1,1,2,2,2-pentafluoroethyl)spiro[3,4-dihydropyrrolo[1,2-a]pyrazine-1,4'-piperidine]-1'-yl]methanone.

(39) a $Na_V$1.8 blocker, such as PF-04531083, PF-06372865 and such as those disclosed in WO2008/135826 (US2009048306), WO2006/011050 (US2008312235), WO2013/061205 (US2014296313), US20130303535, WO2013131018, US8466188, WO2013114250 (US2013274243), WO2014/120808 (US2014213616), WO2014/120815 (US2014228371) WO2014/120820 (US2014221435), WO2015/010065 (US20160152561), WO2015/089361 (US20150166589), WO2019/014352 (US20190016671), WO2018/213426, WO2020/146682, WO2020/146612, WO2020/014243, WO2020/014246, WO2020/092187, WO2020/092667 (US2020140411), WO2020/140959, WO2020/151728, WO2020/261114, WO2021/032074, CN112390745, CN111808019, CN112225695, CN112457294, CN112300051, CN112300069, CN112441969, or CN112479996;

(39a) a $Na_V$1.8 blocker such as 4,5-dichloro-2-(4-fluoro-2-methoxyphenoxy)-N-(2-oxo-1,2-dihydropyridin-4-yl)benzamide, 2-(4-fluoro-2-methoxyphenoxy)-N-(2-oxo-1,2-dihydropyridin-4-yl)-4-(perfluoroethyl)benzamide, 4,5-dichloro-2-(4-fluorophenoxy)-N-(2-oxo-1,2-dihydropyridin-4-yl)benzamide, 4,5-dichloro-2-(3-fluoro-4-methoxyphenoxy)-N-(2-oxo-1,2-dihydropyridin-4-yl)benzamide, 2-(4-fluoro-2-methoxyphenoxy)-N-(2-oxo-1,2-dihydropyridin-4-yl)-5-(trifluoromethyl)benzamide, N-(2-oxo-1,2-dihydropyridin-4-yl)-2-(4-(trifluoromethoxy)phenoxy)-4-(trifluoromethyl)benzamide, 2-(4-fluorophenoxy)-N-(2-oxo-1,2-dihydropyridin-4-yl)-4-(perfluoroethyl)benzamide, 5-chloro-2-(4-fluoro-2-methoxyphenoxy)-N-(2-oxo-1,2-dihydropyridin-4-yl)benzamide, N-(2-oxo-1,2-dihydropyridin-4-yl)-2-(4-(trifluoromethoxy)phenoxy)-5-(trifluoromethyl)benzamide, 2-(4-fluoro-2-methylphenoxy)-N-(2-oxo-1,2-dihydropyridin-4-yl)-5-(trifluoromethyl)benzamide, 2-(2-chloro-4-fluorophenoxy)-N-(2-oxo-1,2-dihydropyridin-4-yl)-5-(trifluoromethyl)benzamide, 5-chloro-2-(4-fluoro-2-methylphenoxy)-N-(2-oxo-1,2-dihydropyridin-4-yl) benzamide, 4-chloro-2-(4-fluoro-2-methylphenoxy)-N-(2-oxo-1,2-dihydropyridin-4-yl)benzamide, 5-chloro-2-(2-chloro-4-fluorophenoxy)-N-(2-oxo-1,2-dihydropyridin-4-yl)benzamide, 2-((5-fluoro-2-hydroxybenzyl)oxy)-N-(2-oxo-1,2-dihydropyridin-4-yl)-4-(trifluoromethyl)benzamide, N-(2-oxo-1,2-dihydropyridin-4-yl)-2-(o-tolyloxy)-5-(trifluoromethyl)benzamide, 2-(2,4-difluorophenoxy)-N-(2-oxo-1,2-dihydropyridin-4-yl)-4-(trifluoromethyl)benzamide, N-(2-oxo-1,2-dihydropyridin-4-yl)-2-(2-(trifluoromethoxy)phenoxy)-5-(trifluoromethyl)benzamide, 2-(4-fluorophenoxy)-N-(2-oxo-1,2-dihydropyridin-4-yl)-5-(trifluoromethyl)benzamide, 2-(4-fluoro-2-methyl-phenoxy)-N-(2-oxo-1H-pyridin-4-yl)-4-(trifluoromethyl)benzamide, [4-[[2-(4-fluoro-2-methyl-phenoxy)-4-(trifluoromethyl)benzoyl] amino]-2-oxo-1-pyridyl]methyl dihydrogen phosphate, 2-(4-fluoro-2-(methyl-$d_3$)phenoxy)-N-(2-oxo-1,2-dihydropyridin-4-yl)-4-(trifluoromethyl)benzamide, (4-(2-(4-fluoro-2-(methyl-$d_3$)phenoxy)-4-(trifluoromethyl)benzamido)-2-oxopyridin-1(2H)-yl)methyl dihydrogen phosphate, 3-(4-fluoro-2-methoxyphenoxy)-N-(3-(methylsulfonyl)phenyl)quinoxaline-2-carboxamide, 3-(2-chloro-4-fluorophenoxy)-N-(3-sulfamoylphenyl)quinoxaline-2-carboxamide, 3-(2-chloro-4-methoxyphenoxy)-N-(3-sulfamoylphenyl)quinoxaline-2-carboxamide, 3-(4-chloro-2-methoxyphenoxy)-N-(3-sulfamoylphenyl)quinoxaline-2-carboxamide, 4-(3-(4-(trifluoromethoxy)phenoxy)quinoxaline-2-carboxamido)picolinic acid, 2-(2,4-difluorophenoxy)-N-(3-sulfamoylphenyl)quinoline-3-carboxamide, 2-(4-fluoro-2-methoxyphenoxy)-N-(3-sulfamoylphenyl)quinoline-3-carboxamide, 3-(2,4-difluorophenoxy)-N-(3-sulfamoylphenyl)quinoxaline-2-carboxamide, N-(3-sulfamoylphenyl)-2-(4-(trifluoromethoxy)phenoxy)quinoline-3-carboxamide, N-(3-sulfamoylphenyl)-3-(4-(trifluoromethoxy)phenoxy)quinoxaline-2-carboxamide, 3-(4-chloro-2-methylphenoxy)-N-(3-sulfamoylphenyl)quinoxaline-2-carboxamide, 5-(3-(4-(trifluoromethoxy)phenoxy)quinoxaline-2-carboxamido)picolinic acid, 3-(4-fluoro-2-methoxyphenoxy)-N-(2-oxo-2,3-dihydro-1H-benzo[d]imidazol-5-yl)quinoxaline-2-carboxamide, 3-(4-fluoro-2-methoxyphenoxy)-N-(pyridin-4-yl)quinoxaline-2-carboxamide, 3-(4-fluorophenoxy)-N-(3-sulfamoylphenyl)quinoxaline-2-carboxamide, N-(3-cyanophenyl)-3-(4-fluoro-2-methoxyphenoxy)quinoxaline-2-carboxamide, N-(4-carbamoylphenyl)-3-(4-fluoro-2-methoxyphenoxy)quinoxaline-2-carboxamide, 4-(3-(4-(trifluoromethoxy)phenoxy)quinoxaline-2-carboxamido)benzoic acid, N-(4-cyanophenyl)-3-(4-fluoro-2-methoxyphenoxy) quinoxaline-2-carboxamide, 5-(4,5-dichloro-2-(4-fluoro-2-methoxyphenoxy)benzamido)picolinic acid, 5-(2-(2,4-dimethoxyphenoxy)-4,6-bis(trifluoromethyl)benzamido)picolinic acid, 4-(4,5-dichloro-2-(4-fluoro-2-methoxyphenoxy) benzamido)benzoic acid, 5-(2-(4-fluoro-2-methoxyphenoxy)-4,6-bis(trifluoromethyl)benzamido)picolinic acid, 4-(2-(4-fluoro-2-methoxyphenoxy)-4-(perfluoroethyl)benzamido)benzoic acid, 5-(2-(4-fluoro-2-methoxyphenoxy)-4-(perfluoroethyl)benzamido)picolinic acid, 4-(2-(4-fluoro-2-methylphenoxy)-4-(trifluoromethyl)benzamido)benzoic acid, 5-(4,5-dichloro-2-(4-fluoro-2-methoxyphenoxy)benzamido)picolinic acid, 4-(2-(2-chloro-4-fluorophenoxy)-4-(perfluoroethyl)benzamido)benzoic acid, 4-(2-(4-fluoro-2-methylphenoxy)-4-(perfluoroethyl)benzamido)benzoic acid, 4-(4,5-dichloro-2-(4-(trifluoromethoxy)phenoxy)benzamido)benzoic acid, 4-(4,5-dichloro-2-(4-chloro-2-methylphenoxy)benzamido)benzoic acid, 5-(4-(tert-butyl)-2-(4-fluoro-2-methoxyphenoxy)benzamido)picolinic acid, 5-(4,5-dichloro-2-(4-(trifluoromethoxy)phenoxy)benzamido)picolinic acid, 4-(4,5-dichloro-2-(4-fluoro-2-methylphenoxy)benzamido)benzoic acid, 5-(4,5-dichloro-2-(2,4-dimethoxyphenoxy)benzamido)picolinic acid, 5-(4,5-dichloro-2-(2-chloro-4-fluorophenoxy)benzamido)picolinic acid, 5-(4,5-dichloro-2-(4-fluoro-2-methylphenoxy)benzamido)picolinic acid, 4-(4,5-dichloro-2-(4-chloro-2-methoxyphenoxy)benzamido)benzoic acid, 5-(4,5-dichloro-2-(2,4-difluorophenoxy)benzamido)picolinic acid, 2-(4-fluorophenoxy)-N-(3-sulfamoylphenyl)-5-(trifluoromethyl)benzamide, 2-(4-fluorophenoxy)-N-(3-sulfamoylphenyl)-4-(trifluoromethyl)benzamide, 2-(2-chloro-4-fluorophenoxy)-N-(3-sulfamoylphenyl)-5-(trifluoromethyl)benzamide, 2-(4-fluorophenoxy)-N-(3-sulfamoylphenyl)-4-(trifluoromethyl)benzamide, 2-(2-chloro-4-fluorophenoxy)-N-(3-sulfamoylphenyl)-6-(trifluoromethyl)benzamide, 2-(2-chloro-4-fluorophenoxy)-5-(difluoromethyl)-N-(3-sulfamoylphenyl)benzamide, 2-(4-fluorophenoxy)-4-(perfluoroethyl)-N-(3-sulfamoylphenyl)benzamide, 2-(4-chloro-2-methoxyphenoxy)-4-(perfluoroethyl)-N-(3-sulfamoylphenyl)benzamide, 2-(4-fluoro-2-methoxyphenoxy)-N-(3-sulfamoylphenyl)-5-(trifluoromethyl)benzamide, 5-chloro-2-(4-fluoro-2-methylphenoxy)-N-(3-sulfamoylphenyl)benzamide, 4,5-dichloro-2-(4-fluoro-2-methoxyphenoxy)-N-(3-sulfamoylphenyl)benzamide, 2,4-dichloro-6-(4-chloro-2-methoxyphenoxy)-N-(3-sulfamoylphenyl)benzamide, 2,4-dichloro-6-(4-fluoro-2-methylphenoxy)-N-(3-sulfamoylphenyl)benzamide, 2-(4-fluoro-2-methoxyphenoxy)-N-(3-sulfamoylphenyl)-4,6-bis(trifluoromethyl)benzamide, 2-(4-fluoro-2-methylphenoxy)-N-(3-sulfamoylphenyl)-4,6-bis(trifluoromethyl)benzamide, 5-chloro-2-(2-chloro-4-fluorophenoxy)-N-(3-sulfamoylphenyl)benzamide, 2-(4-fluoro-2-methoxyphenoxy)-N-(3-sulfamoylphenyl)-4-(trifluoromethoxy)benzamide, 2-(4-fluoro-2-methoxyphenoxy)-N-(3-sulfamoylphenyl)-4-(trifluoromethyl)benzamide, 4,5-dichloro-2-(4-fluorophenoxy)-N-(3-sulfamoylphenyl)benzamide, 2-(4-fluoro-2-methoxyphenoxy)-4-(perfluoroethyl)-N-(3-sulfamoylphenyl)benzamide, 5-fluoro-2-(4-fluoro-2-methylphenoxy)-N-(3-sulfamoylphenyl)benzamide, 2-(2-chloro-4-fluorophenoxy)-4-cyano-N-(3-sulfamoylphenyl)benzamide, N-(3-sulfamoylphenyl)-2-(4-(trifluoromethoxy)phenoxy)-4-(trifluoromethyl)benzamide, N-(3-carbamoyl-4-fluoro-phenyl)-2-fluoro-6-[2-(trideuteriomethoxy)-4-(trifluoromethoxy)phenoxy]-3-(trifluoromethyl)benzamide, N-(3-carbamoyl-4-fluoro-phenyl)-2-fluoro-6-[2-methoxy-4-(trifluoromethoxy)phenoxy]-3-(trifluoromethyl)benzamide, N-(3-carbamoyl-4-fluorophenyl)-2-fluoro-6-[2-(trideuteriomethoxy)-4-(trifluoromethoxy)phenoxy]-3-(trifluoromethoxy)benzamide, 4-[[2-fluoro-6-[2-methoxy-4-(trifluoromethoxy)phenoxy]-3-(trifluoromethyl)benzoyl]amino]pyridine-2-carboxamide, 4-[[3-chloro-2-fluoro-6-[2-methoxy-4-(trifluoromethoxy)phenoxy]benzoyl]amino]pyridine-2-carboxamide, 4-[[2-fluoro-6-[2-(trideuteriomethoxy)-4-(trifluoromethoxy)phenoxy]-3-(trifluoromethyl)benzoyl]amino]pyridine-2-carboxamide, N-(3-carbamoyl-4-fluoro-phenyl)-3-(difluoromethyl)-2-fluoro-6-[2-methoxy-4-(trifluoromethoxy)phenoxy]benzamide, 4-[[2-fluoro-6-[2-(trideuteriomethoxy)-4-(trifluoromethoxy)phenoxy]-3-(trifluoromethoxy)benzoyl]amino]pyridine-2-carboxamide, N-(3-carbamoyl-4-fluoro-phenyl)-6-[2-chloro-4-(trifluoromethoxy)phenoxy]-2-fluoro-3-(trifluoromethyl)benzamide, N-(3-carbamoyl-4-fluoro-phenyl)-2-fluoro-6-[2-methyl-4-(trifluoromethoxy)phenoxy]-3-(trifluoromethyl)benzamide, N-(3-carbamoyl-4-fluoro-phenyl)-2,3,4-trifluoro-6-[2-methoxy-4-(trifluoromethoxy)phenoxy]benzamide, N-(2-carbamoyl-4-pyridyl)-3-fluoro-5-[2-methoxy-4-(trifluoromethoxy)phenoxy]-2-(trifluoromethyl)pyridine-4-carboxamide, 4-[[6-[2-(difluoromethoxy)-4-(trifluoromethoxy)phenoxy]-2-fluoro-3-(trifluoromethyl)benzoyl]amino]pyridine-2-carboxamide, N-(3-carbamoyl-4-fluoro-phenyl)-6-[3-chloro-4-(trifluoromethoxy)phenoxy]-2-fluoro-3-(trifluoromethyl)benzamide, N-(3-carbamoyl-4-fluoro-phenyl)-2-fluoro-6-[4-(trifluoromethoxy)phenoxy]-3-(trifluoromethyl)benzamide, N-(4-carbamoyl-3-fluoro-phenyl)-2-fluoro-6-[2-methoxy-4-(trifluoromethoxy)phenoxy]-3-(trifluoromethyl)benzamide, 4-[[2-fluoro-6-[2-(trideuteriomethoxy)-4-(trifluoromethoxy)phenoxy]-4-(trifluoromethyl)benzoyl]amino]pyridine-2-carboxamide, N-(3-carbamoyl-4-fluoro-phenyl)-2-fluoro-6-[3-fluoro-4-(trifluoromethoxy)phenoxy]-3-(trifluoromethyl)benzamide, N-(3-carbamoyl-4-fluoro-phenyl)-2-[2-methoxy-4-(trifluoromethoxy)phenoxy]-5-(1,1,2,2,2-pentafluoroethyl)benzamide, 4-[[4-(difluoromethoxy)-2-fluoro-6-[2-methoxy-4-(trifluoromethoxy)phenoxy]benzoyl]amino]pyridine-2-carboxamide, N-(3-carbamoyl-4-fluoro-phenyl)-2-fluoro-6-[2-fluoro-4-(trifluoromethoxy)phenoxy]-3-(trifluoromethyl)benzamide, 4-[[4-cyclopropyl-2-fluoro-6-[2-methoxy-4-(trifluoromethoxy)phenoxy]benzoyl]amino]pyridine-2-carboxamide, N-(3-carbamoyl-4-fluoro-phenyl)-5-fluoro-2-[2-methoxy-4-(trifluoromethoxy)phenoxy]-4-(trifluoromethyl)benzamide, 5-[[2-fluoro-6-[2-(trideuteriomethoxy)-4-(trifluoromethoxy)phenoxy]-3-(trifluoromethyl)benzoyl]amino]pyridine-2-carboxamide, N-(3-carbamoyl-4-fluoro-phenyl)-2-fluoro-6-(4-fluorophenoxy)-3-(trifluoromethyl)benzamide, 4-(2-fluoro-6-(2-methoxy-4-(trifluoromethoxy)phenoxy)-3-(trifluoromethyl)benzamido)picolinamide, or 4-[[2-fluoro-6-[3-fluoro-2-methoxy-4-(trifluoromethoxy)phenoxy]-3-(trifluoromethyl)benzoyl]amino]pyridine-2-carboxamide;

(40) a combined $Na_V1.7$ and $Na_V1.8$ blocker, such as DSP-2230, Lohocla201 or BL-1021;

(41) a 5-HT3 antagonist, such as ondansetron;

(42) a TPRV 1 receptor agonist, such as capsaicin (NeurogesX®, Qutenza®); and the pharmaceutically acceptable salts and solvates thereof;

(43) a nicotinic receptor antagonist, such as varenicline;

(44) an N-type calcium channel antagonist, such as Z-160;

(45) a nerve growth factor antagonist, such as tanezumab;

(46) an endopeptidase stimulant, such as senrebotase;

(47) an angiotensin II antagonist, such as EMA-401;

(48) acetaminophen (including without limitation intravenous acetaminophen (e.g., Ofirmev®));

(49) bupivacaine (including without limitation bupivacaine liposome injectable suspension (e.g., Exparel®) bupivacaine ER (Posimir), bupivacaine collagen (Xaracoll) and transdermal bupivacaine (Eladur®)); and

(50) bupivacaine and meloxicam combination (e.g., HTX-011/Zynrelef™).

In one embodiment, the additional appropriate therapeutic agents are selected from V-116517, Pregabalin, controlled release Pregabalin, Ezogabine (Potiga®). Ketamine/amitripty line topical cream (Amiket®), AVP-923, Perampanel (E-2007), Ralfinamide, transdermal bupivacaine (Eladur®), CNV1014802, JNJ-10234094 (Carisbamate), BMS-954561 or ARC-4558.

In another embodiment, the additional appropriate therapeutic agents are selected from N-(6-amino-5-(2,3,5-trichlorophenyl)pyridin-2-yl)acetamide; N-(6-amino-5-(2-chloro-5-methoxyphenyl)pyridin-2-yl)-1-methyl-1H-pyrazole-5-carboxamide; or 3-((4-(4-(trifluoromethoxy)phenyl)-1H-imidazol-2-yl)methyl)oxetan-3-amine.

In another embodiment, the additional therapeutic agent is selected from a GlyT2/5HT2 inhibitor, such as Operanserin (VVZ149), a TRPV modulator such as CA008, CMX-020, NEO6860, FTABS, CNTX4975, MCP101, MDR16523, or MDR652, a EGR1 inhibitor such as Brivoglide (AYX1), an NGF inhibitor such as Tanezumab, Fasinumab, ASP6294, MEDI7352, a Mu opioid agonist such as Cebranopadol, NKTR181 (oxycodegol), a CB-1 agonist such as NEO1940 (AZN1940), an imidazoline 12 agonist such as CR4056 or a p75NTR-Fc modulator such as LEVI-04.

In another embodiment, the additional therapeutic agent is oliceridine or ropivacaine (TLC590).

In another embodiment, the additional therapeutic agent is a $Na_V1.7$ blocker such as ST-2427 or ST-2578 and those disclosed in WO2010129864, WO2015157559, WO2017059385, WO2018183781, WO2018183782, WO2020072835, and WO2002036297. In some embodiments, the additional therapeutic agent is a $Na_V1.7$ blocker disclosed in WO2020072835. In some embodiments, the additional therapeutic agent is a $Na_V1.7$ blocker disclosed in WO2022036297.

In another embodiment, the additional therapeutic agent is ASP18071, CC-8464, ANP-230, ANP-231, NOC-100, NTX-1175, ASN008, NW3509, AM-6120, AM-8145, AM-0422, BL-017881, NTM-006, Opiranserin (Unafra™), brivoligide, SR419, NRD.E1, LX9211, LY3016859, ISC-17536, NFX-88, LAT-8881, AP-235, NYX 2925, CNTX-6016, S-600918, S-637880, RQ-00434739, KLS-2031, MEDI 7352, or XT-150.

In another embodiment, the additional therapeutic agent is Olinvyk, Zynrelef, Seglentis, Neumentum, Nevakar, HTX-034, CPL-01, ACP-044, HRS-4800, Tarlige, BAY2395840, LY3526318, Eliapixant, TRV045, RTA901, NRD1355-E1, MT-8554, LY3556050, AP-325, tetrodotoxin, Otenaproxesul, CFTX-1554, Funapide, iN1011-N17, JMKX000623, ETX-801, or ACD440.

In another embodiment, the additional therapeutic agent is a compound disclosed in WO2021257490, WO2021257420, WO2021257418, WO2020014246, WO2020092187, WO2020092667, WO2020261114, CN112457294, CN112225695, CN111808019, WO2021032074, WO2020151728, WO2020140959, WO2022037641, WO2022037647, CN112300051, CN112300069, WO2014120808, WO2015089361, WO2019014352, WO2021113627, WO2013086229, WO2013134518, WO2014211173, WO2014201206, WO2016141035, WO2021252818, WO2021252822, and WO2021252820.

In some embodiments, the additional therapeutic agent is a compound disclosed in WO2013086229. In some embodiments, the additional therapeutic agent is a compound disclosed in WO2013134518. In some embodiments, the additional therapeutic agent is a compound disclosed in WO2014211173. In some embodiments, the additional therapeutic agent is a compound disclosed in WO2014201206. In some embodiments, the additional therapeutic agent is a compound disclosed in WO2016141035. In some embodiments, the additional therapeutic agent is a compound disclosed in WO2021252818. In some embodiments, the additional therapeutic agent is a compound disclosed in WO2021252822. In some embodiments, the additional therapeutic agent is a compound disclosed in WO2021252820. In some embodiments, the additional therapeutic agent is a compound disclosed in WO2020072835. In some embodiments, the additional therapeutic agent is a compound disclosed in WO2022036297.

In another embodiment, the additional therapeutic agent is a sodium channel inhibitor (also known as a sodium channel blocker), such as the $Na_V1.7$ and $Na_V1.8$ blockers identified above.

The amount of additional therapeutic agent present in the compositions of this invention may be no more than the amount that would normally be administered in a composition comprising that therapeutic agent as the only active agent. The amount of additional therapeutic agent in the presently disclosed compositions may range from about 10% to 100% of the amount normally present in a composition comprising that agent as the only therapeutically active agent.

Compound 1 or pharmaceutically acceptable compositions thereof may also be incorporated into compositions for coating an implantable medical device, such as prostheses, artificial valves, vascular grafts, stents and catheters. Accordingly, another aspect includes a composition for coating an implantable device comprising Compound 1 or salt thereof, and in classes and subclasses herein, and a carrier suitable for coating said implantable device. In still another aspect, an implantable device may be coated with a composition comprising Compound 1 or salt thereof, and a carrier suitable for coating said implantable device. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition.

Synthesis of the Compounds of the Invention (The synthesis is not part of the claimed invention)

Compound **1** can be prepared from known materials by the methods described in the Examples, other similar methods, and other methods known to one skilled in the art. As one skilled in the art would appreciate, the functional groups of the intermediate compounds in the methods described below may need to be protected by suitable protecting groups. Protecting groups may be added or removed in accordance with standard techniques, which are well-known to those skilled in the art. The use of protecting groups is described in detail in T.G.M. Wuts et al., Greene's Protective Groups in Organic Synthesis (4th ed. 2006).

EXAMPLES

Abbreviations

Unless otherwise noted, or where the context dictates otherwise, the following abbreviations shall be understood to have the following meanings:

| **Abbreviation** | **Definition** |
|---|---|
| TPGS | Tocopherol polyethylene glycol succinate |
| HB/APAP | Hydrocodone bitartrate/acetaminophen |
| NPRS | Numeric Pain Rating Scale |
| VRS | Verbal Categorical Rating Scale |
| qd | Once per day |
| tid | Three times per day |
| q24h | Every 24 hours |
| q12h | Every 12 hours |
| q6h | Every 6 hours |
| n | Number of subjects meeting a specified criterion |
| n (%) | Number of subjects meeting a specified criterion (percentage of the total number of subjects in study arm meeting that criterion) |
| SPID24 | NPRS pain intensity difference, relative to baseline, from 0 to 24 hours after the first dose for a given study arm |
| SD | Standard deviation |
| SE | Standard error |
| CI | Confidence interval |
| SPID22 | NPRS pain intensity difference, relative to baseline, from 2 to 24 hours after the first dose for a given study arm |
| SPID48 | NPRS pain intensity difference, relative to baseline, from 0 to 48 hours after the first dose for a given study arm |
| $C_{max}$ | Maximum observed concentration |
| $AUC_{\tau}$ | Area under the curve over a dosing interval ($\tau$) |
| TEAE | Treatment-emergent adverse event |

Example 1

Preparation of (2R,3S,4S,5R)-4-[[3-(3,4-difluoro-2-methoxy-phenyl)-4,5-dimethyl-5-(trifluoromethyl)tetrahydrofuran-2-carbonyl]amino]pyridine-2-carboxamide (Compound 1)

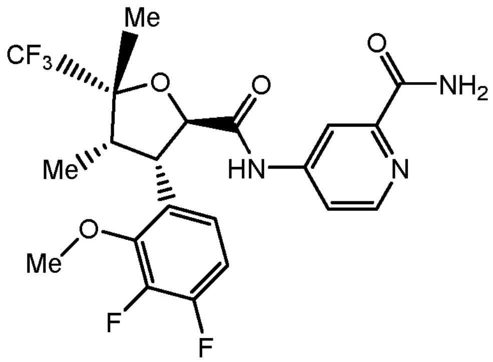

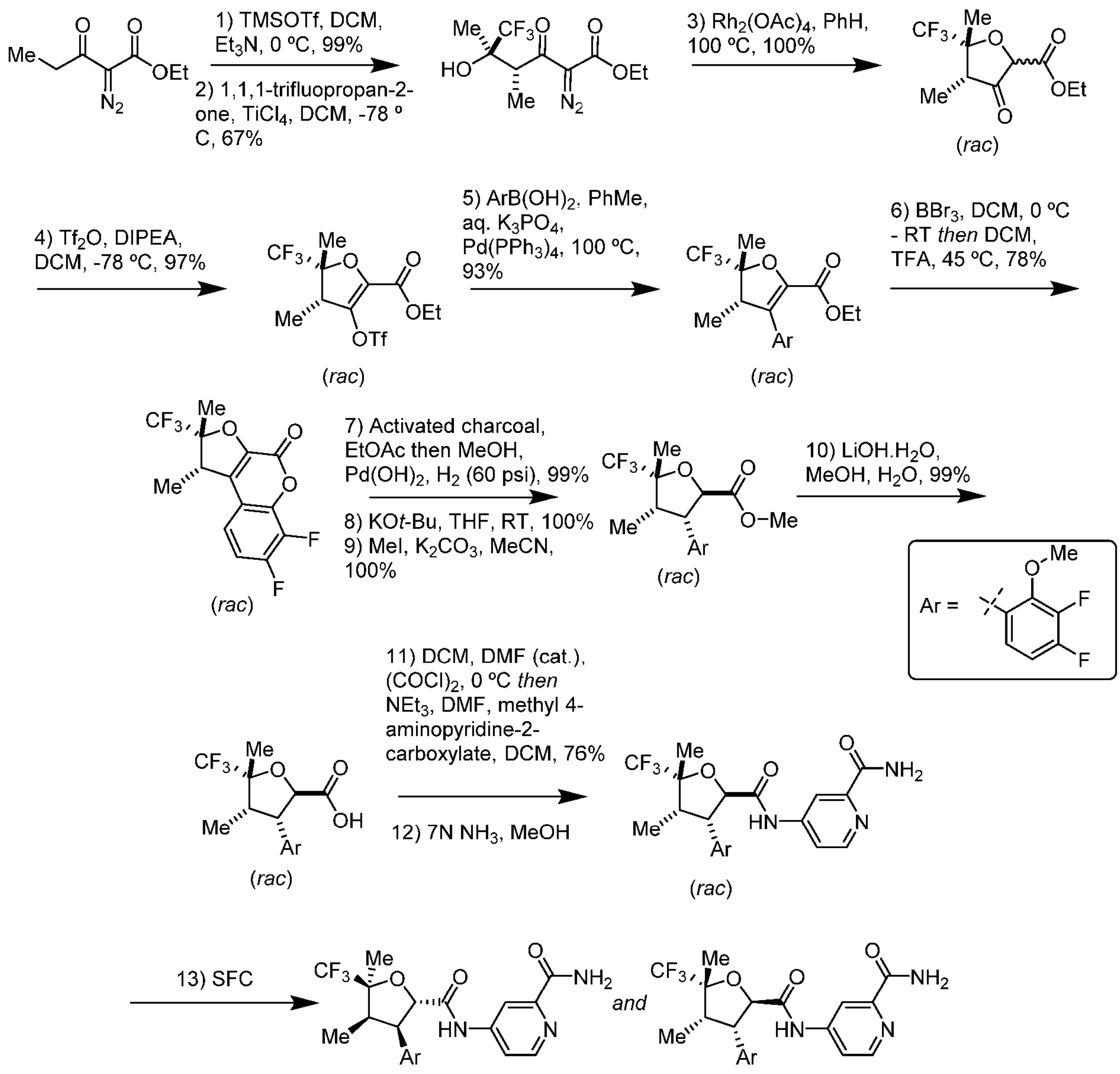


**first eluting isomer** **second eluting isomer (Compound 1)**

**Step 1:**

$NEt_3$ (7.7 mL, 55.2 mmol) was added to a solution of ethyl 2-diazo-3-oxo-pentanoate (6.69 g, 39.3 mmol) in DCM (80 mL) with stirring at 0 °C under nitrogen. Trimethylsilyl trifluoromethanesulfonate (8.5 mL, 47.0 mmol) was added dropwise over 5 mins and the mixture was stirred for a further 30 mins at 0 °C. The reaction mixture was diluted with pentane (100 mL), the layers separated and the organic phase washed with dilute aqueous sodium bicarbonate (100 mL)

and brine (100 mL). The organic layer was dried ($MgSO_4$), and concentrated *in vacuo* to give ethyl (*Z*)-2-diazo-3-trimethylsilyloxy-pent-3-enoate (9.4 g, 99%) as a red oil. $^1H$ NMR (500 MHz, Chloroform-d) δ 5.33 (q, J = 7.0 Hz, 1H), 4.25 (q, J = 7.1 Hz, 2H), 1.67 (d, J = 7.0 Hz, 3H), 1.29 (t, J = 7.1 Hz, 3H), 0.22 (s, 9H) ppm.

**Step 2:**

To a solution of 1,1,1-trifluoropropan-2-one (8 mL, 89.4 mmol) in DCM (80 mL) stirring at -78 °C was added $TiCl_4$ (70 mL of 1 M in DCM, 70.00 mmol) *via* cannula. To the resulting solution, a solution of ethyl (Z)-2-diazo-3-trimethylsilyloxy-pent-3-enoate (36.1 g of 31.3 %w/w, 46.6 mmol) in 40 mL of DCM was added dropwise over 15 mins. After 100 mins the reaction was carefully quenched with water, allowing the temperature to rise slowly, and then extracted with DCM. The combined organic layers were dried ($MgSO_4$), filtered, and concentrated *in vacuo.* Purification by flash chromatography (330 g $SiO_2$, 0 to 20% EtOAc in heptane) gave ethyl 2-diazo-6,6,6-trifluoro-5-hydroxy-4,5-dimethyl-3-oxo-hexanoate (8.82 g, 67%), which was stored as a solution in toluene. $^1H$ NMR (500 MHz, Chloroform-d) δ 4.33 (q, J = 7.1 Hz, 2H), 4.14 (q, J = 7.0 Hz, 1H), 3.98 (s, 1H), 1.43 (q, J = 1.2 Hz, 3H), 1.35 (t, J = 7.1 Hz, 3H), 1.31 (dq, J = 7.0, 1.4 Hz, 3H) ppm. ESI-MS *m*/*z* calc. 282.08273, found 283.1 $(M+1)^+$; 281.0 $(M-1)^-$.

**Step 3:**

A solution of dirhodium tetraacetate (245 mg, 0.55 mmol) in benzene (32 mL) was heated at reflux for 10 min before a solution of ethyl 2-diazo-6,6,6-trifluoro-5-hydroxy-4,5-dimethyl-3-oxo-hexanoate (10 g, 35.4 mmol) in benzene (13 mL) was added slowly *via* addition funnel while refluxing for 60 mins. The mixture was then concentrated *in vacuo* to give ethyl *rac*-(4R,5R)-4,5-dimethyl-3-oxo-5-(trifluoromethyl)tetrahydrofuran-2-carboxylate (9.0 g, 100%) as a green coloured residue containing residual catalyst, and as a mixture of epimers at the position next to the ester. This material was used without further purification. $^1H$ NMR (500 MHz, Chloroform-d) δ 4.83 - 4.57 (m, 1H), 4.38 - 4.16 (m, 2H), 2.60 (dddd, J = 9.3, 8.2, 5.6, 1.4 Hz, 1H), 1.73 - 1.63 (m, 3H), 1.30 (t, J = 7.1 Hz, 3H), 1.24 (ddq, J = 6.4, 4.1, 1.9 Hz, 3H) ppm.

**Step 4:**

To a stirred solution of ethyl *rac*-(4*R*,5*R*)-4,5-dimethyl-3-oxo-5-(trifluoromethyl)tetrahydrofuran-2-carboxylate (48 g, 188.83 mmol) in DCM (400 mL) stirring at -78 °C was added DIPEA (29.680 g, 40 mL, 229.64 mmol). A solution of trifluoromethylsulfonyl trifluoromethanesulfonate (53.440 g, 32 mL, 189.41 mmol) in DCM (200 mL) was added to the reaction mixture at the same temperature over 1h. The reaction mixture was stirred for 30 mins at 0 °C before being quenched with 100 mL saturated aqueous $NaHCO_3$ solution. The organic layer was separated and aqueous layer extracted with DCM (160 mL). The combined organic layers were dried ($MgSO_4$) and concentrated *in vacuo* to give ethyl *rac*-(4*R*,5*R*)-2,3-dimethyl-2-(trifluoromethyl)-4-(trifluoromethylsulfonyloxy)-3H-furan-5-carboxylate (71 g, 97%). $^1H$ NMR (400 MHz, Chloroform-d) δ 4.38-4.32 (m, 2H), 3.29-3.23 (m, 1H), 1.64 (s, 3H), 1.37-1.33 (m, 6H) ppm.

**Step 5:**

To stirred a solution of ethyl *rac*-(4*R*,5*R*)-2,3-dimethyl-2-(trifluoromethyl)-4-(trifluoromethylsulfonyloxy)-3H-furan-5-carboxylate (26 g, 67.311 mmol) in toluene (130.00 mL) was added (3,4-difluoro-2-methoxy-phenyl)boronic acid (14 g, 74.5 mmol) followed by $K_3PO_4$ (100 mL of 2 M, 200.00 mmol) under an argon atmosphere. The reaction was degassed before tetrakis(triphenylphosphine)palladium(0) (4 g, 3.46 mmol) was added. After further degassing, the reaction was heated at 100 °C for 2 hours. The reaction was diluted in water and the aqueous layer extracted with EtOAc (2 x100 mL). The combined organic layers were concentrated *in vacuo.* Purification by flash chromatography ($SiO_2$, 0 to 10% EtOAc in heptane) gave ethyl 4-(3,4-difluoro-2-methoxy-phenyl)-2,3-dimethyl-2-(trifluoromethyl)-3H-furan-5-carboxylate (24.4 g, 93%) as a 6:1 diastereomeric mixture, with the major isomer believed to be ethyl *rac*-(4*R,*5*R*)-4-(3,4-difluoro-2-methoxy-phenyl)-2,3-dimethyl-2-(trifluoromethyl)-3H-furan-5-carboxylate. Major isomer: $^1H$ NMR (400 MHz, Chloroform-d) δ 6.88 - 6.79 (m, 2H), 4.17 - 4.09 (m, 2H), 3.90 (s, 3H), 3.46 (q, J = 7.4 Hz, 1H), 1.67 (s, 3H), 1.12 (t, J = 7.4 Hz, 3H), 1.06 (dd, J = 5.4, 2.7 Hz, 3H) ppm. Minor isomer $^1H$ NMR (400 MHz, Chloroform-d) δ 6.88 - 6.79 (m, 2H), 4.17-4.09 (m, 2H), 3.88(s, 3H), 3.76-3.71(m, 1H), 1.51 (s, 3H), 1.12 (t, J = 7.4 Hz, 3H), 0.99 (dd, J = 5.4, 2.7 Hz, 3H) ppm. ESI-MS m/z calc. 380.1047, found 381.02 $(M+1)^+$.

**Step 6:**

To an ice-cooled solution of ethyl 4-(3,4-difluoro-2-methoxy-phenyl)-2,3-dimethyl-2-(trifluoromethyl)-3H-furan-5-carboxylate (110 g, 243.0 mmol) in DCM (360 mL) was added $BBr_3$ (370 mL of 1 M, 370.0 mmol) dropwise. Upon completion the mixture was quenched by addition of water and aqueous sodium bicarbonate solution, the aqueous layer

extracted with DCM and the combined organic layers dried ($MgSO_4$) and concentrated *in vacuo.* The residue was dissolved in DCM (430 mL) at ambient temperature and TFA (40 mL, 519.2 mmol) was added, then the reaction was heated to 45 °C. Upon completion, the mixture was quenched by addition of aqueous sodium bicarbonate solution and the aqueous layer extracted with DCM, dried ($MgSO_4$) and concentrated *in vacuo* to give the desired product in a 5:1 mixture of diastereomers. Recrystallization was carried out by solubilizing the crude in the smallest possible amount of DCM and adding a layer of heptane on top of this solution (liquid-liquid diffusion). After approx. 1 hour, 56.5 g (d.r. 97:3 syn:anti) from the first and second crystallization was obtained, and a further 4.6 g (d.r. 96:4 syn:anti) from the third crystallization was obtained. The first to third batches were combined to give 6,7-difluoro-1,2-dimethyl-2-(trifluoromethyl)-1H-furo[2,3-c]chromen-4-one (61 g, 78%), with the major isomer believed to be *rac*-(1*S*,2*R*)-6,7-difluoro-1,2-dimethyl-2-(trifluoromethyl)-1H-furo[2,3-c]chromen-4-one. ESI-MS *m/z* calc. 320.04718, found 321.5 $(M+1)^+$; 319.6 $(M-1)^-$.

**Step 7:**

*rac*-(1*S*,2*R*)-6,7-Difluoro-1,2-dimethyl-2-(trifluoromethyl)-1H-furo[2,3-c]chromen-4-one (30 g, 93.69 mmol) was dissolved in EtOAc (400 mL) and stirred with activated charcoal (6 g, 499.6 mmol) (0.2 g/g of substrate) at ambient temperature for 4 hours and 30 minutes. The mixture was filtered through a pad of celite, washing with EtOAc. The filtrate was concentrated *in vacuo* to give a white solid. The white solid was suspended in MeOH (600 mL) and added to a suspension of $Pd(OH)_2$ (13.62 g of 20% w/w, 19.40 mmol) in MeOH (150 mL) in a 2.25 L Parr bottle. The resulting mixture was shaken in the Parr hydrogenator under a hydrogen pressure of 60 psi overnight. The suspension was filtered through celite under a nitrogen atmosphere, rinsed with MeOH and then with EtOAc, and the resulting filtrate was concentrated *in vacuo* to give methyl *rac*-(2*R*,3*S*,4*S*,5*R*)-3-(3,4-difluoro-2-hydroxy-phenyl)-4,5-dimethyl-5-(trifluoromethyl)tetrahydrofuran-2-carboxylate (32.75 g, 99%). $^1H$ NMR (400 MHz, Methanol-d4) $\delta$ 7.05 (ddq, J = 9.4, 5.9, 1.9 Hz, 1H), 6.57 (ddd, J = 10.0, 9.0, 7.6 Hz, 1H), 5.01 (d, J = 6.0 Hz, 1H), 4.34 (dd, J = 8.4, 6.0 Hz, 1H), 3.49 (s, 3H), 3.01 - 2.86 (m, 1H), 1.50 (q, J = 1.2 Hz, 3H), 0.89 (dq, J = 7.6, 1.9 Hz, 3H) ppm. ESI-MS *m/z* calc. 354.08905, found 353.3 $(M-1)^-$.

**Step 8:**

A solution of methyl *rac*-(2*R*,3*S*,4*S*,5*R*)-3-(3,4-difluoro-2-hydroxy-phenyl)-4,5-dimethyl-5-(trifluoromethyl)tetrahydrofuran-2-carboxylate (60.8 g, 171.6 mmol) in THF (620 mL) was cooled to 1 °C, and potassium *tert*-butoxide (65.0472 g, 579.7 mmol) was added over 10 mins, keeping the internal temperature below 10 °C. The mixture was stirred at 0 °C for a further 5 min, and then the mixture was warmed slightly. When the temperature had reached 13 °C, the reaction was cooled down again with an ice bath before adding 2 M HCl (365 mL, to pH 1), keeping the internal temperature below 15 °C. Water (300 mL) was added, the layers were separated, and the aqueous layer was extracted with EtOAc (110 mL). The combined organic extracts were washed with brine (300 mL), dried ($MgSO_4$), filtered and concentrated *in vacuo* to give *rac*-(2*R*,3*S*,4*S*,5*R*)-3-(3,4-difluoro-2-hydroxy-phenyl)-4,5-dimethyl-5-(trifluoromethyl)tetrahydrofuran-2-carboxylic acid (58.22 g, 100%). $^1H$ NMR (400 MHz, Methanol-$d_4$) $\delta$ 7.00 (ddd, J = 8.4, 5.6, 2.3 Hz, 1H), 6.69 (ddd, J = 10.1, 8.8, 7.5 Hz, 1H), 4.98 (d, J = 10.5 Hz, 1H), 4.18 (dd, J = 10.5, 7.6 Hz, 1H), 2.83 (p, J = 7.5 Hz, 1H), 1.59 (q, J = 1.2 Hz, 3H), 0.76 (dq, J = 7.2, 2.2 Hz, 3H) ppm. ESI-MS *m/z* calc. 340.0734, found 339.0 $(M-1)^-$.

Step 9:

To a solution of *rac*-(2*R*,3*S*,4*S*,5*R*)-3-(3,4-difluoro-2-hydroxy-phenyl)-4,5-dimethyl-5-(trifluoromethyl)tetrahydrofuran-2-carboxylic acid (58.39 g, 171.6 mmol) in acetonitrile (300 mL) was added $K_2CO_3$ (82.6 g, 597.7 mmol) and MeI (37 mL, 594.3 mmol). The reaction was heated to 80 °C (internally temperature reached 61 °C) for 5 hours before being cooled to ambient temperature and diluted with DCM (350 mL). The mixture was filtered, washing the filter cake with more DCM (350 mL) and the filtrate was concentrated *in vacuo* to give methyl *rac*-(2*R*,3*S*,4*S*,5*R*)-3-(3,4-difluoro-2-methoxyphenyl)-4,5-dimethyl-5-(trifluoromethyl)tetrahydrofuran-2-carboxylate (64.7 g, 100%) as an orange oil containing some residual $K_2CO_3$. This material was used in the next step without further purification. $^1H$ NMR (400 MHz, Chloroform-d) $\delta$ 6.91 (ddd, J = 7.6, 5.7, 1.9 Hz, 1H), 6.85 (td, J = 9.1, 7.2 Hz, 1H), 4.91 (d, J = 10.2 Hz, 1H), 4.13 (dd, J = 10.2, 8.0 Hz, 1H), 4.00 (d, J = 2.7 Hz, 3H), 3.71 (s, 3H), 2.72 (p, J = 7.7 Hz, 1H), 1.62 (q, J = 1.2 Hz, 3H), 0.76 (dq, J = 7.5, 2.4 Hz, 3H) ppm.

**Step 10:**

Methyl *rac*-(2*R*,3*S*,4*S*,5*R*)-3-(3,4-difluoro-2-methoxy-phenyl)-4,5-dimethyl-5-(trifluoromethyl)tetrahydrofuran-2-carboxylate (63.2 g, 171.6 mmol) was dissolved in MeOH (500 mL) and water (300 mL). $LiOH \cdot H_2O$ (14.8882 g, 354.8 mmol) was added and the resultant mixture stirred at ambient temperature for 2 hours. The MeOH was removed *in vacuo* and the mixture was diluted in MTBE (320 mL). 2 M HCl (440 mL) was added to reach pH 1, the layers were separated and the aqueous layer extracted twice with MTBE (100 mL). The combined organic layers were dried ($MgSO_4$),

filtered and concentrated *in vacuo* to give *rac*-(2*R*,3*S*,4*S*,5*R*)-3-(3,4-difluoro-2-methoxy-phenyl)-4,5-dimethyl-5-(trifluoromethyl)tetrahydrofuran-2-carboxylic acid (60.3 g, 99%) as an orange oil. $^1$H NMR (400 MHz, DMSO-$d_6$) δ 12.96 (s, 1H), 7.40 - 6.82 (m, 2H), 4.96 (dd, J = 15.5, 10.5 Hz, 1H), 4.08 (dd, J = 10.4, 7.6 Hz, 1H), 3.93 (d, J = 2.2 Hz, 3H), 2.67 (p, J = 7.7 Hz, 1H), 1.59 - 1.49 (m, 3H), 0.77 - 0.63 (m, 3H) ppm. ESI-MS *m*/*z* calc. 354.08905, found 353.1 $(M-1)^-$.

**Step 11:**

To a solution of *rac*-(2*R*,3*S*,4*S*,5*R*)-3-(3,4-difluoro-2-methoxy-phenyl)-4,5-dimethyl-5-(trifluoromethyl)tetrahydrofuran-2-carboxylic acid (158.6 g, 447.7 mmol) and DMF (135 μL, 1.74 mmol) in DCM (1.5 L) stirring at 0 °C under nitrogen was added oxalyl chloride (79 mL, 905.6 mmol) *via* dropping funnel, over 30 mins. Halfway through addition the ice bath was removed and the mixture allowed to warm ambient temperature over the remainder of the addition. The mixture was stirred at ambient temperature for a further 1 hour before being evaporated *in vacuo.* The residue was dissolved in DCM (700 mL) and added via dropping funnel to a solution of methyl 4-aminopyridine-2-carboxylate (81.5 g, 535.7 mmol), DMF (135 μL, 1.744 mmol) and $Et_3N$ (95 mL, 681.6 mmol) in DCM (780 mL) stirring at -10 °C. The rate of addition was controlled so as to keep internal temperature below 5 °C (~15 mins). Following addition, the mixture was diluted in water (600 mL), the layers were separated and the aqueous phase was further extracted with DCM (100 mL). Solid formed at the interface between the layers and was collected by filtration to provide filtered desired product (43.2g). The filtrate was washed further with water (600 mL), dried ($MgSO_4$), filtered and concentrated *in vacuo.* The residue was suspended in MeOH (360 mL) and stirred rapidly for 20 mins. The mixture was filtered and the solid washed with MeOH and dried under vacuum for 30 mins. This material was combined with the previously obtained product to give methyl *rac*-(2*R*,3*S*,4*S*,5*R*)-4-[[3-(3,4-difluoro-2-methoxy-phenyl)-4,5-dimethyl-5-(trifluoromethyl)tetrahydrofuran-2-carbonyl]amino]pyridine-2-carboxylate (166.2 g, 76%) as a white solid. $^1$H NMR (500 MHz, DMSO-$d_6$) δ 10.74 (s, 1H), 8.57 (d, J = 5.4 Hz, 1H), 8.36 (d, J = 2.0 Hz, 1H), 7.85 (dd, J = 5.5, 2.2 Hz, 1H), 7.16 (qd, J = 9.2, 6.3 Hz, 2H), 5.11 (d, J = 10.1 Hz, 1H), 4.25 (dd, J = 10.2, 7.7 Hz, 1H), 3.95 (d, J = 2.0 Hz, 3H), 3.87 (s, 3H), 2.77 (p, J = 7.6 Hz, 1H), 1.61 (s, 3H), 0.81 - 0.65 (m, 3H) ppm. ESI-MS *m*/*z* calc. 488.13705, found 489.6 $(M+1)^+$; 487.6 $(M-1)^-$.

**Step 12:**

Methanolic ammonia (3 L of 7 M, 21.00 mol) was added to methyl *rac*-(2*R*,3*S*,4*S*,5*R*)-4-[[3-(3,4-difluoro-2-methoxy-phenyl)-4,5-dimethyl-5-(trifluoromethyl)tetrahydrofuran-2-carbonyl]amino]pyridine-2-carboxylate (166 g, 339.9 mmol) and the reaction stirred at ambient temperature overnight. The mixture was concentrated *in vacuo* to give *rac*-(2*R*,3*S*,4*S*,5*R*)-4-[[3-(3,4-difluoro-2-methoxy-phenyl)-4,5-dimethyl-5-(trifluoromethyl)tetrahydrofuran-2-carbonyl]amino]pyridine-2-carboxamide (173 g) as an off-white solid, which was used in the next step without further purification. ESI-MS *m*/*z* calc. 473.1374, found 474.6 $(M+1)^+$; 472.6 $(M-1)^-$.

**Step 13:**

*rac*-(2*R*,3*S*,4*S*,5*R*)-4-[[3-(3,4-difluoro-2-methoxy-phenyl)-4,5-dimethyl-5-(trifluoromethyl) tetrahydrofuran-2-carbonyl]amino]pyridine-2-carboxamide (670 mg, 1.415 mmol) was purified by chiral SFC (using a (R'R) Whelk O-1 column, 3-5 μm particle size, 5.0 cm x 3.0 mm from Regis Technologies with Solvent A: liquid $CO_2$ [58-60 bar/40 °C; Solvent B: methanol HPLC grade with 20 mM $NH_3$ on a UPC2-SFC instrument from Waters Corp.) to give:

**First Eluting Isomer:** (2*S*,3*R*,4*R*,5*S*)-4-[[3-(3,4-difluoro-2-methoxy-phenyl)-4,5-dimethyl-5-(trifluoromethyl)tetrahydrofuran-2-carbonyl]amino]pyridine-2-carboxamide (6, 198 mg): $^1$H NMR (500 MHz, Methanol-$d_4$) δ 8.52 (d, J = 5.5 Hz, 1H), 8.30 (d, J = 2.0 Hz, 1H), 7.94 (dd, J = 5.5, 2.2 Hz, 1H), 7.16 (ddd, J = 8.2, 5.6, 2.3 Hz, 1H), 7.02 (ddd, J = 9.9, 8.9, 7.5 Hz, 1H), 5.12 (d, J = 10.4 Hz, 1H), 4.37 (dd, J = 10.4, 8.0 Hz, 1H), 4.03 (d, J = 2.2 Hz, 3H), 2.84 (p, J = 7.6 Hz, 1H), 1.70 (d, J = 1.1 Hz, 3H), 0.86 (dq, J = 7.4, 2.4 Hz, 3H) ppm. ESI-MS *m*/*z* calc. 473.1374, found 474.6 $(M+1)^+$; 472.7 $(M-1)^-$.

**Second Eluting Isomer:** (2*R*,3*S*,4*S*,5*R*)-4-[[3-(3,4-difluoro-2-methoxy-phenyl)-4,5-dimethyl-5-(trifluoromethyl)tetrahydrofuran-2-carbonyl]amino]pyridine-2-carboxamide **(Compound 1,** 195 mg): $^1$H NMR (500 MHz, Methanol-$d_4$) δ 8.39 (d, J = 5.5 Hz, 1H), 8.16 (d, J = 2.0 Hz, 1H), 7.80 (dd, J = 5.5, 2.0 Hz, 1H), 7.02 (ddd, J = 8.2, 5.7, 2.4 Hz, 1H), 6.88 (ddd, J = 9.9, 8.8, 7.5 Hz, 1H), 4.98 (d, J = 10.4 Hz, 1H), 4.23 (dd, J = 10.4, 7.9 Hz, 1H), 3.89 (d, J = 2.2 Hz, 3H), 2.70 (p, J = 7.6 Hz, 1H), 1.56 (d, J = 1.1 Hz, 3H), 0.72 (dq, J = 7.6, 2.4 Hz, 3H) ppm. ESI-MS *m*/*z* calc. 473.1374, found 474.6 $(M+1)^+$; 472.8 $(M-1)^-$.

The absolute stereochemistry of the first and second eluting isomer was determined by single-crystal X-ray crystallography of Compound 1.

***Compound 1 - Solid Form A***

Crystallization of Compound 1 in methanol at 60 °C produced a crystalline form of Compound 1, which is referred to herein as Form A. Form A was characterized by XRPD, TGA, and DSC analysis.

The XRPD pattern of Form A is depicted in Figure 1, and the corresponding data are summarized in the following table:

| Angle (° 2θ ± 0.2) | Rel. Intensity (%) |
|---|---|
| 7.3 | 19.9 |
| 9.9 | 28.3 |
| 13.9 | 100.0 |
| 15.7 | 20.1 |
| 19.0 | 50.4 |
| 20.1 | 31.8 |
| 20.3 | 19.3 |
| 25.4 | 14.7 |

The TGA thermogram of Form A is depicted in Figure 2 and shows negligible weight loss from ambient temperature up until thermal degradation.

The DSC thermogram of Form A is depicted in Figure 3 and shows a melting onset of 186 °C with a peak at 187 °C.

***Compound 1 - Solid Form B***

Compound 1 was dissolved in ethyl acetate (6 volumes) at 68 °C. The mixture was cooled to 50 °C over 1 hour, and *n*-heptane (6 volumes) was added over 5 hours. The mixture was then cooled to 20 °C over a further 5 hours and held overnight. The resulting solid material was filtered, washed with heptane (3 volumes), and dried to produce a crystalline form of Compound 1, which is referred to herein as Form B. Form B was characterized by XRPD, solid state NMR ($^{13}C$ and $^{19}F$), TGA, DSC, IR, and single-crystal X-ray analysis.

The XRPD pattern of Form B is depicted in Figure 4, and the corresponding data are summarized in the following table:

| Angle (° 2θ ± 0.2) | Rel. Intensity (%) |
|---|---|
| 7.6 | 11.3 |
| 9.2 | 10.5 |
| 12.0 | 10.0 |
| 12.8 | 36.7 |
| 14.1 | 59.3 |
| 15.1 | 24.0 |
| 15.2 | 39.4 |
| 16.2 | 23.9 |
| 16.9 | 31.9 |
| 17.6 | 15.1 |
| 18.4 | 63.1 |
| 18.5 | 100.0 |
| 18.7 | 51.7 |
| 19.3 | 64.2 |
| 20.3 | 64.6 |

(continued)

| Angle (° 2θ ± 0.2) | Rel. Intensity (%) |
|---|---|
| 21.7 | 11.6 |
| 22.0 | 29.3 |
| 22.2 | 29.7 |
| 22.9 | 15.1 |
| 23.6 | 27.3 |
| 24.0 | 10.9 |
| 24.2 | 16.8 |
| 25.2 | 30.0 |
| 26.9 | 15.6 |
| 27.0 | 10.7 |
| 27.4 | 17.0 |
| 28.6 | 10.8 |
| 28.9 | 20.9 |

The solid state $^{13}C$ NMR spectrum of Form B is depicted in Figure 5, and the corresponding data are summarized in the following table:

| Chemical Shift [ppm] | Rel. Intensity (%) |
|---|---|
| 172.5 | 23.1 |
| 172.1 | 29.4 |
| 168.5 | 18.8 |
| 168.3 | 17.8 |
| 168.0 | 20.1 |
| 151.5 | 36.8 |
| 148.3 | 100.0 |
| 147.8 | 35.0 |
| 127.7 | 83.3 |
| 122.7 | 70.4 |
| 116.6 | 53.1 |
| 115.1 | 44.5 |
| 110.6 | 51.6 |
| 86.5 | 13.0 |
| 80.2 | 60.4 |
| 63.2 | 42.3 |
| 44.3 | 99.1 |
| 23.0 | 51.8 |
| 13.1 | 51.7 |

The solid state $^{19}F$ NMR spectrum of Form B is depicted in Figure 6, and the corresponding data are summarized in the following table:

| Chemical Shift [ppm] | Rel. Intensity |
|---|---|
| -137.1 | 12.5 |
| -152.8 | 5.8 |

The TGA thermogram of Form B is depicted in Figure 7 and shows negligible weight loss from ambient temperature up until thermal degradation.

The DSC thermogram of Form B is depicted in Figure 8 and shows a melting onset of 182 °C with a peak at 183 °C.

The IR spectrum of Form B is depicted in Figure 9 and includes peaks at 3501, 3356, 1684, 1565, 1505, and 1122 $cm^{-1}$.

Crystals having Form B were grown for single-crystal X-ray analysis by dissolving 1 mg of Compound 1 material in 500 μL of ethanol, which was allowed to evaporate slowly over several days. The thermal ellipsoid plot, at 50% probability, is depicted in Figure 10, and the unit cell parameters are reported in the following table:

| | |
|---|---|
| Crystal System: | Orthorhombic |
| Space Group: | $P2_12_12_1$ |
| a (Å) | 7.3929(2) |
| b (Å) | 14.5827(4) |
| c (Å) | 18.9312(6) |
| a (°) | 90 |
| β (°) | 90 |
| γ (°) | 90 |
| V ($Å^3$) | 2040.94(10) |
| Z | 4 |
| Temperature | 100K |

Example 2

E-VIPR Assay Detecting and Measuring $Na_V$ Inhibition Properties

Sodium ion channels are voltage-dependent proteins that can be activated by inducing membrane voltage changes by applying electric fields. The electrical stimulation instrument and methods of use, referred to as E-VIPR, are described in International Publication No. WO 2002/008748 A3 and C.-J. Huang et al. Characterization of voltage-gated sodium channel blockers by electrical stimulation and fluorescence detection of membrane potential, 24 Nature Biotech. 439-46 (2006). The instrument comprises a microtiter plate handler, an optical system for exciting the coumarin dye while simultaneously recording the coumarin and oxonol emissions, a waveform generator, a current- or voltage-controlled amplifier, and parallel electrode pairs that are inserted into assay plate wells. Under integrated computer control, this instrument passes user-programmed electrical stimulus protocols to cells within the wells of the microtiter plate.

16-20 hours prior to running the assay on E-VIPR, HEK cells expressing a truncated form of human $Na_V$ 1.8 with full channel activity were seeded into microtiter 384-well plates, pre-coated with matrigel, at a density of 25,000 cells per well. 2.5-5% KIR2.1 Bacmam virus was added to the final cell suspension before seeding into cell plates. HEK cells were grown in Dulbecco's Modified Eagle's Medium (DMEM) supplemented with 10% FBS (Fetal Bovine Serum, qualified; Sigma #F4135), 1% NEAA (Non-Essential Amino Acids, Gibco #11140), 1% HEPES (Gibco #15630), 1% Pen-Strep (Penicillin-Streptomycin; Gibco #15140) and 5 μg/ml Blasticidin (Gibco #R210-01). Cells were expanded in vented cap cell culture flasks, with 90-95% humidity and 5% $CO_2$.

Reagents and Stock Solutions:

100 mg/mL Pluronic F-127 (Sigma #P2443), in dry DMSO
Compound Plates: Corning 384-well Polypropylene Round Bottom #3656

Cell Plates: 384-well tissue culture treated plates (Greiner #781091-2B)
2.5-5% KIR 2.1 Bacmam virus (produced in-house), prepared as described in Section 3.3 of J. A. Fornwald et al., Gene Expression in Mammalian Cells Using BacMam, a Modified Baculovirus System, 1350 Methods in Molecular Biology 95-116 (2016). The concentration used can be dependent on viral titer of each batch.

5 mM $DiSBAC_6(3)$, a voltage sensitive oxonol acceptor (CAS number 169211-44-3; 5-[3-(1,3-dihexylhexahydro-4,6-dioxo-2-thioxo-5-pyrimidinyl)-2-propen-1-ylidene]-1,3-dihexyldihydro-2-thioxo-4,6(1H,5H)-pyrimidinedione), in dry DMSO. The preparation of $DiSBAC_6(3)$ is analogous to that of $DiSBAC_4(3)$ as described in Voltage Sensing by Fluorescence Resonance Energy Transfer in Single Cells, Gonzalez, J.E. and Tsien, R.Y. (1995) Biophys. J. 69, 1272-1280.

5 mM CC2-DMPE, a commercially available membrane-bound coumarin phospholipid FRET donor (ThermoFisher Scientific catalog number K1017, CAS number 393782-57-5; tetradecanoic acid, 1,1'-[(1R)-1-[8-(6-chloro-7-hydroxy-2-oxo-2H-1-benzopyran-3-yl)-3-hydroxy-3-oxido-8-oxo-2,4-dioxa-7-aza-3-phosphaoct-1-yl]-1,2-ethanediyl] ester) was prepared in dry DMSO. See also, Improved indicators of cell membrane potential that use fluorescence resonance energy transfer, Gonzalez, J.E. and Tsien, R.Y. (1997) Chem. Biol. 4, 269-277.

Voltage Assay Background Suppression Compound (VABSC-1) is prepared in $H_2O$ (89-363 mM, range used to maintain solubility)

Human Serum (HS, Millipore #S1P1-01KL, or Sigma SLBR5469V and SLBR5470V as a 50%/50% mixture, for 25% assay final concentration)

Bath 1 Buffer:
Sodium Chloride 160 mM (9.35 g/L), Potassium Chloride, 4.5 mM (0.335 g/L), Glucose 10 mM (1.8 g/L), Magnesium Chloride (Anhydrous) 1 mM (0.095 g/L), Calcium Chloride 2 mM (0.222 g/L), HEPES 10 mM (2.38 g/L) in water.
Na/TMA Cl Bath 1 Buffer:

Sodium Chloride 96 mM (5.61 g/L), Potassium Chloride 4.5 mM (0.335 g/L),
Tetramethylammonium (TMA)-Cl 64 mM (7.01 g/ L), Glucose 10 mM (1.8 g/L),
Magnesium Chloride (Anhydrous) 1 mM (0.095 g/L), Calcium Chloride 2 mM (0.222 g/L) HEPES 10 mM (2.38 g/L) in water.

Hexyl Dye Solution (2X concentration):
Bath 1 Buffer containing 0.5% β-cyclodextrin (made fresh prior to each use, Sigma #C4767), 8 μM CC2-DMPE and 2 μM $DiSBAC_6(3)$. The solution was made by adding 10% Pluronic F127 stock equal to combined volumes of CC2-DMPE and $DiSBAC_6(3)$. The order of preparation was first mix Pluronic and CC2-DMPE, then add $DiSBAC_6(3)$, then while vortexing add Bath 1/β-Cyclodextrin.

Compound Loading Buffer (2X concentration): Na/TMA Cl Bath1 Buffer containing HS (omitted in experiments run in the absence of human serum (HS))50%, VABSC-1 1 mM, BSA 0.2 mg/ml (in Bath-1), KCl 9 mM, DMSO 0.625%.

Assay Protocol (7 key Steps):

1) To reach the final concentration in each well, 375 nL of each compound was pre-spotted (in neat DMSO) into polypropylene compound plates at 240x desired final concentration from an intermediate stock concentration of 0.075 mM, in an 11 point dose response, 3-fold dilution, resulting in a top dose of 300 nM final concentration in the cell plate. Vehicle control (neat DMSO), and positive control (an established $Na_V1.8$ inhibitor, 25 μM final in assay in DMSO) were added manually to the outermost columns of each plate respectively. The compound plate was backfilled with 45 μL per well of Compound Loading Buffer resulting in a 240 fold dilution of compound following a 1:1 transfer of compound into the cell plate (see Step 6). Final DMSO concentration for all wells in the assay was 0.625% (0.75% DMSO was supplemented to the Compound Loading Buffer for a final DMSO concentration of 0.625%). This assay dilution protocol was adjusted to enable a higher dose range to be tested in the presence of HS or if the final assay volume was altered.
2) Hexyl Dye Solution was prepared.
3) Cell plates were prepared. On the day of the assay, the media was aspirated, and the cells were washed three times with 80 μL of Bath-1 buffer, maintaining 25 μL residual volume in each well.
4) 25 μL per well of Hexyl Dye Solution was dispensed into the cell plates. The cells were incubated for 20 minutes at room temperature or ambient conditions in darkness.

5) 45 μL per well of Compound Loading Buffer was dispensed into compound plates.
6) The cell plates were washed three times with 80 μL per well of Bath-1 Buffer, leaving 25 μL of residual volume. Then 25 μL per well from compound plate was transferred to each cell plate. The mixture was incubated for 30 minutes at room temperature/ambient conditions.
7) The cell plate containing compound was read on E-VIPR using the current-controlled amplifier to deliver stimulation wave pulses using a symmetrical biphasic waveform. The user-programmed electrical stimulus protocols were 1.25-4 Amps and 4 millisecond pulse width (dependent on electrode composition) were delivered at 10 Hz for 10 seconds. A pre-stimulus recording was performed for each well for 0.5 seconds to obtain the un-stimulated intensities baseline. The stimulatory waveform was followed by 0.5 seconds of post-stimulation recording to examine the relaxation to the resting state. All E-VIPR responses were measured at 200 Hz acquisition rate.

Data Analysis:

Data were analyzed and reported as normalized ratios of emission intensities measured in the 460 nm and 580 nm channels. The response as a function of time was reported as the ratios obtained using the following formula:

$$R(t) = \frac{(\text{intensity } 460 \text{ nm})}{(\text{intensity } 580 \text{ nm})}$$

The data were further reduced (i.e. normalized) by calculating the initial ($R_i$) and final ($R_f$) ratios. These were the average ratio values during part or all of the pre-stimulation period and during sample points during the stimulation period. The fluorescence ratio ($R_f/R_i$) was then calculated and reported as a function of time.

Control responses were obtained by performing assays in the presence of the positive control, and in the absence of pharmacological agents (DMSO vehicle negative control). Responses to the negative (*N*) and positive (*P*) controls were calculated as above. The compound antagonist % activity *A* was then defined as:

$$A = \frac{X - N}{P - N} \times 100$$

where X is the ratio response of the test compound (i.e. the maximum amplitude of the ratio response or number of action potential peaks, at the beginning of the pulse train in the presence of the test compound). Using this analysis protocol, a dose response curve was plotted and an $IC_{50}$ value of < 0.01 μM was determined for Compound 1 of the present invention.

Example 3

SDD Tablet Process

***Step 1: Preparation of Compound 1 Spray-Dried Dispersion (SDD)***

About 2466.7 gm of dichloromethane and about 2466.7 gm of methanol were combined in a glass vessel. To the resulting mixture was added about 100 gm of Compound 1 at room temperature. Upon complete dissolution of Compound 1, about 300 gm of HPMCAS was added to the mixture at room temperature. Upon complete dissolution of the HPMCAS, the resulting mixture was spray-dried using a MicraSpray (MS-35) spray dryer with a 0.8 mm two fluid nozzle, 3 mm spacer, 2.6 mm air cap, 150 mesh PTFE inline filter, and a collection container attached to the cycle and pulseback filters. The process parameters of the MS-35 spray dryer are summarized in the following table:

**Spray Dryer Process Parameters**

| Parameter | Target | (+ / -) Range |
|---|---|---|
| Outlet Temp (°C) | 48.0 | 45.0-51.0 |
| Process Gas Flow (kg/hr) | 35.0 | 33-37 |
| Nozzle Gas Flow (kg/hr) | 4.5 | 4.2-4.8 |
| Solution Feed Rate (kg/hr) | | |
| Solution Feed Rate (gm/min) | 30.0 | 27-33 |
| Estimated Spray time (hr) | 3.0 | |

To begin spray drying, the MS-35 was preheated, and when the target outlet temperature of 48 °C was reached an equilibrium solution was sprayed until all the parameters were stable and within the target range. Once all the parameters have stabilized and are within the target range the MS-35 spray dryer began spraying the solution containing Compound 1 and the polymer. The process parameters may be adjusted during the run to maintain it within the working range.

The wet SDD was transferred to appropriate size trays. Each tray was filled to a depth of about 1 inch of powder and placed in a vacuum oven at 40 °C with nitrogen purging. After 12-72 hours samples were pulled from the trays to check for residual solvent levels. Once the solvent levels were below the specifications (dichloromethane (<600 ppm) and methanol (<3000 ppm)) all powder from the trays were combined.

The SDD was transferred to a secondary drying chamber for further drying. Upon completion of drying, X-ray powder diffraction (XRPD) analysis was performed at room temperature in reflection mode using a PANalytical Empyrean system equipped with a sealed tube source and a PIXcel 1D Medipix-3 detector (Malvern PANalytical Inc, Westborough, Massachusetts). The X-Ray generator operated at a voltage of 45 kV and a current of 40 mA with copper radiation (1.54060Å). The powder sample was placed in a back-loading sample holder and loaded into the instrument. The sample was scanned over the range of about 3° to about 40°2θ with a step size of 0.0131° and 48.195 s per step. The obtained XRPD diffractogram is depicted in Figure 11.

The composition of the SDD is summarized in Table 1:

**Table 1 Composition of Compound 1 Spray-Dried Dispersion**

| Component | Content (% w/w) |
|---|---|
| Compound 1 | 25.0 |
| Hydroxypropyl Methylcellulose Acetate Succinate | 75.0 |
| **Total** | **100.0** |

***Step 2: Preparation of Tablet Comprising Compound 1 SDD***

Compound 1 SDD from Step 1, microcrystalline cellulose, lactose monohydrate, and croscarmellose sodium were each passed through a 20 mesh screen and combined in a 10 L Bohle blender. The mixture was blended for about 2.5 minutes at 32 rpm. Sodium stearyl fumarate was passed through a 60 mesh screen and added to the blended mixture and further blended for about 1.5 minutes at 32 rpm.

The resulting blended mixture was dry granulated using a GERTEIS® roller compactor and an in-line mill. The resulting milled granules were added to a 5 L Bohle blender along with microcrystalline cellulose and croscarmellose sodium that was passed through a 20 mesh screen. The resulting mixture was blended for 8.5 minutes at 32 rpm. After blending, sodium stearyl fumarate that was passed through a 60 mesh screen was added to the blended mixture and blended for an additional 2 minutes at 32 rpm. Process parameters for the roller compactor and in-line mill are summarized in Table 2:

**Table 2 Process Parameters**

| Parameter | Target | (+ / -) Range |
|---|---|---|
| Roll Gap (mm) | 2.0 | 1.0-3.0 |
| Roll Pressure (kN/cm) | 9 | 6-12 |
| Roll Speed (rpm) | 2 | 1.5-2.5 |
| Gap Control | On | |
| Agitator speed (rpm) | 15 | |
| Granulation Speed cw/ccw (rpm) | 80/80 | 60-100/60-100 |

(continued)

| Parameter | Target | (+ / -) Range |
|---|---|---|
| Oscillation (cw/ccw) (deg) | 330-360 | 320-340/350-370 |
| PID "P value" | 3 | NA |
| PID "I value" | 3000 | 2000-7000 |
| PID "D value" | 0 | NA |
| Torque Control | off | |
| Tamp/Feed Ratio (%) | 165 | 110-500 |
| Feed Factor | 0.3 | 0.1-0.9 |
| Roll type | Smooth/Smooth | |
| Screen Size (mm) | 1.00 | |
| Rotor type | Pocketed rotor | |

The resulting mixture was then compressed into tablets using a PICCOLA® tablet press to yield tablets with each containing 10 mg of Compound 1. The press had a 8 mm standard round concave tooling and paddle feeder. The turret speed was set to 30 rpm and the paddle speed was set to 25 rpm. The target weight and hardness of the tablets is summarized in Table 3:

Table 3

| | Weight (mg) | Hardness (kP) |
|---|---|---|
| Target | 200 | 8.00 |
| Acceptance Limits | 180-220 | 6.0-10.0 |

The resulting tablets were analyzed by X-ray powder diffraction (XRPD) and solid state nuclear magnetic resonance (ssNMR) after being ground into a uniform powder. XRPD analysis was performed at room temperature in reflection mode using a PANalytical Empyrean system equipped with a sealed tube source and a PIXcel 1D Medipix-3 detector (Malvern PANalytical Inc, Westborough, Massachusetts). The X-Ray generator operated at a voltage of 45 kV and a current of 40 mA with copper radiation (1.54060 Å). The powder sample was placed in a back-loading sample holder and loaded into the instrument. The sample was scanned over the range of about 3° to about 40°2θ with a step size of 0.0131° and 48.195s per step, and scanned over the range of about 14° to about 16°2θ with a step size of 0.0131° and 1497.870s per step. The obtained XRPD diffractogram is depicted in Figures 12A and 12B.

Solid state NMR analysis was conducted on a Bruker-Biospin 400 MHz wide-bore spectrometer equipped with Bruker-Biospin 4mm HFX probe was used. Samples were packed into 4mm $ZrO_2$ rotors and spun under Magic Angle Spinning (MAS) condition with spinning speed typically set to 12.5 kHz. The proton relaxation time was measured using $^{1}H$ MAS $T_1$ saturation recovery relaxation experiment in order to set up proper recycle delay of the $^{13}C$ cross-polarization (CP) MAS experiment. The fluorine relaxation time was measured using $^{19}F$ MAS $T_1$ saturation recovery relaxation experiment in order to set up proper recycle delay of the $^{19}F$ MAS experiment. The CP contact time of carbon CPMAS experiment was set to 2 ms. A CP proton pulse with linear ramp (from 50% to 100%) was employed. The carbon Hartmann-Hahn match was optimized on external reference sample (glycine). Both carbon and fluorine spectra were recorded with proton decoupling using TPPM15 decoupling sequence with the field strength of approximately 100 kHz. The obtained fluorine and carbon NMR spectra are depicted in Figures 13A and 13B, respectively, and the peak lists appear in Tables 4 and 5.

**Table 4. $^{19}F$ MAS peak list:**

| Peak # | δ [ppm] | Intensity |
|---|---|---|
| 1 | -40.1 | 1.51E0 |
| 2 | -73.2 | 1.23E1 |
| 3 | -105.9 | 4.26E0 |
| 4 | -121.8 | 3.33E0 |
| 5 | -137.9 | 4.80E0 |
| 6 | -155.6 | 6.33E0 |
| 7 | -171.1 | 8.30E0 |

(continued)

| Peak # | δ [ppm] | Intensity |
|---|---|---|
| 8 | -220.6 | 1.49E0 |

**Table 5. $^{13}C$ CPMAS peak list:**

| Peak # | δ [ppm] | Intensity |
|---|---|---|
| 1 | 170.6 | 8.85E0 |
| 2 | 151.5 | 4.48E0 |
| 3 | 125.2 | 3.23E0 |
| 4 | 92.6 | 3.24E0 |
| 5 | 89.2 | 2.87E1 |
| 6 | 84.3 | 2.33E1 |
| 7 | 75.1 | 9.99E1 |
| 8 | 72.7 | 9.87E1 |
| 9 | 65.3 | 3.92E1 |
| 10 | 61.7 | 3.37E1 |
| 11 | 44.5 | 3.52E0 |
| 12 | 33.2 | 1.92E1 |
| 13 | 31.0 | 4.74E0 |
| 14 | 27.4 | 4.85E0 |
| 15 | 20.7 | 1.11E1 |

**Table 6. Tablet Compositions, 10 mg**

| Component | Content (% w/w) | Amount per Tablet (mg) |
|---|---|---|
| Compound 1 SDD | 20.00 | 40.00 |
| Microcrystalline Cellulose (Avicel PH101)* | 25.0 | 95.00 |
| Microcrystalline Cellulose (Avicel PH200)** | 22.5 | |
| Lactose Monohydrate | 25.00 | 50.00 |
| Croscarmellose Sodium | 4.50 | 9.00 |
| Sodium Stearyl Fumarate | 3.00 | 6.00 |
| **Total** | **100.0** | **200.0** |
| *Avicel PH101 has a particle size of about 50 microns.<br>* *Avicel PH200 has a particle size of about 180 microns. | | |

Example 3A

SDD (25% DL) Tablets

**Table 7. Tablet Compositions, 10 mg, 20 mg, and 50 mg**

| | Component | % wt Coated Tablet | mg/tablet 10 mg | mg/tablet 20 mg | mg/tablet 50 mg |
|---|---|---|---|---|---|
| Intragranular blend | Compound 1 SDD | 48.54 | 40.0 | 80.0 | 200.0 |
| | Microcrystalline Cellulose (Avicel PH101) | 22.57 | 18.6 | 37.2 | 93.0 |
| | Croscarmellose sodium | 1.46 | 1.2 | 2.4 | 6.0 |
| | Magnesium stearate | 0.24 | 0.2 | 0.4 | 1.0 |
| Extragranular blend | Microcrystalline Cellulose (Acivel PH102) | 22.09 | 18.2 | 36.4 | 91.0 |
| | Croscarmellose sodium | 1.46 | 1.2 | 2.4 | 6.0 |
| | Magnesium stearate | 0.73 | 0.6 | 1.2 | 3.0 |
| | Opadry Blue | 2.91 | 2.4 | 4.8 | 12.0 |
| | Core Tablet Total | | 80.0 | 160.0 | 400.0 |
| | Final Tablet Total | 100.00 | 82.4 | 164.8 | 412.0 |

**Table 8. Tablet Compositions, 10 mg and 50 mg**

| Component | % wt Final Blend | mg/tablet 10 mg | mg/tablet 50 mg |
|---|---|---|---|
| Compound 1 SDD (25% DL) | 50.00 | 40.0 | 200.0 |
| Microcrystalline Cellulose | 44.50 | 35.6 | 178.0 |
| Croscarmellose sodium | 4.50 | 3.6 | 18.0 |
| Magnesium stearate | 1.00 | 0.8 | 4.0 |
| Total | 100% | 80 mg | 400 mg |
| CCS IG/EG ratio: 75/25<br>MgST IG/EG ration: 25/75<br>Grannules wt% in final blend: 75% | | | |

Example 4: A Study of the Efficacy and Safety of Compound **1** in Subjects with Pain Following Bunionectomy

A randomized, double-blind, placebo-controlled, 5-arm, parallel-design study to evaluate the efficacy and safety of Compound **1** on acute surgical pain is conducted. Bunionectomy is a well-established, multi-dose, surgical, acute pain model. A randomized, double-blind study design was used to avoid observer bias and reduce symptoms or outcomes arising from the subjects' knowledge of treatment. An opioid reference arm assessing a standard-of-care treatment (hydrocodone bitartrate (5 mg)/acetaminophen (325 mg) (HB/APAP)) was included to establish the ability of the study to successfully observe a treatment effect for Compound **1.**

Study Subjects

Male and female patients between the ages of 18 and 75 years (inclusive) with pain that is ≥4 on an 11-point Numeric Pain Rating Scale (NPRS) and is moderate or severe on the Verbal Categorical Rating Scale (VRS) after bunionectomy are included in the study.

Study Drugs

Investigational Drug: Compound **1.** The investigational drug was administered orally in 10 mg tablets. Tablets were prepared according to Example 3. The investigational drug was administered every 12 hours (q12h). A low, mid, and high dosing regime were tested. The first dose in the low dosing regime was 20 mg, and the subsequent doses were 10 mg q12h. The first dose in the mid dosing regime was 60 mg, and the subsequent doses were 30 mg q12h. The first dose in the high dosing regime was 100 mg, and the subsequent doses were 50 mg q12h.

Reference Drug: HB/APAP. The reference drug was administered orally in 5 mg/325 mg capsules, supplied as

over-encapsulated 5 mg/325 mg tablets. The reference drug was administered in a dose of 5 mg/325 mg every 6 hours (q6h).

Study Protocol

The schedule for the study is summarized in Table 9. After a screening period, subjects receive a primary unilateral first metatarsal bunionectomy repair on Day minus one under regional anesthesia (Mayo and popliteal block). A continuous popliteal sciatic block infusion (0.2% ropivacaine) is started after surgery, and remains in place until approximately 3 AM on Day 1. After removal of the popliteal sciatic block, each subject is randomized when the subject requests the first dose of study drug for pain relief and reports pain ≥ 4 on the NPRS and moderate or severe pain on the VRS. The NPRS and VRS criteria are designed to ensure subjects have sufficient pain to determine if the study drugs are effective. Subjects that do not meet the NPRS and VRS criteria within 9 hours of removal of the popliteal sciatic block are not enrolled in the study.

**Table 9**. Study Schedule

| Study Day | Event |
|---|---|
| Day -28 | Beginning of screening period |
| Day -1 | Bunionectomy |
| Day 1 (approximately 3 AM) | Removal of popliteal block |
| Day 1 | Randomization |
| Days 1-3 | Administration of study drug |
| Days 12-16 | Safety follow-up phone interview |

264 subjects were randomized approximately 2:2:1:2:2 to five treatment groups: Compound **1** (high dose); Compound 1 (mid dose); Compound **1** (low dose); HB/APAP (opiod reference); or placebo (*see* Table 2). Randomization was stratified by site and baseline NPRS (<8 versus ≥8). To maintain the blind, all subjects receive the same number of capsules in a double-dummy design.

**Table 10**. Compound 1 Treatment Groups

| Treatment | Active Dose | Number of Subjects |
|---|---|---|
| Compound **1** (high dose) | 100 mg first dose, then 50 mg q12h | 60 |
| Compound **1** (mid dose) | 60 mg first dose, then 30 mg q12h | 62 |
| Compound **1** (low dose) | 20 mg first dose, then 10 mg q12h | 33 |
| HB/APAP | 5 mg/325 mg q6h | 60 |
| Placebo | | 59 |

Compound 1 was administered every 12 hours (q12h). The final dose of Compound 1 is given 36 hours after the first dose. HB/APAP placebo capsules were administered every 6 hours (q6h).

In the Reference Arm, HB/APAP was administered every 6 hours (q6h) at a dose of 5 mg/325 mg. The final dose of HB/APAP was given 42 hours after the first dose. Compound 1 placebo capsules are administered every 12 hours (q12h).

In the Placebo Arm, Compound 1 placebo capsules were administered every 12 hours (q12h), and HB/APAP placebo capsules were administered every 6 hours (q6h).

Subjects had a Safety Follow-up Phone Interview 14 (± 2) days after the last study drug dose for the purpose of collecting information on adverse events, medications, and treatments and procedures.

Efficacy Assessments

**11-point (0 to 10) Numeric Pain Rating Scale:** NPRS scores are frequently used in bunionectomy studies and are recognized by the FDA as a valid pain intensity measure. On the 11-point NPRS, a score of 0 denotes no pain, and a score of 10 denotes the worst pain intensity imaginable. Subjects reported their pain on the 11-point NPRS immediately before their first dose of the study drug (baseline NPRS score) and at intervals over 48 hour period (i.e. 0.5, 1, 1.5, 2, 3, 4, 5, 6, 8, 12, 16, 20, 24, 28, 32, 36, 40, 44, and 48 hours (±5 minutes)) after the first dose of the study drug. Pain intensity is also

recorded on the NPRS immediately before each administration of rescue medication. Pain intensity scores that are collected within 4 hours after a dose of rescue medication are deemed missing, and the missing values are imputed as the intensity recorded immediately before the administration of rescue medication.

SPID-48 is the sum of the pain intensity difference (PID) over the 48 hour time period. A pain intensity score of 0 (no pain) to 10 (worse possible pain) is obtained before starting the study and throughout the 48 time period. The pain score at each assessment time is subtracted from the baseline pain score to provide the total sum score or SPID-48. A higher SPID-48 is better and indicates a reduction in pain intensity compared to the baseline score.

**4-point Verbal Categorical Rating Scale:** Subjects report their pain on the 4-point VRS (none, mild, moderate, or severe) immediately before their first dose of the study drug (baseline VRS).

Efficacy Results

**SPID48 Scores.** The SPID scores for the subjects in the Placebo Arm, Investigational Arm, and Reference Arm appear Table 11 below.

**Table 11. Bunionectomy Efficacy Results**

| Treatment Group: | **Placebo** n=59 | **High-dose Compound 1** (100 mg first dose/50 mg every 12 hours) n=60 | **Mid-dose Compound 1** (60 mg first dose/30 mg every 12 hours) n=62 | **Low-dose Compound 1** (20 mg first dose/10 mg every 12 hours) n=33 | **Hydrocodone bitartrate /acetaminophen reference arm** (5 mg/325 mg every six hours) n=60 |
|---|---|---|---|---|---|
| Mean SPID48 | 101.0 | 137.8 | 86.9 | 112.9 | 115.6 |
| MeanSPID48 difference from placebo | N/A | 36.8 | -14.1 | 11.9 | 14.7 |
| p-value vs. placebo | N/A | p = 0.0251 | p = 0.3859 | p = 0.5379 | p = 0.3706 |

The **bunionectomy** study met its primary endpoint, showing a statistically significant improvement in SPID48, as recorded on a Numeric Pain Rating Scale (NPRS), for those treated with Compound 1 at the high dose compared to placebo. Higher SPID48 values represent greater improvements in pain relief. The onset of action was rapid and was sustained through the duration of assessment.

Safety Results

Safety **evaluations** include adverse events, clinical laboratory assessments, clinical evaluation of vital signs, electrocardiograms, and physical examinations. No patients discontinued treatment due to AEs and no patients had SAEs. All AEs were mild or moderate. The most common AEs (incidence >10% in either placebo, HB/APAP or Compound 1 high-dose group, respectively) were headache (12%, 7%, 8%) and nausea (9%, 18%, 8%).

Compound 1 was also studied in patients undergoing abdominoplasty and demonstrated statistically significant improvement in SPID48 as well (NCT05034952).

Example 5: Prophetic

A Study of the Efficacy and Safety of Compound **1** in Subjects with painful diabetic peripheral neuropathy

A randomized, double-blind, active-controlled, dose-ranging, 4-arm, parallel-design study to evaluate the safety and efficacy of Compound **1** in treating subjects with painful diabetic peripheral neuropathy is conducted. A randomized, double-blind study design is selected to avoid observer bias and reduce symptoms or outcomes arising from the subjects' knowledge of treatment. A pregabalin reference arm assessing a standard-of-care treatment (100 mg tid) is included to establish the ability of the study to successfully observe a treatment effect for Compound **1.**

Study Subjects

Subjects who meet eligibility criteria during Screening Visits 1 and 2 enter a 7 day Run-in Period to establish their

baseline Numeric Pain Rating Scale (NPRS) pain score. Male and female patients between the ages of 18 and 75 years (inclusive) with pain that is ≥ 4 on an 11-point NPRS are included in the study. A total of approximately 150 subjects are randomized 2:1:1:2 to 4 treatment arms: Compound 1 (high, mid, or low dose) or pregabalin (reference arm) (Table 3). Randomization is stratified by sex (female and male) and body mass index (≥30 and <30 kg/m$^2$). To maintain the blind, all subjects receive the same number of pharmaceutical composition once daily (qd) in the morning and the same dose form 3 times per day in a double dummy design. After the Treatment Period, subjects taper off capsule (pregabalin reference or matched placebo) study drug for 7 days (4 days of dosing every 12 hours, then 3 days of dosing qd), and the safety follow up visit occurs an additional 7 (± 2) days later.

**Table 12 Treatment Arms**

| Treatment | Active Dose | Number of Subjects (Planned) |
|---|---|---|
| Compound **1** (high dose) | 69 mg qd | 50 |
| Compound **1** (mid dose) | 46 mg qd | 25 |
| Compound **1** (low dose) | 23 mg qd | 25 |
| Pregabalin | 100 mg tid | 50 |

qd: once daily; tid: 3 times per day
Note: To maintain the blind, all subjects receive the same number of tablets and the same number of capsules at the same respective frequency (i.e., qd for tablets and tid for capsules during the Treatment Period) in a double-dummy design.

Study Drugs

Investigational Drug: Compound **1.** The investigational drug is administered orally in a tablet containing 23 mg of active ingredient. The investigational drug is administered once daily. A low, mid, and high dosing regime are tested. The low dose is 23 mg, mid dose is 46 mg, and the high dose is 69 mg.

Reference Drug: Pregabalin. The reference drug is administered orally in a 100 mg capsule tid. The doses and dose frequency are summarized in Table 4 below.

**Table 13 Study Drug**

| Drug Name | Dosing Form/ Route | Dosage | How Supplied |
|---|---|---|---|
| Compound **1** | Tablet/oral | 23, 46, or 69 mg qd | Supplied as 23-mg tablet |
| Placebo | Tablet/oral | 0 mg qd | Supplied as tablets |
| Pregabalin | Capsule/oral | 100 mg tid | Supplied as 100-mg capsules |
| Pregabalin placebo | Capsule/oral | 0 mg tid | Supplied as capsules |

Compound **1** is administered once daily (qd) and pregabalin is administered three times daily.

In the Placebo Arm, Compound **1** placebo capsules are administered once daily.

After the Treatment Period, subjects taper off capsule (pregabalin reference or matched placebo) study drug for 7 days; tablet (Compound **1** or matched placebo) study drug is not provided during the taper. Subjects have a safety follow up visit 14 (± 2) days after the end of the treatment period.

Efficacy Assessments

11-point (0 to 10) Numeric Pain Rating Scale: NPRS scores are recognized by the FDA as a valid pain intensity measure. On the 11-point NPRS, a score of 0 denotes no pain, and a score of 10 denotes the worst pain intensity imaginable. Subjects report their pain on the 11-point NPRS immediately before their first dose of the study drug (baseline NPRS score) and 0.5, 1, 1.5, 2, 3, 4, 5, 6, 8, 12, 16, 20, 24, 28, 32, 36, 40, 44, and 48 hours (±5 minutes) after the first dose of study drug.

From Day -7 through Week 12, subjects report their average daily pain during the last 24 hours on the NPRS via e-diary; during the treatment period, the NPRS is completed in the morning before the first daily dose. Subjects report their current pain score before each administration of rescue medication. The NPRS scores from the daily e-diary are used in the primary endpoint analysis, and the proportion of subjects with ≥30%, ≥50%, and ≥70% reduction in weekly average scores as reported in the daily e-diary are used in the secondary endpoint analyses.

Daily Sleep Interference Scale (DSIS): Pain frequently interferes with sleep, and sleep is important to quality of life. The FDA recommends evaluating the effect of analgesics on sleep. DSIS is commonly used in neuropathic pain studies and is assessed on an 11-point NPRS and is thus evaluated in this study. The 11 point scale ranges from 0 (none) to 10 (severe).

Patient Global Impression of Change (PGIC): The PGIC is commonly used in neuropathic pain studies, and the Initiative on Methods, Measurement, and Pain Assessment in Clinical Trials (IMMPACT) group recommends it as a core outcome measure for chronic pain studies and is thus evaluated in this study. There is some evidence that PGIC may be more sensitive in neuropathic pain studies than pain intensity assessments because it may assess additional quality of life measures. The assessment consists of a single item on a 7-point scale from 1 (very much improved) to 7 (very much worse).

Neuropathic Pain Symptom Inventory (NPSI): The NPSI is a well-characterized method for quantifying distinct symptoms experienced by a diversity of neuropathic pain patients as well as treatment effects and is thus evaluated in this study. In addition, it has been used to study the underlying basis for individual symptoms qualities.

Short-form McGill Pain Questionnaire-2 (SF-MPQ-2): The SF-MPQ-2 is designed to provide an overall assessment of pain-related symptoms by including a range of neuropathic and non neuropathic pain descriptors and is thus evaluated in this study. It assesses 15 specific sensory and affective pain descriptors and provides a total score and sensory and affective subscale scores.

Columbia Suicide Severity Rating Scale (C-SSRS): The FDA recommends the evaluation of suicidality in clinical studies and is thus evaluated in this study. The C-SSRS evaluates this through a series of questions about suicidal thoughts and behaviors.

SF-36: The SF-36 is included because it is a commonly used health status questionnaire measuring 8 domains: physical functioning, role limitations due to physical problems, social functioning, bodily pain, mental health, role limitations due to emotional problems, vitality, and general health perception. Physical and mental component summary scores can be calculated.

BDI: The BDI is recommended as a core outcome measure of emotional functioning in chronic pain clinical trials and is thus evaluated in this study. It has well-established reliability and validity and can address less severe depression scores after treatment.

Bedside Sensory Testing Kit (BSTK): The BSTK is used for the purpose of standardized assessment and phenotyping of subjects' pain, using the usual components of a neurosensory examination and is thus evaluated in this study.

<u>Efficacy Results</u>

**Baseline NPRS Scores.** The baseline NPRS scores for the subjects in the Placebo Arm, Investigational Arm, and Reference Arm are reported. The NPRS scores reflect the mean (and standard deviation) of the NPRS scores of the subjects in each arm.

<u>Safety Results</u>

Safety evaluations include adverse events, clinical laboratory assessments, clinical evaluation of vital signs, electrocardiograms, and physical examinations.

<u>Example 6: Prophetic</u>

<u>A Study of the pharmacokinetics of Compound 1 in subjects with painful diabetic peripheral neuropathy</u>

A population PK analysis of plasma concentration versus time data of Compound 1 and Compound **1a** (Compound **1** metabolite) is performed using the nonlinear mixed-effects modeling approach. A population approach may also be used to investigate the exposure-response relationship for the efficacy and safety variables. At clinic visits during the study described in Example 4, PK samples are collected.

All efforts are made to obtain the applicable PK samples at the exact nominal time relative to dosing of the first daily dose. Acceptable windows for sampling times are shown in Table 14. Samples collected outside of these acceptable windows are considered protocol deviations.

**Table 14. Acceptable Pharmacokinetic Sampling Windows**

| Sampling Time | Time From Scheduled Sampling Allowed |
|---|---|
| Predose (before first daily dose) | Within -30 minutes |

(continued)

| Sampling Time | Time From Scheduled Sampling Allowed |
|---|---|
| From ≥2 to <6 hours after the first daily dose | ± 15 minutes |
| From ≥6 hours after the first daily dose | ± 30 minutes |

Example 7: Prophetic

Study of the Efficacy and Safety of Compound **1** in subjects with painful diabetic peripheral neuropathy

A randomized, double-blind, active-controlled, dose-ranging, 4-arm, parallel-design study to evaluate the safety and efficacy of Compound **1** in treating subjects with painful diabetic peripheral neuropathy is conducted. A randomized, double-blind study design is selected to avoid observer bias, reduce the possibility for unblinding, and reduce symptoms or outcomes arising from the subjects' knowledge of treatment. A pregabalin reference arm assessing a standard-of-care treatment (100 mg tid) is included to establish the ability of the study to successfully observe a treatment effect for Compound 1.

Study Subjects

Subjects who meet eligibility criteria during Screening Visits 1 and 2 enter a 7 day Run-in Period to establish their baseline Numeric Pain Rating Scale (NPRS) pain score and Daily Sleep Interference Scale (DSIS) score. In extenuating circumstances, the Day 1 window may be extended by up to 3 days (i.e., there may be up to a 3-day gap between ending the Run-In Period and starting the Treatment Period). Male and female patients between the ages of 18 and 80 years (inclusive) with pain that is ≥ 4 on an 11-point NPRS are included in the study, provided that they have had the presence of bilateral pain in the lower extremities due to diabetic peripheral neuropathy for at least one year, and weekly average NPRS pain score of ≥ 4 on an 11-point NPRS with limited variation in the 7-day Run-In Period (SD < 25% of mean). Optionally, to avoid confounding medical history, sujects receiving hormone replacement therapy may be excluded. A total of approximately 175 subjects are randomized 2:2:1:2 to 4 treatment arms: Compound 1 (high, mid, or low dose) or pregabalin (reference arm) (Table 6). Randomization is stratified by sex (female and male) and body mass index (with one group having a body mass index less than a given cutoff and the other having a body mass index greater than or equal to that cutoff, with the cutoff likely falling between 30 and 35 kg/m$^2$, inclusive). To maintain the blind, all subjects will receive the same number of tablets once daily (qd) in the morning and the same number of capsules 3 times per day (tid) in a double-dummy design. After the Treatment Period, subjects taper off capsule (pregabalin reference or matched placebo) study drug for 7 days (4 days of dosing every 12 hours, then 3 days of dosing qd), and the safety follow up visit occurs an additional 7 (± 2) days later.

**Table 15 Treatment Arms**

| Treatment | Active Dose | Number of Subjects (Planned) |
|---|---|---|
| Compound **1** (high dose) | 69 mg qd | 50 |
| Compound **1** (mid dose) | 46 mg qd | 25 |
| Compound **1** (low dose) | 23 mg qd | 25 |
| Pregabalin | 100 mg tid | 50 |

qd: once daily; tid: 3 times per day
Note: To maintain the blind, all subjects receive the same number of tablets and the same number of capsules at the same respective frequency (i.e., qd for tablets and tid for capsules during the Treatment Period) in a double-dummy design.

Study Drugs

Investigational Drug: Compound 1. The investigational drug is administered orally in a tablet containing 23 mg of active ingredient. The investigational drug is administered once daily. A low, mid, and high dosing regime are tested, as described in Table 6.

Reference Drug: Pregabalin. Pregabalin is an anticonvulsant approved for the treatment of painful diabetic neuropathy; it is considered first line treatment in most international clinical guidelines and forms a key part of management of neuropathic pain. The reference drug is administered orally in a 100 mg capsule tid. The doses and dose frequency are

summarized in Table 7 below.

**Table 16 Study Drug**

| Drug Name | Dosing Form/ Route | Dosage | How Supplied |
|---|---|---|---|
| Compound 1 | Tablet/oral | 23, 46, or 69 mg qd | Supplied as 23-mg tablet |
| Placebo | Tablet/oral | 0 mg qd | Supplied as tablets |
| Pregabalin | Capsule/oral | 100 mg tid | Supplied as 100-mg capsules |
| Pregabalin placebo | Capsule/oral | 0 mg tid | Supplied as capsules |

Compound **1** is administered once daily (qd) and pregabalin is administered three times daily.

In the Placebo Arm, Compound **1** placebo capsules are administered once daily.

After the Treatment Period, subjects taper off capsule (pregabalin reference or matched placebo) study drug for 7 days; tablet (Compound **1** or matched placebo) study drug is not provided during the taper. Subjects have a safety follow up visit 14 (± 2) days after the end of the treatment period.

Efficacy and Safety Assessments

11-point (0 to 10) Numeric Pain Rating Scale: NPRS scores are recognized by the FDA as a valid pain intensity measure and are used as a standard pain assessment scale in many pain registration studies. On the 11-point NPRS, a score of 0 denotes no pain, and a score of 10 denotes the worst pain intensity imaginable. Subjects report their pain on the 11-point NPRS immediately before their first dose of the study drug (baseline NPRS score) and 0.5, 1, 1.5, 2, 3, 4, 5, 6, 8, 12, 16, 20, 24, 28, 32, 36, 40, 44, and 48 hours (±5 minutes) after the first dose of study drug.

From Day -7 through Week 12, subjects report their average daily pain during the last 24 hours on the NPRS via e-diary; during the treatment period, the NPRS is completed in the morning before the first daily dose. Subjects report their current pain score before each administration of rescue medication. The NPRS scores from the daily e-diary, especially the change from baseline in the weekly average at Week 12, are used in the primary endpoint analysis, and the proportion of subjects with ≥30%, ≥50%, and ≥70% reduction in weekly average scores, especially at Week 12, as reported in the daily e-diary are used in the secondary endpoint analyses. For the primary endpoint, daily NPRS scores are averaged over a weekly period to reduce the impact of individual high or low pain scores, and the change from baseline in this weekly average may be used as an additional endpoint.

Daily Sleep Interference Scale (DSIS): Pain frequently interferes with sleep, and sleep is important to quality of life. The FDA recommends evaluating the effect of analgesics on sleep. DSIS is commonly used in neuropathic pain studies and is assessed on an 11-point NPRS and is thus evaluated in this study. The 11 point scale ranges from 0 (none) to 10 (severe). The DSIS is completed each morning before the first daily dose in an e-diary to describe how pain interfered with the subject's sleep. The change from baseline in the weekly average of DSIS, especially at Week 12, may be used in the secondary endpoint analysis.

Patient Global Impression of Change (PGIC): The PGIC is commonly used in neuropathic pain studies, and the Initiative on Methods, Measurement, and Pain Assessment in Clinical Trials (IMMPACT) group recommends it as a core outcome measure for chronic pain studies and is thus evaluated in this study. There is some evidence that PGIC may be more sensitive in neuropathic pain studies than pain intensity assessments because it may assess additional quality of life measures. The assessment consists of a single item on a 7-point scale from 1 (very much improved) to 7 (very much worse). The PGIC is completed at selected study visits to quantify the change in subjects' overall status. The proportion of subjects categorized as improved on the PGIC assessment, especially at Week 12, may be used in the secondary endpoint analysis.

Neuropathic Pain Symptom Inventory (NPSI): The NPSI is a well-characterized method for quantifying distinct symptoms experienced by a diversity of neuropathic pain patients as well as treatment effects and is thus evaluated in this study. In addition, it has been used to study the underlying basis for individual symptoms qualities. The NPSI is completed at selected study visits to quantify the change in subjects' distinct pain symptoms. The change from baseline in the total intensity score on the NPSI, especially at Week 12, may be used as an endpoint.

Short-form McGill Pain Questionnaire-2 (SF-MPQ-2): The SF-MPQ-2 is designed to provide an overall assessment of pain-related symptoms by including a range of neuropathic and non neuropathic pain descriptors and is thus evaluated in this study. It assesses 15 specific sensory and affective pain descriptors and provides a total score and sensory and affective subscale scores. The SF-MPQ-2 is completed at selected site visits to quantify the change in subjects' overall and specific pain symptoms. The change from baseline in pain characteristics and intensity using the SF-MPQ-2, especially at Week 2, may be used as an endpoint.

SF-36: The SF-36 is included because it is a commonly used health status questionnaire measuring 8 domains: physical functioning, role limitations due to physical problems, social functioning, bodily pain, mental health, role limitations due to emotional problems, vitality, and general health perception. Physical and mental component summary scores can be calculated. The change from baseline in the SF-36, especially at Week 12, may be used as an endpoint.

BDI: The BDI is recommended as a core outcome measure of emotional functioning in chronic pain clinical trials and is thus evaluated in this study. It has well-established reliability and validity and can address less severe depression scores after treatment. The change from baseline in the BDI, especially at Week 12, may be used as an endpoint.

Bedside Sensory Testing Kit (BSTK): The BSTK is used for the purpose of standardized assessment and phenotyping of subjects' pain, using the usual components of a neurosensory examination and is thus evaluated in this study.

Proportion of subjects using Rescue Medication and Total Medication Usage: Acetaminophen is permitted as a pain rescue medication as needed (prn) throughout the study. In an e-diary, subjects record rescue medication use and their current pain score on the NPRS immediately before each administration of rescue medication. Data on the use of rescue medications is collected for descriptive analysis across the 4 treatment groups, and the proportion of subjects using the rescue medication and the total medication usage may be used as an endpoint.

Pharmacokinetic Analysis

Blood samples will be collected from subjects who were administered any dose level of Compound **1** at various time points to evaluate the plasma concentrations of Compound 1 and its metabolite, Compound **1a.**

Efficacy Results

**Baseline Scores.** The baseline NPRS scores for the subjects in the Placebo Arm, Investigational Arm, and Reference Arm are reported. The NPRS scores reflect the mean (and standard deviation) of the NPRS scores of the subjects in each arm. Baseline values for the NPRS daily pain intensity scores and DSIS are defined as the average score from Day -7 to Day -1. For ECGs, the baseline value is defined as the average of the non-mising pretreatment measurements (triplicate) on Day 1. For all other variales, baseline values are defined as the most recent non-missing measurement collected before the first dose of study drug. Change (absolute change) from baseline is calculated as post-baeline value - baseline value.

**Statistical Analysis.** The primary efficacy endpoint is the change from baseline in the weekly average of the daily pain intensity on an NPRS at Week 12. The primary analysis will be a within-group comparison in any Compound 1 dose group. The primary efficacy analysis will be based on a mixed-effects model for repeated measures (MMRM), with change from baseline in weekly average of daily pain intensity score as the dependent variable; and fixed effects of treatment group, time (categorical), treatment group-by-time interaction, baseline weekly average of daily pain intensity, and baseline weekly average of daily pain intensity-by-time interaction. The least squares (LS) mean change from baseline at Week 12 for each group will be presented with the corresponding SE, and the corresponding 95% confidence interval and P value. The study is not powered for comparison between the Compound **1** doses and the pregabalin reference arm. An additional analysis of the primary endpoint wil be performed based on the same MMRM described above to estimate the mean difference between each of the Compound **1** doses and pregabalin. The LS mean differences in the change from baseline versus pregabalin at Week 12 and the corresponding 80% confidence interval will be presented. The proportion of subjects with ≥30% reduction in the weekly average of daily pain intensity on the NPRS at Week 12 will be summarized descriptively by treatment group and analyzed using the Cochran-Mantel-Hanzel test (stratified by sex and BMI). The proportion of subjects with ≥50% and ≥70% reduction will be analyzed similarly. The proportion of subjects categorized as improved at Week 12 on the PGIC assessment will be summarized descriptively by treatment group and analyzed using the Cochran-Mantel-Hanzel test (stratified by sex and BMI). The change from baseline in the weekly average of the DSIS at Week 12 will be analyzed similarly to the primary endpoint. The change from baseline in the NPRS pain score at each week will be analyzed using an MMRM similarly to the primary analysis. The changes from baseline in NPSI, SF-36, SF-MPQ-2, and BDI-2 at 12 weeks will be analyzed by an analysis of covariance (ANOVA) model. The use of rescue medication will be summarized descriptively with the percentage of subjects using rescue medication and the total medication usage. No statistical testing will be conducted on the safety endpoints.

Safety Results

Safety evaluations include adverse events, clinical laboratory assessments, clinical evaluation of vital signs, electrocardiograms, physical examinations, and C-SSRS. The safety and tolerability based on the incidence and type of adverse events and changes from baseline in clinically significant laboratory test results, vital signs, and ECGs at each visit may be used in the secondary endpoint analysis.

Example 8 SDD Tablet Process

Compound 1 spray-dried dispersion (SDD), microcrystalline cellulose, and croscarmellose sodium were weighed and passed through a sieve. The sieved materials were then added to a bin blender and blended to form an intragranular (IG) blend. Magnesium stearate was weighed and passed through a sieve. The sieved magnesium stearate was then added to the IG blend, and the resulting IG blend was dry granulated using a roller compactor and in-line mill to produce milled granules.

Microcrystalline cellulose and croscarmellose sodium (for the extragranular (EG) blend) were weighed and passed through a sieve. The sieved materials were added to a bin blender containing the milled granules and the resulting mixture was blended to form the EG blend. Magnesium stearate was weighed and passed through a sieve. The sieved magnesium stearate was then added to the EG blend, which was further blended. The resulting blend was compressed into tablets containing the equivalent of 50 mg of Compound 1 using a tablet press. The tablets were then film coated using Opadry blue (85F105173).

Tablets of the present disclosure, including tablets described in Tables 6, 7, and of Example 3, were made according to this method.

Example 9: Prophetic SDD Tablet Process

Compound 1 spray-dried dispersion (SDD), which may be prepared according to Example 3, microcrystalline cellulose, and croscarmellose sodium are weighed and passed through a sieve. The sieved materials are then added to a bin blender and are blended to form an intragranular (IG) blend. Magnesium stearate is weighed and passed through a sieve. The sieved magnesium stearate is then added to the IG blend, and the resulting IG blend is dry granulated using a roller compactor and in-line mill to produce milled granules.

Microcrystalline cellulose and croscarmellose sodium (for the extragranular (EG) blend) are weighed and passed through a sieve. The sieved materials are added to a bin blender containing the milled granules and the resulting mixture is blended to form the EG blend. Magnesium stearate is weighed and passed through a sieve. The sieved magnesium stearate is then added to the EG blend, which is further blended. The resulting blend is compressed into tablets containing the desired amount of Compound 1 using a tablet press. The tablets are then optionally film coated.

Tablets of the present disclosure, including tablets described in Tables 6, 7, and 8 of Example 3, can be made according to this method.

Many modifications and variations of the embodiments described herein may be made, as is apparent to those skilled in the art. The specific embodiments described herein are offered by way of example only. The invention is as defined in the claims.

## Claims

1. Compound 1:

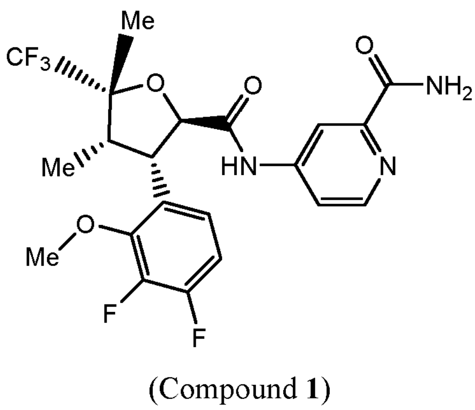

(Compound **1**)

or a pharmaceutically acceptable salt thereof, for use in a method of treating or lessening the severity of pain in a subject, comprising administering to the subject Compound 1 or a pharmaceutically acceptable salt thereof in an amount of 10 mg to 300 mg per day, optionally in an amount of 20 mg to 200 mg per day.

2. Compound 1 or a pharmaceutically acceptable salt thereof for use of claim 1, wherein Compound 1, or a pharmaceutically acceptable salt thereof, is administered in an amount of 10 mg to 300 mg on a first day, optionally in an amount of 20 mg to 200 mg on a first day, optionally in an amount of 20 mg to 30 mg on a first day, optionally in an amount of 60 mg to 90 mg on a first day, optionally in an amount of 100 mg to 150 mg on a first day, optionally in an amount of 5 mg to 200 mg per day after the first day.

**3.** Compound **1** or a pharmaceutically acceptable salt thereof for use of any one of claims 1 to 2, wherein Compound **1,** or a pharmaceutically acceptable salt thereof, is administered in two doses per day, or is administered in a first dose and a subsequent dose on the first day, wherein the first dose is larger than the subsequent dose, optionally wherein the subsequent dose is administered 12 hours after the first dose, optionally wherein the first dose is between 20 mg and 100 mg optionally the first dose is 20 mg or optionally wherein the first dose is 60 mg, or wherein the first dose is 100 mg.

**4.** Compound **1** or a pharmaceutically acceptable salt thereof for use of claim 3, wherein the subsequent dose is between 10 mg and 100 mg, or wherein the subsequent dose is between 10 mg and 50 mg, or wherein the subsequent dose is 10 mg, or wherein the subsequent dose is 30 mg, or wherein the subsequent dose is 50 mg.

**5.** Compound **1** or a pharmaceutically acceptable salt thereof for use of any one of claims 2 to 4, wherein Compound **1,** or a pharmaceutically acceptable salt thereof, is administered in two doses per day after the first day, or in two doses of 10 mg to 50 mg per day after the first day, or in two doses of 10 mg per day after the first day, or in two doses of 30 mg per day after the first day, or in two doses of 50 mg per day after the first day, optionally wherein a 12 hour period lapses between administration of each of the two doses.

**6.** Compound **1** or a pharmaceutically acceptable salt thereof for use of claim 1, wherein:

(i) Compound **1,** or a pharmaceutically acceptable salt thereof, is administered in a first dose of 100 mg and a subsequent dose of 50 mg administered 12 hours after the first dose, and a dose of 50 mg every 12 hours thereafter; or
(ii) Compound **1,** or a pharmaceutically acceptable salt thereof, is administered every 12 hours in a dose of 50 mg.

**7.** Compound **1** or a pharmaceutically acceptable salt thereof for use of claim 1, wherein Compound **1,** or a pharmaceutically acceptable salt thereof, is administered in one dose per day.

**8.** Compound **1** or a pharmaceutically acceptable salt thereof for use of claim 7, wherein Compound **1,** or a pharmaceutically acceptable salt thereof, is administered once per day in a dose of 70 mg.

**9.** Compound **1** or a pharmaceutically acceptable salt thereof for use of any one of claims 1 to 8, wherein:

(i) Compound **1,** or a pharmaceutically acceptable salt thereof, is administered for at least 1 week, or for at least 6 weeks, or for at least two days, and/or
(ii) the Compound **1,** or a pharmaceutically acceptable salt thereof, is administered orally or intravenously, and/or
(iii) (a) the pain comprises chronic pain, gut pain, neuropathic pain optionally post-herpetic neuralgia, small-fiber neuropathy, idiopathic small-fiber neuropathy, diabetic neuropathy, or diabetic peripheral neuropathy; musculoskeletal pain optionally osteoarthritis pain, acute pain optionally acute post-operative pain, inflammatory pain, cancer pain, idiopathic pain, postsurgical pain optionally bunionectomy pain, abdominoplasty pain, or herniorrhaphy pain, or visceral pain, optionally wherein the pain is associated with multiple sclerosis, Charcot-Marie-Tooth syndrome, incontinence, pathological cough, or cardiac arrhythmia, or (b) the Compound **1,** or a pharmaceutically acceptable salt thereof is for use in a method of treating or lessening the severity in a subject of trigeminal neuralgia, migraines treated with botox, cervical radiculopathy, occipital neuralgia, axillary neuropathy, radial neuropathy, ulnar neuropathy, brachial plexopathy, thoracic radiculopathy, intercostal neuralgia, lumbosacral radiculopathy, iliolingual neuralgia, pudendal neuralgia, femoral neuropathy, meralgia paresthetica, saphenous neuropathy, sciatic neuropathy, peroneal neuropathy, tibial neuropathy, lumbosacral plexopathy, traumatic neuroma stump pain or postamputation pain; and/or
(iv) the subject experienced a baseline pain score of at least 4 on an 11-point Numeric Pain Rating Scale prior to administration of Compound **1,** or a pharmaceutically acceptable salt thereof or the subject experienced a baseline pain level of moderate or severe on a Verbal Categorical Rating Scale prior to administration of Compound **1,** or a pharmaceutically acceptable salt thereof, and/or
(v) the method comprises administering to the subject Compound 1 in non-salt form, and/or
(vi) the subject is treated with one or more additional therapeutic agents administered concurrently with, prior to,

or subsequent to treatment with the compound, or pharmaceutically acceptable salt.

**10.** A solid dispersion comprising:

(2*R*,3*S*,4*S*,5*R*)-4-[[3-(3,4-difluoro-2-methoxy-phenyl)-4,5-dimethyl-5-(trifluoromethyl) tetrahydrofuran-2-carbonyl]amino]pyridine-2-carboxamide (Compound **1)** or a pharmaceutically acceptable salt thereof; and
at least one polymer.

**11.** The solid dispersion of claim 10, wherein

(i) Compound **1,** or a pharmaceutically acceptable salt thereof, and the polymer are co-spray-dried with a solvent, optionally wherein the polymer is selected from: hydroxypropyl methylcellulose acetate succinate (HPMCAS), polyvinyl caprolactam-polyvinyl acetate-polyethylene glycol graft co-polymer, and any combination thereof, optionally wherein the polymer is HPMCAS, and/or
(ii) the solid dispersion comprises:

20 wt % to 50 wt % of Compound **1,** or a pharmaceutically acceptable salt thereof; and 50 wt % to 80 wt % of the polymer,
optionally 25 wt % of Compound 1, or a pharmaceutically acceptable salt thereof; and
optionally 75 wt % of the polymer, and/or

(iii) Compound 1 is substantially amorphous.

**12.** A pharmaceutical composition comprising the solid dispersion of any one of claims 10 to 11, optionally wherein the pharmaceutical composition is a tablet.

**13.** The pharmaceutical composition of claim 12, wherein

(i) the pharmaceutical composition comprises 40 to 60 wt % of a spray-dried dispersion comprising Compound **1,** or a pharmaceutically acceptable salt thereof, 35 to 55 wt % of at least one filler, 1 to 6 wt % of at least one disintegrant, and 0.5 to 2 wt % of at least one lubricant, optionally wherein the filler is selected from: microcrystalline cellulose, lactose monohydrate, mannitol, and any combination thereof;

optionally wherein the disintegrant is selected from: croscarmellose sodium, crospovidone, and any combination thereof;
optionally wherein the lubricant is selected from: sodium stearyl fumarate, magnesium stearate, and any combination thereof;
optionally wherein the filler comprises microcrystalline cellulose and lactose monohydrate and the disintegrant comprises croscarmellose sodium, and wherein the lubricant comprises sodium stearyl fumarate, or

(ii) the pharmaceutical composition further comprises at least one filler, at least one lubricant, and at least one disintegrant.

**14.** The pharmaceutical composition of any one of claims 12 to 13, wherein the pharmaceutical composition comprises 1 to 50 mg, optionally 10 mg or 50 mg, of Compound 1, or a pharmaceutically acceptable salt thereof.

**15.** The pharmaceutical composition of claim 12, wherein the pharmaceutical composition comprises:

a) 47.5 to 52.5 wt % of a spray-dried dispersion of Compound 1;
b) 42.5 to 47.5 wt % of microcrystalline cellulose;
c) 2.5 to 3.5 wt % of croscarmellose sodium; and
d) 0.75 to 1.25 wt % of magnesium stearate.

**16.** The pharmaceutical composition of claim 15, wherein the pharmaceutical composition is a tablet comprising:

a) 200 mg of a spray-dried dispersion comprising 25 wt.% of Compound 1 and
75 wt.% of HPMCAS;
b) 184 mg of microcrystalline cellulose;

c) 12 mg of croscarmellose sodium; and
d) 4 mg of magnesium stearate.

**17.** Compound **1** or a pharmaceutically acceptable salt thereof for use of any one of claims 1 to 9, wherein administering to the subject Compound **1,** or a pharmaceutically acceptable salt thereof, comprises administering the pharmaceutical composition of any one of claims 12 to 16.

**18.** Compound **1** or a pharmaceutically acceptable salt thereof for use of claim 1, wherein Compound **1,** or a pharmaceutically acceptable salt thereof, is administered in an amount of 50 mg to 150 mg on a first day, or wherein Compound **1,** or a pharmaceutically acceptable salt thereof, is administered in an amount of 20 mg to 100 mg on a first day.

**Patentansprüche**

**1.** Verbindung 1:

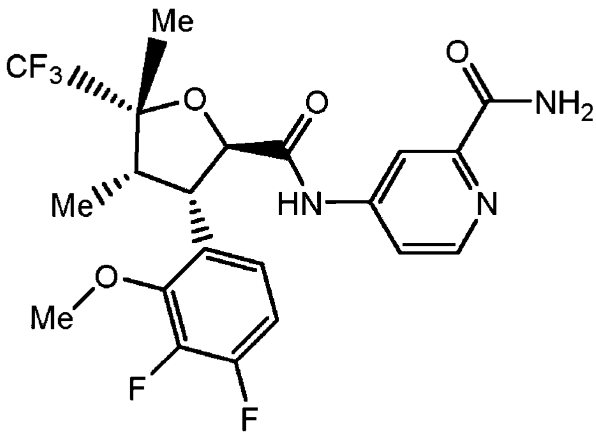

(Verbindung 1)

oder pharmazeutisch annehmbares Salz davon zur Verwendung in einem Verfahren zur Behandlung oder Linderung der Schmerzintensität bei einem Individuum, umfassend das Verabreichen der Verbindung 1 oder eines pharmazeutisch annehmbaren Salzes davon in einer Menge von 10 mg bis 300 mg pro Tag, gegebenenfalls in einer Menge von 20 mg bis 200 mg pro Tag an das Individuum.

**2.** Verbindung 1 oder ein pharmazeutisch annehmbares Salz zur Verwendung nach Anspruch 1, wobei Verbindung 1 oder ein pharmazeutisch annehmbares Salz davon in einer Menge von 10 mg bis 300 mg am ersten Tag, gegebenenfalls in einer Menge von 20 mg bis 200 mg am ersten Tag, gegebenenfalls in einer Menge von 20 mg bis 30 mg am ersten Tag, gegebenenfalls in einer Menge von 60 mg bis 90 mg am ersten Tag, gegebenenfalls in einer Menge von 100 mg bis 150 mg am ersten Tag, gegebenenfalls in einer Menge von 5 mg bis 200 mg pro Tag nach dem ersten Tag verabreicht wird.

**3.** Verbindung 1 oder ein pharmazeutisch annehmbares Salz davon zur Verwendung nach einem der vorhergehenden Ansprüche 1 bis 2, wobei Verbindung 1 oder ein pharmazeutisch annehmbares Salz davon in zwei Dosen pro Tag verabreicht wird, oder an einem ersten Tag in einer ersten Dosis und einer nachfolgenden Dosis verabreicht wird, wobei die erste Dosis größer ist als die nachfolgende Dosis, wobei gegebenenfalls die nachfolgende Dosis 12 Stunden nach der ersten Dosis verabreicht wird, wobei gegebenenfalls die erste Dosis zwischen 20 mg und 100 mg liegt, wobei gegebenenfalls die erste Dosis 20 mg beträgt oder wobei gegebenenfalls die erste Dosis 60 mg beträgt, oder wobei die erste Dosis 100 mg beträgt.

**4.** Verbindung 1 oder ein pharmazeutisch annehmbares Salz davon zur Verwendung nach Anspruch 3, wobei die nachfolgende Dosis zwischen 10 mg und 100 mg liegt, oder wobei die nachfolgende Dosis zwischen 10 mg und 50 mg liegt, oder wobei die nachfolgende Dosis 10 mg beträgt, oder wobei die nachfolgende Dosis 30 mg beträgt, oder wobei die nachfolgende Dosis 50 mg beträgt.

**5.** Verbindung 1 oder ein pharmazeutisch annehmbares Salz davon zur Verwendung nach einem der Ansprüche 2 bis 4, wobei Verbindung 1, oder ein pharmazeutisch annehmbares Salz davon in zwei Dosen pro Tag nach dem ersten Tag, oder in zwei Dosen von 10 mg bis 50 mg pro Tag nach dem ersten Tag, oder in zwei Dosen von 10 mg pro Tag nach dem ersten Tag, oder in zwei Dosen von 30 mg pro Tag nach dem ersten Tag, oder in zwei Dosen von 50 mg pro Tag nach dem ersten Tag verabreicht wird, wobei gegebenenfalls zwischen der Verabreichung jeder der beiden Dosen ein Zeitraum von 12 Stunden liegt.

**6.** Verbindung 1 oder ein pharmazeutisch annehmbares Salz davon zur Verwendung nach Anspruch 1, wobei

(i) Verbindung 1 oder ein pharmazeutisch annehmbares Salz davon in einer ersten Dosis von 100 mg und in einer nachfolgenden Dosis von 50 mg, die 12 Stunden nach der ersten Dosis verabreicht wird, und anschließend alle 12 Stunden in einer Dosis von 50 mg verabreicht wird; oder
(ii) Verbindung 1 oder ein pharmazeutisch annehmbares Salz davon alle 12 Stunden in einer Dosis von 50 mg verabreicht wird.

**7.** Verbindung 1 oder ein pharmazeutisch annehmbares Salz davon zur Verwendung nach Anspruch 1, wobei Verbindung 1 oder ein pharmazeutisch annehmbares Salz davon in einer Dosis pro Tag verabreicht wird.

**8.** Verbindung 1 oder ein pharmazeutisch annehmbares Salz davon zur Verwendung nach Anspruch 7, wobei Verbindung 1 oder ein pharmazeutisch annehmbares Salz davon einmal pro Tag in einer Dosis von 70 mg verabreicht wird.

**9.** Verbindung 1 oder ein pharmazeutisch annehmbares Salz davon zur Verwendung nach einem der Ansprüche 1 bis 8, wobei:

(i) Verbindung 1 oder ein pharmazeutisch annehmbares Salz davon für zumindest 1 Woche, oder für zumindest 6 Wochen, oder für zumindest zwei Tage verabreicht wird, und/oder
(ii) die Verbindung 1 oder ein pharmazeutisch annehmbares Salz davon oral oder intravenös verabreicht wird, und/oder
(iii) (a) wobei die Schmerzen chronische Schmerzen, Darmschmerzen, neuropathische Schmerzen (gegebenenfalls postherpetische Neuralgie, Small-Fiber-Neuropathie, idiopathische Small-Fiber-Neuropathie, diabetische Neuropathie oder diabetische periphere Neuropathie); muskuloskelettale Schmerzen (gegebenenfalls Schmerzen durch Osteoarthritis), akute Schmerzen (gegebenenfalls akute postoperative Schmerzen), entzündliche Schmerzen, Krebsschmerzen, idiopathische Schmerzen, postoperative Schmerzen (gegebenenfalls Schmerzen nach Bunionektomie, Bauchdeckenstraffung oder Herniorrhaphie) oder viszerale Schmerzen umfassen, wobei die Schmerzen gegebenenfalls mit Multipler Sklerose, Charcot-Marie-Tooth-Krankheit, Inkontinenz, pathologischem Husten oder Herzrhythmusstörungen verbunden sind, oder b) die Verbindung 1 oder ein pharmazeutisch annehmbares Salz davon zur Verwendung in einem Verfahren zur Behandlung oder Linderung der Schwere von Trigeminusneuralgie, mit Botox behandelter Migräne, zervikaler Radikulopathie, Okzipitalneuralgie, axillärer Neuropathie, radialer Neuropathie, Ulnarisneuropathie, Armplexusläsion, thorakaler Radikulopathie, Interkostalneuralgie, lumbosakraler Radikulopathie, Ilioinguinalis-Neuralgie, Pudendus-Neuralgie, femoraler Neuropathie, Meralgia paresthetica, Saphenus-Neuropathie, Ischias-Neuropathie, Peroneus-Neuropathie, Tibialis-Neuropathie, lumbosakraler Plexopathie, traumatischen Neuro-Stumpfschmerzen oder Postamputationsschmerzen bei einem Individuum ist; und/oder
(iv) das Individuum vor der Verabreichung von Verbindung 1 oder eines pharmazeutisch annehmbaren Salzes davon einen Ausgangsschmerzwert von zumindest 4 auf einer nummerischen Schmerz-Ratingskala mit 11 Punkten aufwies, oder das Individuum vor der Verabreichung von Verbindung 1 oder eines pharmazeutisch annehmbaren Salzes davon ein Ausgangsschmerzniveau von mäßig oder schwer auf einer verbalen kategorialen Ratingskala aufwies, und/oder
(v) das Verfahren das Verabreichen der Verbindung 1 in Nicht-Salzform an das Individuum umfasst, und/oder
(vi) das Individuum mit einem oder mehreren zusätzlichen therapeutischen Mitteln behandelt wird, das/die zeitgleich mit, vor, oder nach der Behandlung mit der Verbindung oder dem pharmazeutisch annehmbaren Salz davon verabreicht wird.

**10.** Feste Dispersion, umfassend:

(2*R*,3*S*,4*S*,5*R*)-4-[[3-(3,4-Difluor-2-methoxyphenyl)-4,5-dimethyl-5-(trifluormethyl)tetrahydrofuran-2-carbonyl]amino]pyridin-2-carboxamid (Verbindung 1) oder ein pharmazeutisch annehmbares Salz davon; und zumindest ein Polymer.

**11.** Feste Dispersion nach Anspruch 10, wobei

(i) Verbindung 1 oder ein pharmazeutisch annehmbares Salz davon und das Polymer gemeinsam mit einem Lösungsmittel sprühgetrocknet werden, wobei gegebenenfalls das Polymer ausgewählt ist aus: Hydroxypropylmethylcelluloseacetatsuccinat (HPMCAS), Polyvinylcaprolactam-Polyvinylacetat-Polyethylenglycol-Graft-Copolymer und beliebigen Kombinationen davon, wobei gegebenenfalls das Polymer HPMCAS ist, und/oder
(ii) die feste Dispersion Folgendes umfasst:

20 Gew.-% bis 50 Gew.-% von Verbindung 1 oder eines pharmazeutisch annehmbaren Salzes davon; und 50 Gew.-% bis 80 Gew.-% des Polymers, gegebenenfalls 25 Gew.-% von Verbindung 1 oder eines pharmazeutisch annehmbaren Salzes davon; und
gegebenenfalls 75 Gew.-% des Polymers, und/oder

(iii) Verbindung 1 im Wesentlichen amorph ist.

**12.** Pharmazeutische Zusammensetzung, umfassend die feste Dispersion nach einem der Ansprüche 10 bis 11, wobei es sich bei der pharmazeutischen Zusammensetzung gegebenenfalls um eine Tablette handelt.

**13.** Pharmazeutische Zusammensetzung nach Anspruch 12, wobei

(i) die pharmazeutische Zusammensetzung 40 bis 60 Gew.-% einer sprühgetrockneten Dispersion umfasst, die Verbindung 1 oder ein pharmazeutisch annehmbares Salz davon, 35 bis 55 Gew.-% von zumindest einem Füllstoff, 1 bis 6 Gew.-% von zumindest einem Sprengmittel, und 0,5 bis 2 Gew.-% von zumindest einem Schmiermittel umfasst, wobei der Füllstoff gegebenenfalls ausgewählt ist aus: mikrokristalliner Cellulose, Lactose-Monohydrat, Mannitol und einer beliebigen Kombination davon;

wobei gegebenenfalls das Sprengmittel ausgewählt ist aus: Croscarmellose-Natrium, Crospovidon und einer beliebigen Kombination davon;
wobei gegebenenfalls das Schmiermittel ausgewählt ist aus: Natriumstearylfumarat, Magnesiumstearat, und einer beliebigen Kombination davon;
wobei der Füllstoff gegebenenfalls mikrokristalline Cellulose und Lactose-Monohydrat umfasst und das Sprengmittel Croscarmellose-Natrium umfasst und wobei das Schmiermittel Natriumstearylfumarat umfasst, oder

(ii) die pharmazeutischen Zusammensetzung ferner zumindest einen Füllstoff, zumindest ein Schmiermittel und zumindest ein Sprengmittel umfasst.

**14.** Pharmazeutische Zusammensetzung nach einem der Ansprüche 12 bis 13, wobei die pharmazeutische Zusammensetzung 1 bis 50 mg, gegebenenfalls 10 mg oder 50 mg, Verbindung 1 oder ein pharmazeutisch annehmbares Salz davon umfasst.

**15.** Pharmazeutische Zusammensetzung nach Anspruch 12, wobei die pharmazeutische Zusammensetzung Folgendes umfasst:

a) 47,5 bis 52,5 Gew.-% einer sprühgetrockneten Dispersion von Verbindung 1;
b) 42,5 bis 47,5 Gew.-% mikrokristalliner Cellulose;
c) 2,5 bis 3,5 Gew.-% Croscarmellose-Natrium; und
d) 0,75 bis 1,25 Gew.-% Magnesiumstearat.

**16.** Pharmazeutische Zusammensetzung nach Anspruch 15, wobei es sich bei der pharmazeutischen Zusammensetzung um eine Tablette handelt, umfassend:

a) 200 mg einer sprühgetrockneten Dispersion, umfassend 25 Gew.-% Verbindung 1 und 75 Gew.- % HPMCAS;
b) 184 mg mikrokristalliner Cellulose;

c) 12 mg Croscarmellose-Natrium; und
d) 4 mg Magnesiumstearat.

**17.** Verbindung 1 oder ein pharmazeutisch annehmbares Salz davon zur Verwendung nach einem der Ansprüche 1 bis 9, wobei das Verabreichen von Verbindung 1 oder eines pharmazeutisch annehmbaren Salzes davon an das Individuum das Verabreichen der pharmazeutischen Zusammensetzung nach einem der Ansprüche 12 bis 16 umfasst.

**18.** Verbindung 1 oder ein pharmazeutisch annehmbares Salz davon zur Verwendung nach Anspruch 1, wobei Verbindung 1 oder ein pharmazeutisch annehmbares Salz davon in einer Menge von 50 mg bis 150 mg am ersten Tag verabreicht wird oder wobei Verbindung 1 oder ein pharmazeutisch annehmbares Salz davon in einer Menge von 20 mg bis 100 mg am ersten Tag verabreicht wird.

## Revendications

**1.** Composé 1 :

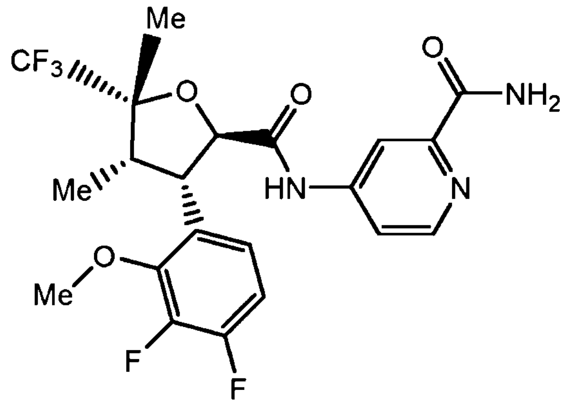

(Composé **1**)

ou sel pharmaceutiquement acceptable de celui-ci, destiné à être utilisé dans une méthode de traitement ou de diminution de la gravité de la douleur chez un sujet, comprenant l'administration au sujet de composé 1 ou d'un sel pharmaceutiquement acceptable de celui-ci en une quantité de 10 mg à 300 mg par jour, éventuellement en une quantité de 20 mg à 200 mg par jour.

**2.** Composé 1 ou sel pharmaceutiquement acceptable de celui-ci destiné à être utilisé selon la revendication 1, le composé 1, ou un sel pharmaceutiquement acceptable de celui-ci, étant administré en une quantité de 10 mg à 300 mg un premier jour, éventuellement en une quantité de 20 mg à 200 mg un premier jour, éventuellement en une quantité de 20 mg à 30 mg un premier jour, éventuellement en une quantité de 60 mg à 90 mg un premier jour, éventuellement en une quantité de 100 mg à 150 mg un premier jour, éventuellement en une quantité de 5 mg à 200 mg par jour après le premier jour.

**3.** Composé 1 ou sel pharmaceutiquement acceptable de celui-ci destiné à être utilisé selon l'une quelconque des revendications 1 à 2, le composé 1, ou un sel pharmaceutiquement acceptable de celui-ci, étant administré en deux doses par jour, ou étant administré en une première dose et une dose ultérieure le premier jour, la première dose étant plus grande que la dose ultérieure, éventuellement la dose ultérieure étant administrée 12 heures après la première dose, éventuellement la première dose étant comprise entre 20 mg et 100 mg, éventuellement la première dose étant de 20 mg ou éventuellement la première dose étant de 60 mg ou la première dose étant de 100 mg.

**4.** Composé 1 ou sel pharmaceutiquement acceptable de celui-ci destiné à être utilisé selon la revendication 3, la dose ultérieure étant comprise entre 10 mg et 100 mg ou la dose ultérieure étant comprise entre 10 mg et 50 mg ou la dose ultérieure étant de 10 mg ou la dose ultérieure étant de 30 mg ou la dose ultérieure étant de 50 mg.

**5.** Composé 1 ou sel pharmaceutiquement acceptable de celui-ci destiné à être utilisé selon l'une quelconque des revendications 2 à 4, le composé 1, ou un sel pharmaceutiquement acceptable de celui-ci, étant administré en deux

doses par jour après le premier jour ou en deux doses de 10 mg à 50 mg par jour après le premier jour ou en deux doses de 10 mg par jour après le premier jour ou en deux doses de 30 mg par jour après le premier jour ou en deux doses de 50 mg par jour après le premier jour, éventuellement une durée de 12 heures s'écoulant entre les administrations de chacune des deux doses.

**6.** Composé 1 ou sel pharmaceutiquement acceptable de celui-ci destiné à être utilisé selon la revendication 1 :

(i) le composé 1, ou un sel pharmaceutiquement acceptable de celui-ci, étant administré en une première dose de 100 mg et une dose ultérieure de 50 mg administrée 12 heures après la première dose et une dose de 50 mg toutes les 12 heures par la suite ; ou
(ii) le composé 1, ou un sel pharmaceutiquement acceptable de celui-ci, étant administré toutes les 12 heures en une dose de 50 mg.

**7.** Composé 1 ou sel pharmaceutiquement acceptable de celui-ci destiné à être utilisé selon la revendication 1, le composé 1, ou un sel pharmaceutiquement acceptable de celui-ci, étant administré en une dose par jour.

**8.** Composé 1 ou sel pharmaceutiquement acceptable de celui-ci destiné à être utilisé selon la revendication 7, le composé 1, ou un sel pharmaceutiquement acceptable de celui-ci, étant administré une fois par jour en une dose de 70 mg.

**9.** Composé 1 ou sel pharmaceutiquement acceptable de celui-ci destiné à être utilisé selon l'une quelconque des revendications 1 à 8 :

(i) le composé 1, ou un sel pharmaceutiquement acceptable de celui-ci, étant administré pendant au moins 1 semaine ou pendant au moins 6 semaines ou pendant au moins deux jours et/ou
(ii) le composé 1, ou un sel pharmaceutiquement acceptable de celui-ci, étant administré par voie orale ou par voie intraveineuse et/ou
(iii)

(a) la douleur comprenant une douleur chronique, une douleur intestinale, une douleur neuropathique éventuellement une névralgie post-herpétique, une neuropathie des petites fibres, une neuropathie des petites fibres idiopathique, une neuropathie diabétique ou une neuropathie diabétique périphérique ; une douleur musculosquelettique éventuellement une douleur liée à l'arthrose, une douleur aiguë éventuellement une douleur aiguë postopératoire, une douleur inflammatoire, une douleur liée à un cancer, une douleur idiopathique, une douleur postopératoire éventuellement une douleur liée à une bunionectomie, une douleur liée à une abdominoplastie ou une douleur liée à une herniorraphie, ou une douleur viscérale, éventuellement la douleur étant associée à la sclérose en plaques, au syndrome de Charcot-Marie-Tooth, à l'incontinence, à une toux pathologique ou à une arythmie cardiaque, ou
(b) le composé 1, ou un sel pharmaceutiquement acceptable de celui-ci, étant destiné à être utilisé dans une méthode de traitement ou de diminution de la gravité chez un sujet d'une névralgie du trijumeau, de migraines traitées avec du botox, d'une radiculopathie cervicale, d'une névralgie occipitale, d'une neuropathie axillaire, d'une neuropathie radiale, d'une neuropathie ulnaire, d'une plexopathie brachiale, d'une radiculopathie thoracique, d'une névralgie intercostale, d'une radiculopathie lombo-sacrée, d'une névralgie ilio-inguinale, d'une névralgie pudendale, d'une neuropathie fémorale, d'une méralgie paresthésique, d'une neuropathie saphène, d'une neuropathie sciatique, d'une neuropathie périnéale, d'une neuropathie tibiale, d'une plexopathie lombo-sacré, d'une douleur de névrome traumatique de moignon ou d'une douleur post-amputation ; et/ou

(iv) le sujet ressentant un score de douleur de base d'au moins 4 sur une échelle numérique d'évaluation de la douleur à 11 chiffres avant l'administration de composé 1, ou d'un sel pharmaceutiquement acceptable de celui-ci, ou le sujet ressentant un niveau de douleur de base modéré à intense sur une échelle d'évaluation verbale simple avant l'administration de composé 1, ou d'un sel pharmaceutiquement acceptable de celui-ci, et/ou
(v) la méthode comprenant l'administration au sujet de composé 1 sous une forme qui n'est pas un sel et/ou
(vi) le sujet étant traité avec un ou plusieurs agents thérapeutiques supplémentaires administrés en même temps que le traitement avec le composé ou le sel pharmaceutiquement acceptable, avant le traitement avec le composé ou le sel pharmaceutiquement acceptable ou après le traitement avec le composé ou le sel pharmaceutiquement acceptable.

**10.** Dispersion solide comprenant :

du (2*R*,3*S*,4*S*,5*R*)-4-[[3-(3,4-difluoro-2-méthoxyphényl)-4,5-diméthyl-5-(trifluorométhyl)tétrahydrofurane-2-carbonyl]amino]pyridine-2-carboxamide (composé 1) ou un sel pharmaceutiquement acceptable de celui-ci ; et au moins un polymère.

**11.** Dispersion solide selon la revendication 10,

(i) le composé 1, ou un sel pharmaceutiquement acceptable de celui-ci, et le polymère étant co-séchés par pulvérisation avec un solvant, éventuellement le polymère étant choisi parmi : l'acétate et succinate d'hydroxypropylméthylcellulose (HPMCAS), un copolymère greffé de polyvinylcaprolactame-poly(acétate de vinyle)-polyéthylèneglycol et une quelconque combinaison de ceux-ci, éventuellement le polymère étant l'HPMCAS, et/ou
(ii) la dispersion solide comprenant :

20 % en poids à 50 % en poids de composé 1, ou d'un sel pharmaceutiquement acceptable de celui-ci ; et 50 % en poids à 80 % en poids du polymère,
éventuellement 25 % en poids de composé 1, ou d'un sel pharmaceutiquement acceptable de celui-ci ; et éventuellement 75 % en poids du polymère, et/ou

(iii) le composé 1 étant pratiquement amorphe.

**12.** Composition pharmaceutique comprenant la dispersion solide selon l'une quelconque des revendications 10 à 11, éventuellement la composition pharmaceutique étant un comprimé.

**13.** Composition pharmaceutique selon la revendication 12,

(i) la composition pharmaceutique comprenant 40 à 60 % en poids d'une dispersion séchée par pulvérisation comprenant du composé 1, ou un sel pharmaceutiquement acceptable de celui-ci, 35 à 55 % en poids d'au moins une matière de charge, 1 à 6 % en poids d'au moins un délitant et 0,5 à 2 % en poids d'au moins un lubrifiant,

éventuellement la matière de charge étant choisie parmi : la cellulose microcristalline, le lactose monohydraté, le mannitol et une quelconque combinaison de ceux-ci ;
éventuellement le délitant étant choisi parmi : la croscarmellose sodique, la crospovidone et une quelconque combinaison de ceux-ci ;
éventuellement le lubrifiant étant choisi parmi : le stéarylfumarate de sodium, le stéarate de magnésium et une quelconque combinaison de ceux-ci ; éventuellement la matière de charge comprenant de la cellulose microcristalline et du lactose monohydraté et le délitant comprenant de la croscarmellose sodique et le lubrifiant comprenant du stéarylfumarate de sodium, ou

(ii) la composition pharmaceutique comprenant en outre au moins une matière de charge, au moins un lubrifiant et au moins un délitant.

**14.** Composition pharmaceutique selon l'une quelconque des revendications 12 à 13, la composition pharmaceutique comprenant 1 à 50 mg, éventuellement 10 mg ou 50 mg, de composé 1, ou d'un sel pharmaceutiquement acceptable de celui-ci.

**15.** Composition pharmaceutique selon la revendication 12, la composition pharmaceutique comprenant :

a) 47,5 à 52,5 % en poids d'une dispersion de composé 1 séchée par pulvérisation ;
b) 42,5 à 47,5 % en poids de cellulose microcristalline ;
c) 2,5 à 3,5 % en poids de croscarmellose sodique ; et
d) 0,75 à 1,25 % en poids de stéarate de magnésium.

**16.** Composition pharmaceutique selon la revendication 15, la composition pharmaceutique étant un comprimé comprenant :

a) 200 mg d'une dispersion séchée par pulvérisation comprenant 25 % en poids de composé 1 et 75 % en poids de HPMCAS ;

b) 184 mg de cellulose microcristalline ;
c) 12 mg de croscarmellose sodique ; et
d) 4 mg de stéarate de magnésium.

**17.** Composé 1 ou sel pharmaceutiquement acceptable de celui-ci destiné à être utilisé selon l'une quelconque des revendications 1 à 9, l'administration au sujet de composé 1, ou d'un sel pharmaceutiquement acceptable de celui-ci, comprenant l'administration de la composition pharmaceutique selon l'une quelconque des revendications 12 à 16.

**18.** Composé 1 ou sel pharmaceutiquement acceptable de celui-ci destiné à être utilisé selon la revendication 1, le composé 1, ou un sel pharmaceutiquement acceptable de celui-ci, étant administré en une quantité de 50 mg à 150 mg un premier jour ou le composé 1, ou un sel pharmaceutiquement acceptable de celui-ci, étant administré en une quantité de 20 mg à 100 mg un premier jour.

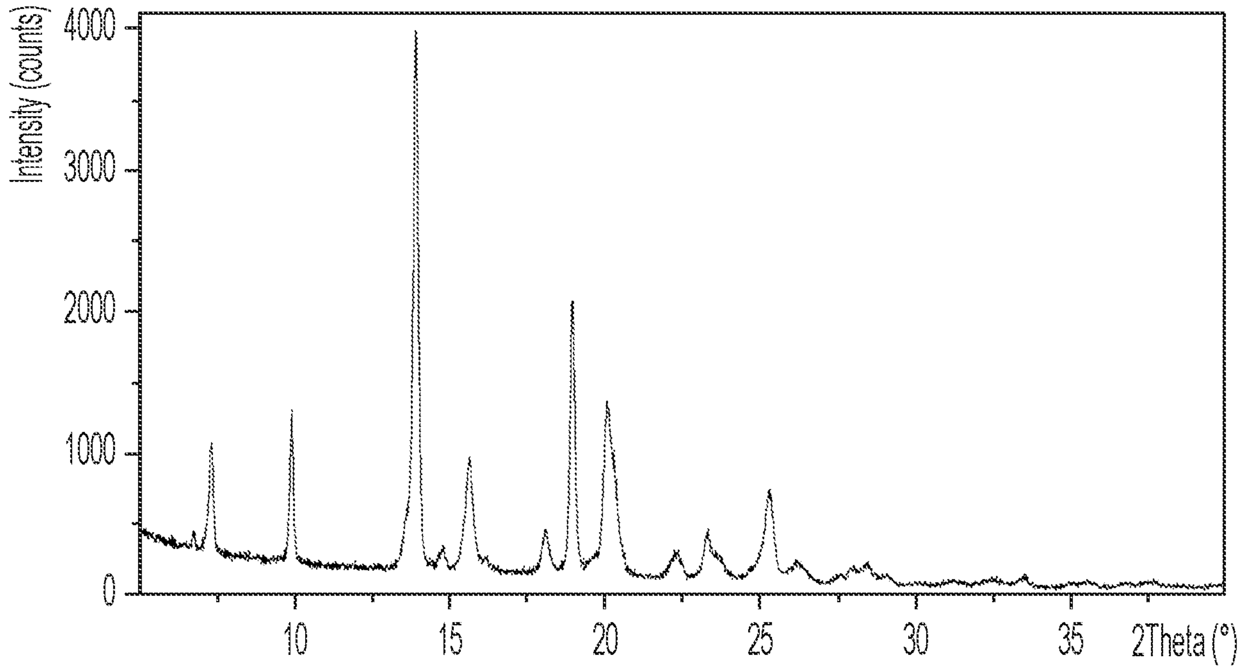
Intensity (counts)
4000
3000
2000
1000
0
10
15
20
25
30
35
2Theta (°)

FIG. 1

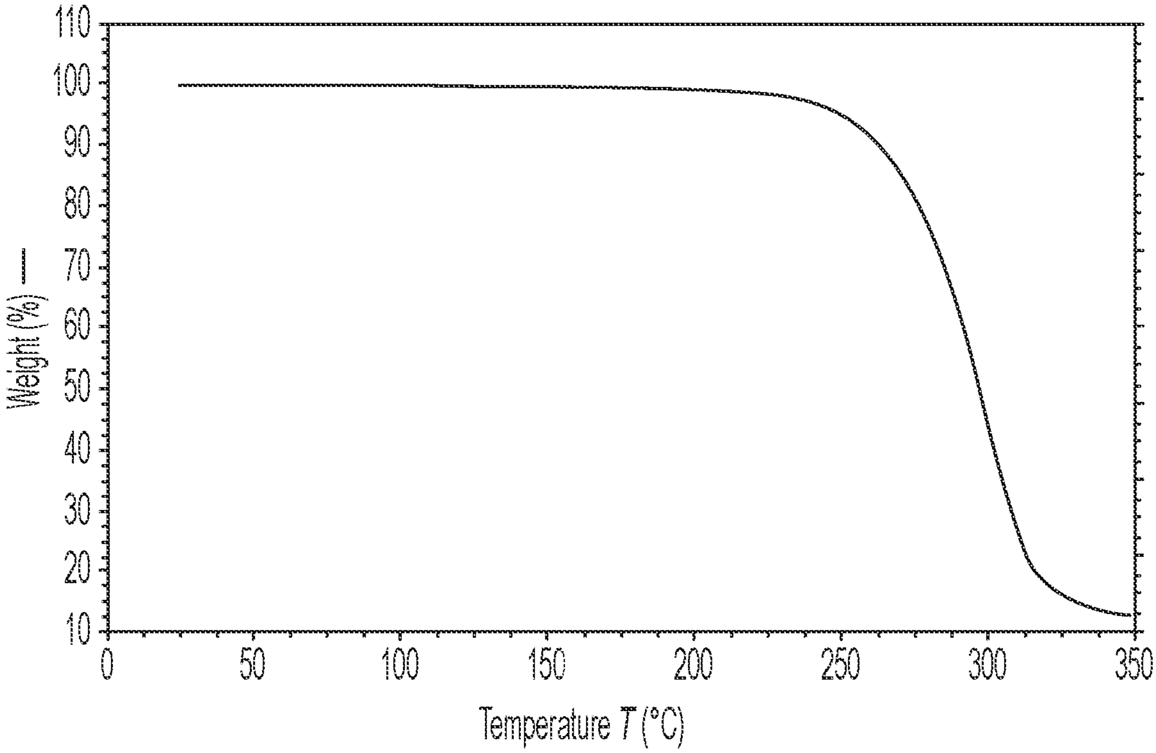
Weight (%)
110
100
90
80
70
60
50
40
30
20
10
0
50
100
150
200
250
300
350
Temperature T (°C)

FIG. 2

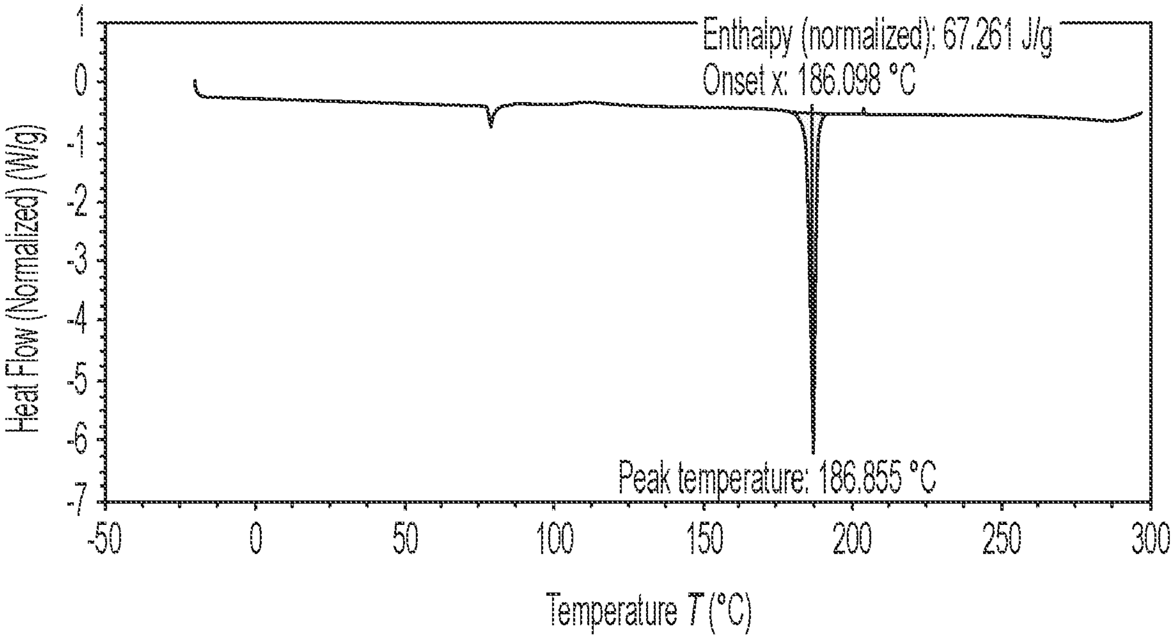
Enthalpy (normalized): 67.261 J/g
Onset x: 186.098 °C
Peak temperature: 186.855 °C
Heat Flow (Normalized) (W/g)
Temperature T (°C)
1
0
-1
-2
-3
-4
-5
-6
-7
-50
0
50
100
150
200
250
300

FIG. 3

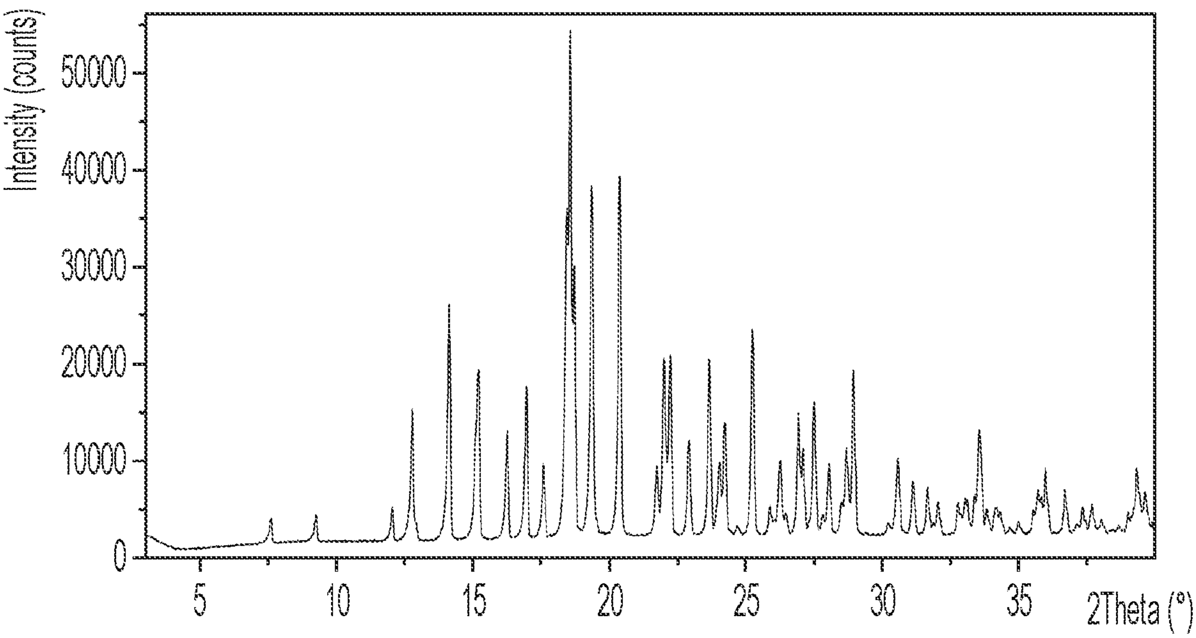
Intensity (counts)
50000
40000
30000
20000
10000
0
5
10
15
20
25
30
35
2Theta (°)

FIG. 4

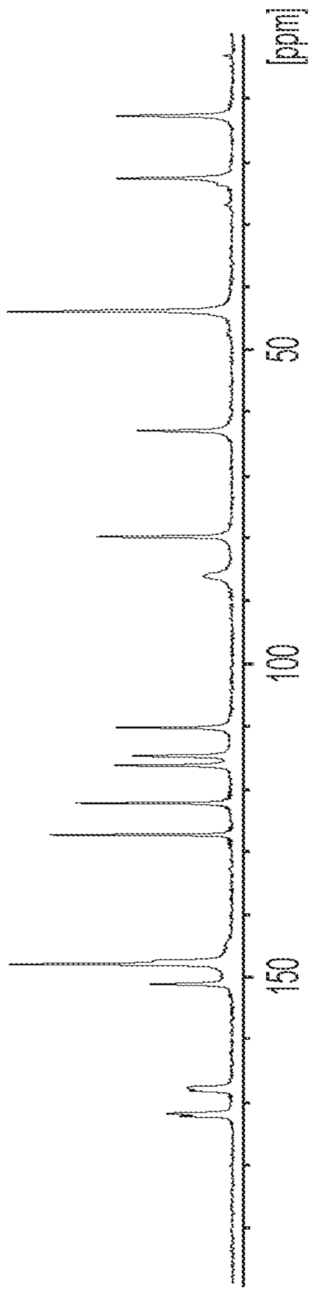


FIG. 5

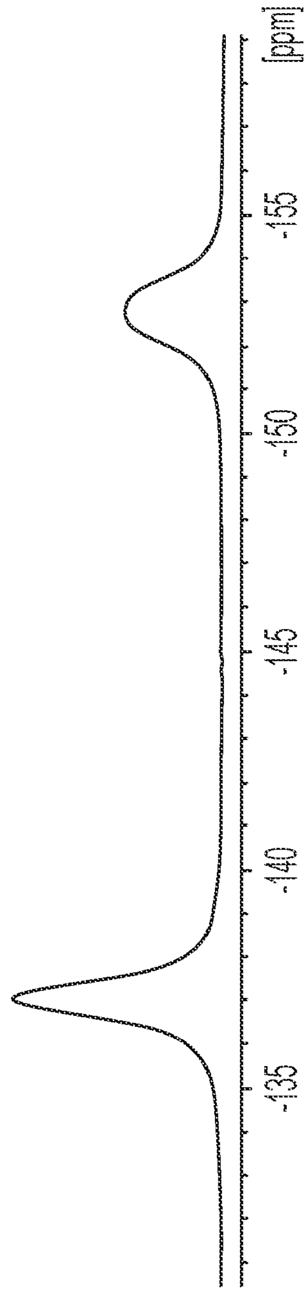


FIG. 6

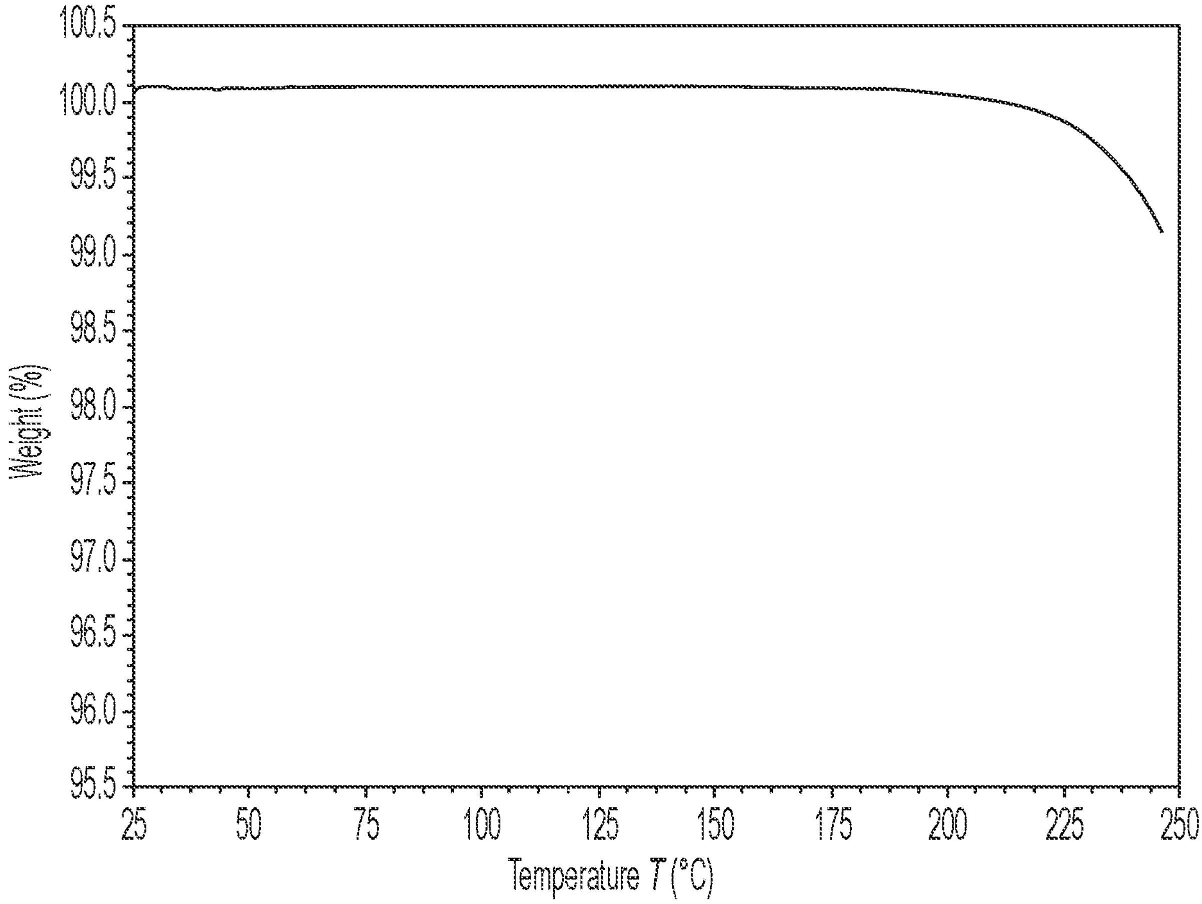
100.5
100.0
99.5
99.0
98.5
98.0
97.5
97.0
96.5
96.0
95.5
Weight (%)
25
50
75
100
125
150
175
200
225
250
Temperature T (°C)

FIG. 7

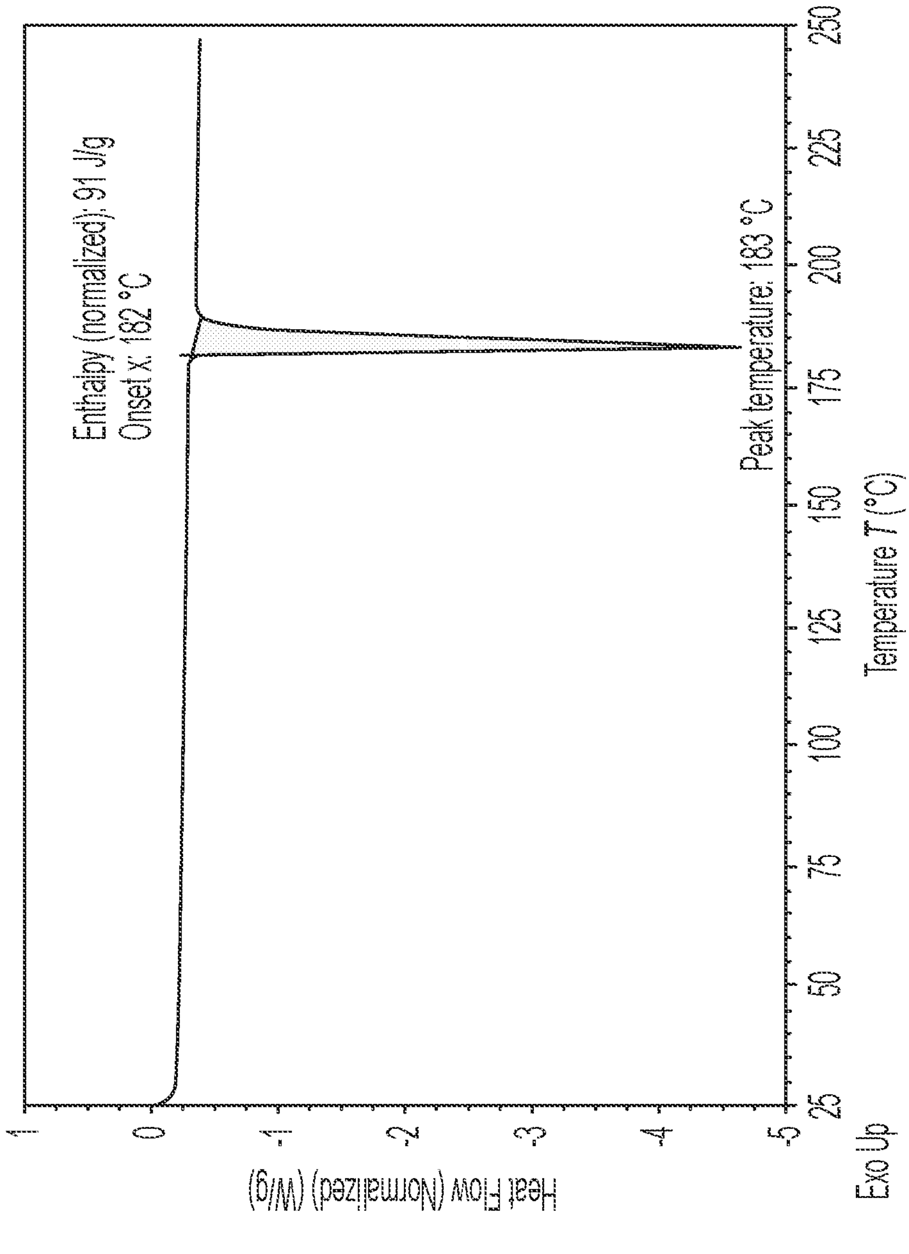
Enthalpy (normalized): 91 J/g
Onset x: 182 °C
Peak temperature: 183 °C
Heat Flow (Normalized) (W/g)
Exo Up
Temperature T (°C)
1
0
-1
-2
-3
-4
-5
25
50
75
100
125
150
175
200
225
250

FIG. 8

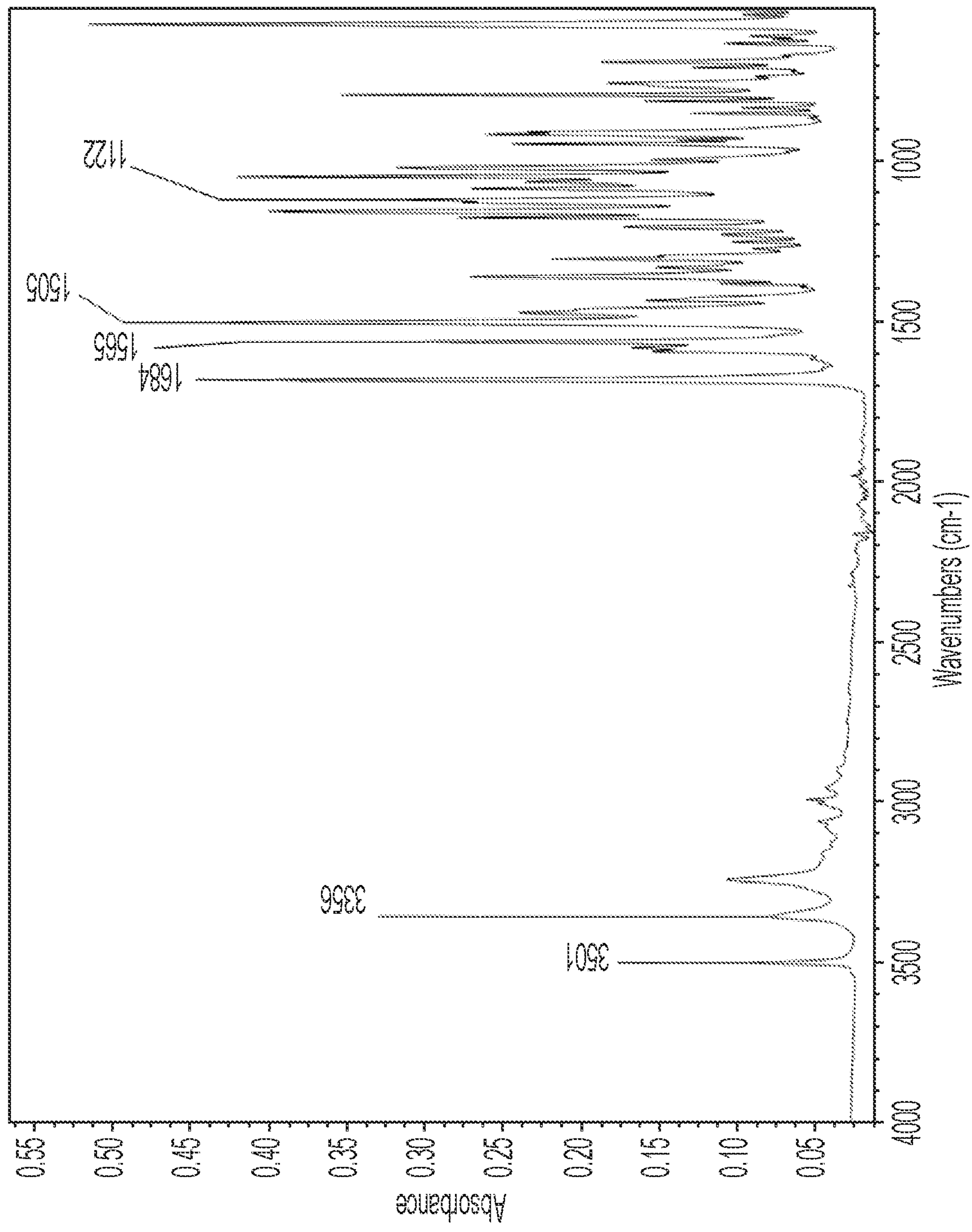
Absorbance
0.55
0.50
0.45
0.40
0.35
0.30
0.25
0.20
0.15
0.10
0.05
Wavenumbers (cm-1)
4000
3500
3000
2500
2000
1500
1000
3501
3356
1684
1565
1505
1122

FIG. 9

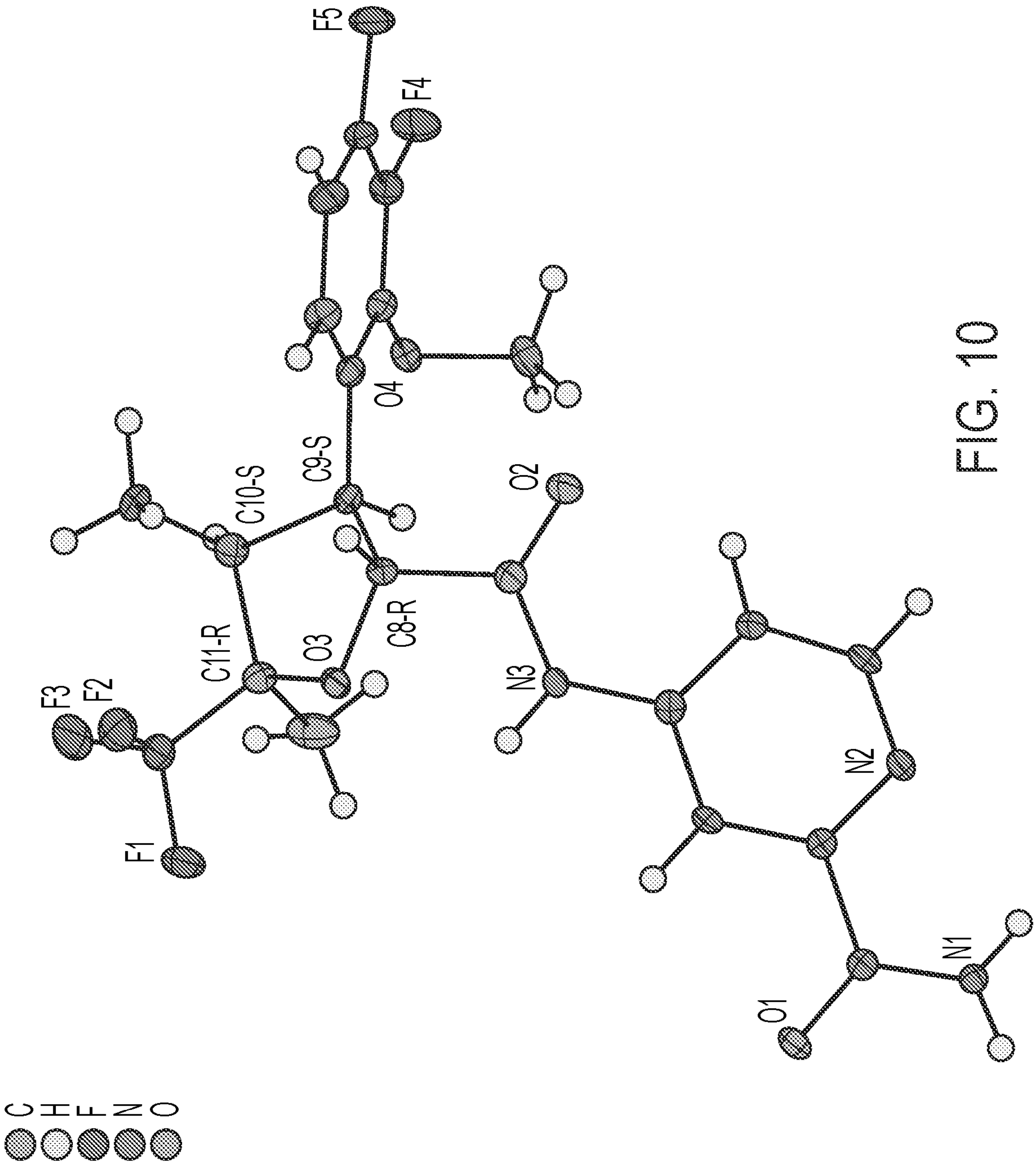

FIG. 10

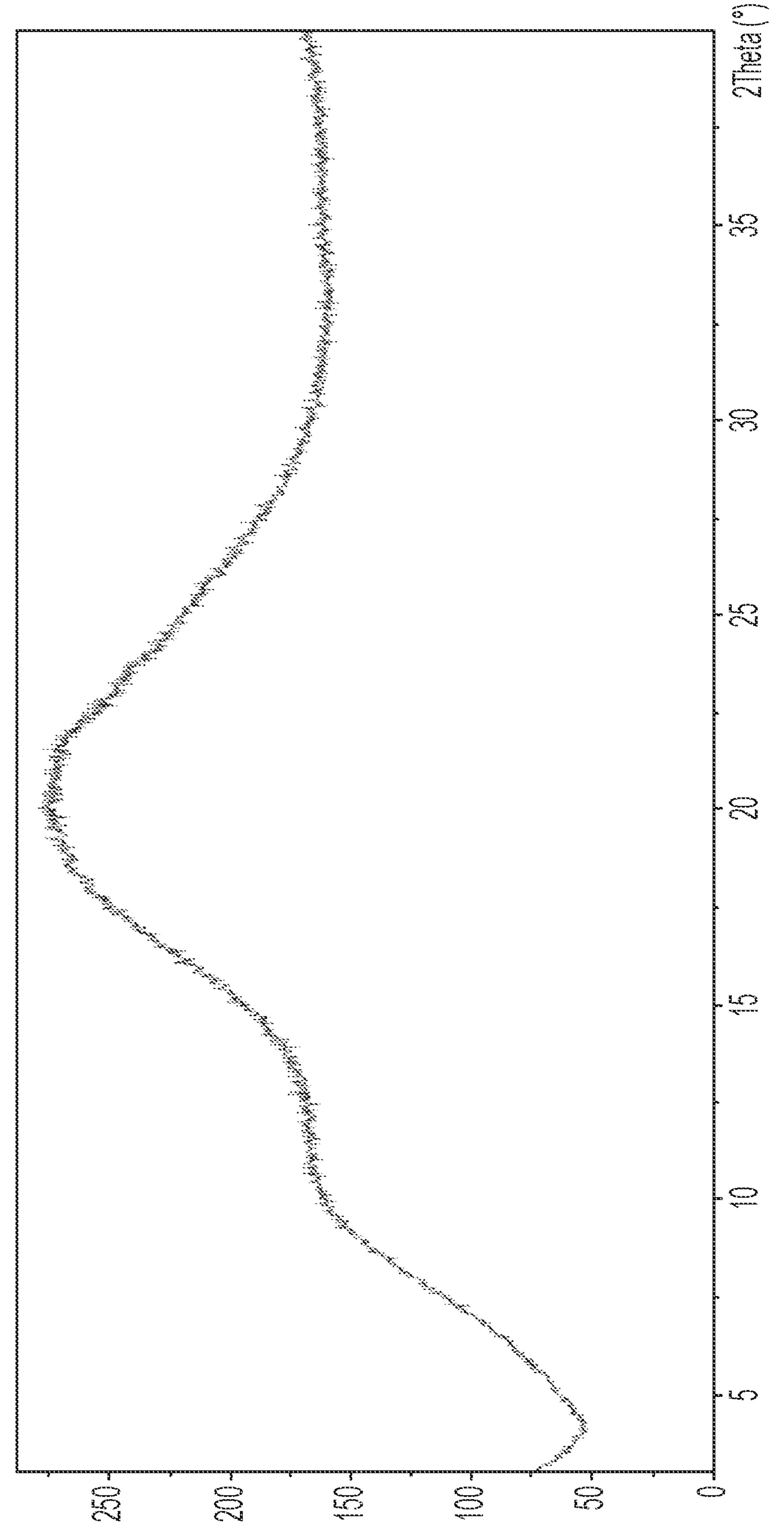


FIG. 11

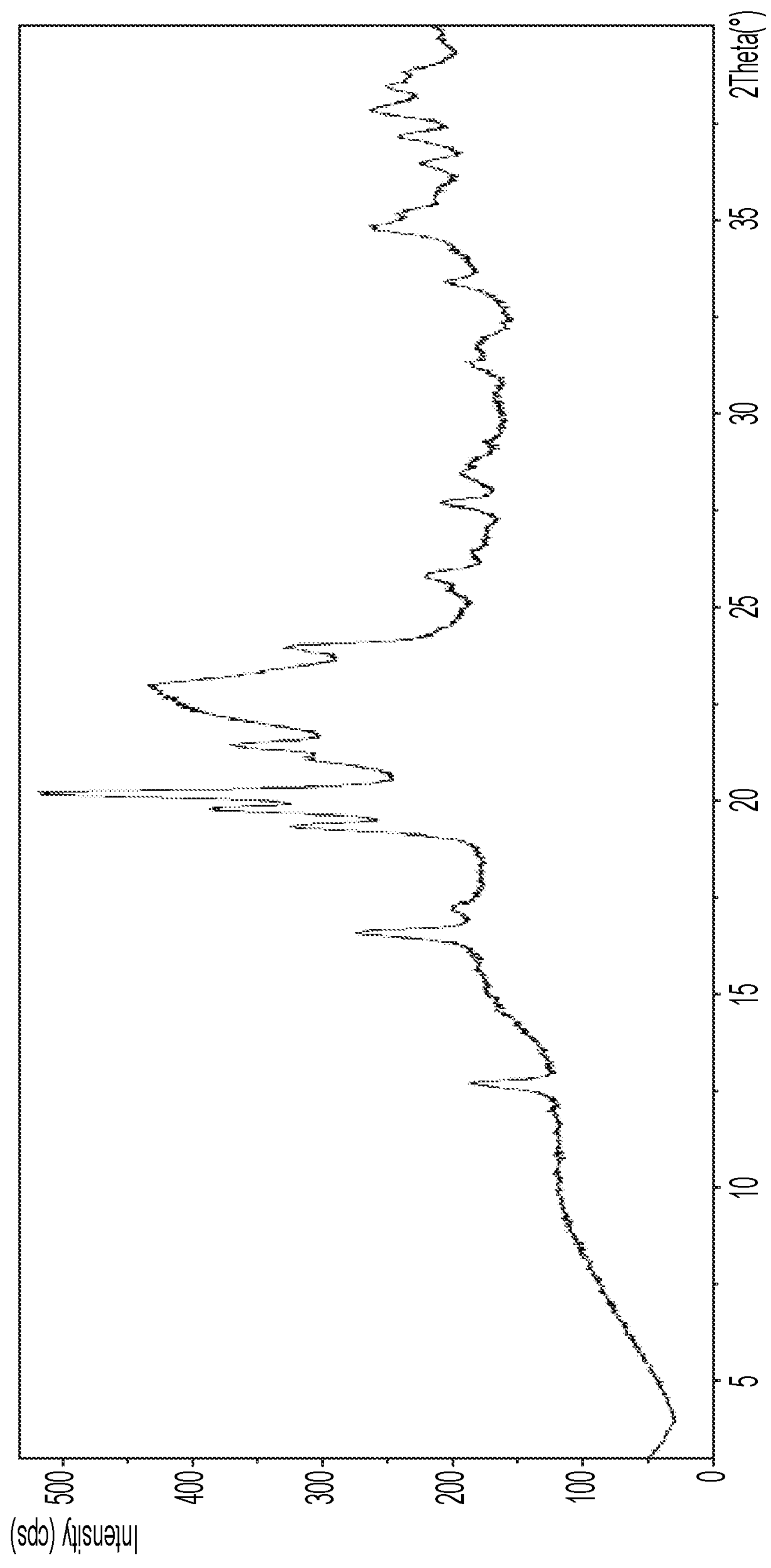


FIG. 12A

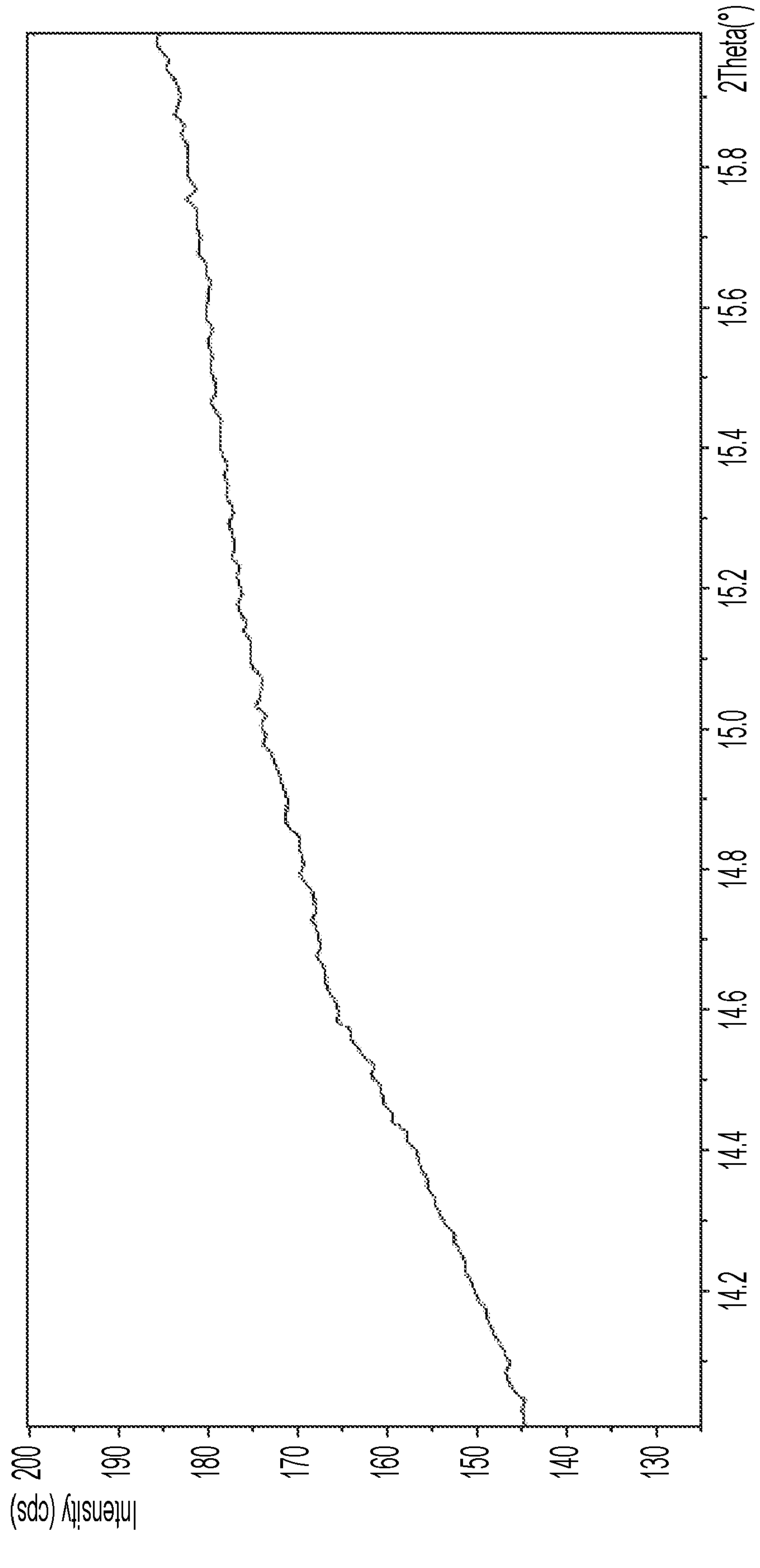
Intensity (cps)
200
190
180
170
160
150
140
130
14.2
14.4
14.6
14.8
15.0
15.2
15.4
15.6
15.8
2Theta(°)

FIG. 12B

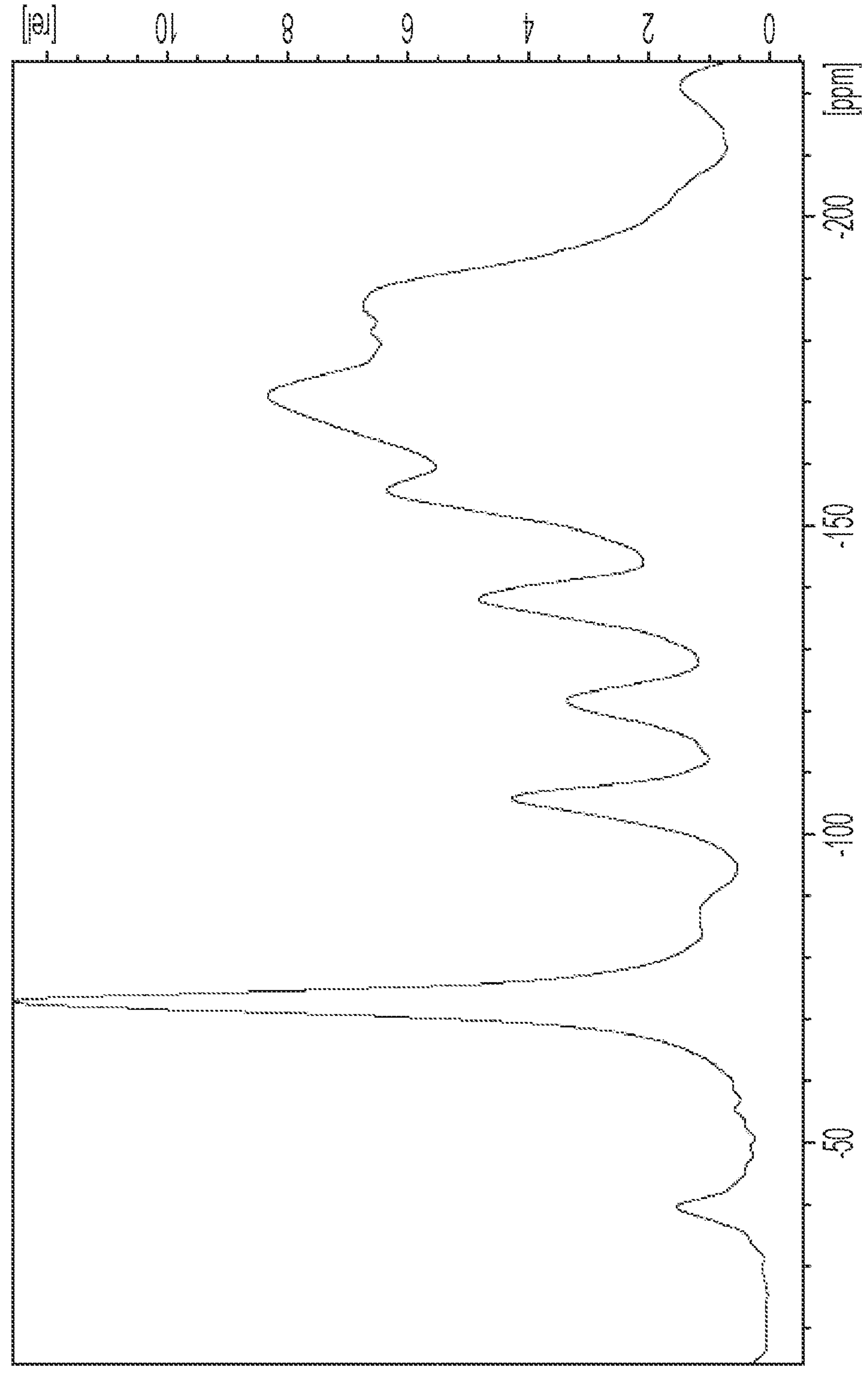


FIG. 13A

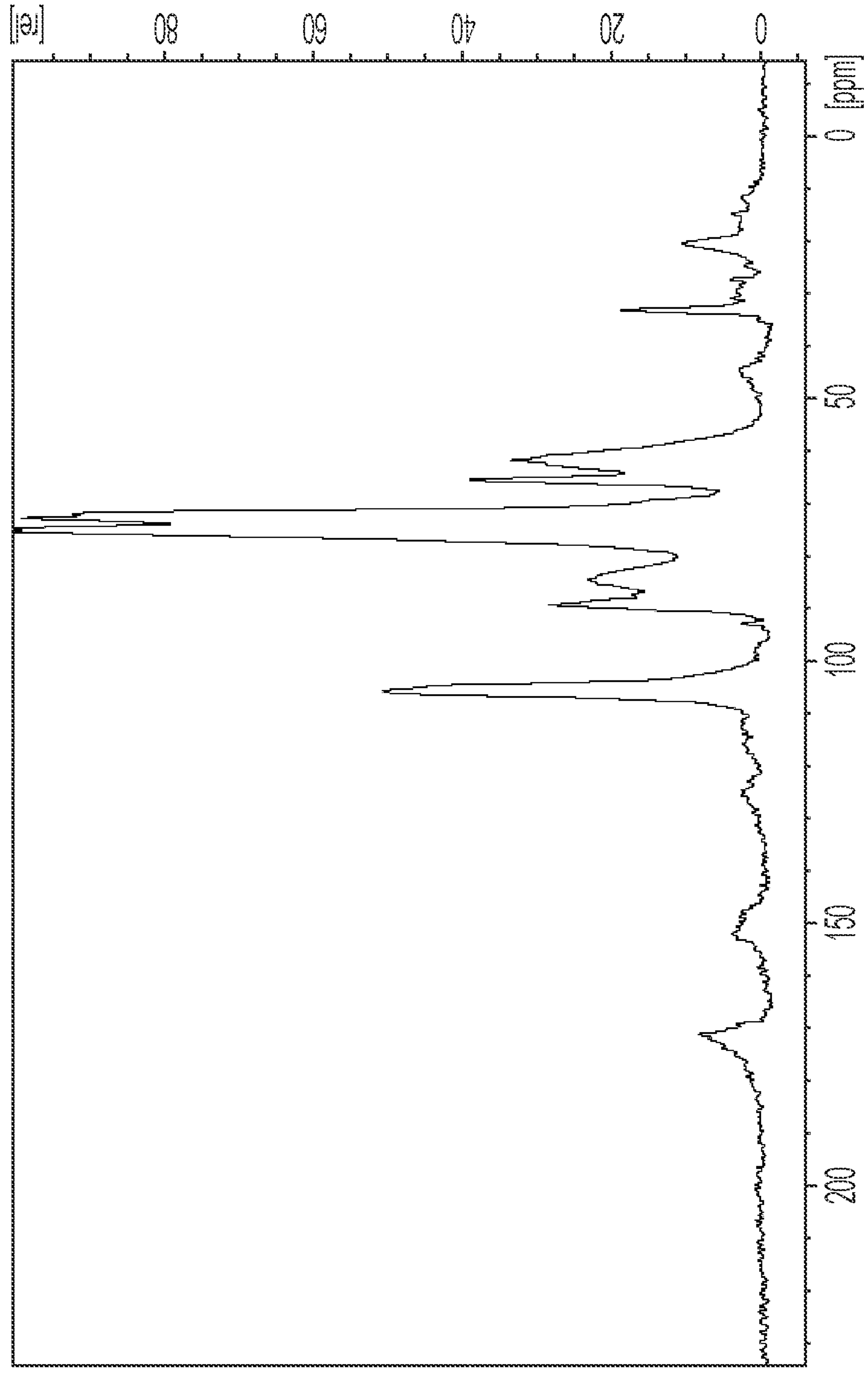


FIG. 13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2019016671 A
- US 20040105820 A
- US 20030144257 A
- WO 2011140425 A
- US 2011306607 A
- WO 2012106499 A
- US 2012196869 A
- WO 2012112743 A
- US 2012245136 A
- WO 2012125613 A
- US 2012264749 A
- WO 2012116440 A
- US 2014187533 A
- WO 2011026240 A
- US 2012220605 A
- US 8883840 B
- US 8466188 B
- WO 2013109521 A
- US 2015005304 A
- WO 2020092667 A
- US 2020140411 A
- CN 111217776
- WO 2008135826 A
- US 2009048306 A
- WO 2006011050 A
- US 2008312235 A
- WO 2013061205 A
- US 2014296313 A
- US 20130303535 A
- WO 2013131018 A
- WO 2013114250 A
- US 2013274243 A
- WO 2014120808 A
- US 2014213616 A
- WO 2014120815 A
- US 2014228371 A
- WO 2014120820 A
- US 2014221435 A
- WO 2015010065 A
- US 20160152561 A
- WO 2015089361 A
- US 20150166589 A
- WO 2019014352 A
- US 20190016671 A
- WO 2018213426 A
- WO 2020146682 A
- WO 2020146612 A
- WO 2020014243 A
- WO 2020014246 A
- WO 2020092187 A
- WO 2020140959 A
- WO 2020151728 A
- WO 2020261114 A
- WO 2021032074 A
- CN 112390745
- CN 111808019
- CN 112225695
- CN 112457294
- CN 112300051
- CN 112300069
- CN 112441969
- CN 112479996
- WO 2010129864 A
- WO 2015157559 A
- WO 2017059385 A
- WO 2018183781 A
- WO 2018183782 A
- WO 2020072835 A
- WO 2002036297 A
- WO 2022036297 A
- WO 2021257490 A
- WO 2021257420 A
- WO 2021257418 A
- WO 2022037641 A
- WO 2022037647 A
- WO 2021113627 A
- WO 2013086229 A
- WO 2013134518 A
- WO 2014211173 A
- WO 2014201206 A
- WO 2016141035 A
- WO 2021252818 A
- WO 2021252822 A
- WO 2021252820 A
- US 6099562 A
- US 5886026 A
- US 5304121 A
- WO 2002008748 A3

## Non-patent literature cited in the description


- **DIELEMAN, J.P. et al.** Incidence rates and treatment of neuropathic pain conditions in the general population.. *Pain*, 2008, vol. 137 (3), 681-8
- *Clin. Ther.*, 2018, vol. 40 (6), 828-49
- **RUSH, A.M.** ; **T.R. CUMMINS**. Painful Research: Identification of a Small-Molecule Inhibitor that Selectively Targets NaV1.8 Sodium Channels.. *Mol. Interv.*, 2007, vol. 7 (4), 192-5
- **ENGLAND, S.** Voltage-gated sodium channels: the search for subtype-selective analgesics.. *Expert Opin. Investig. Drugs*, 2008, vol. 17 (12), 1849-64
- **KRAFTE, D. S.** ; **BANNON, A. W.** Sodium channels and nociception: recent concepts and therapeutic opportunities.. *Curr. Opin. Pharmacol.*, 2008, vol. 8 (1), 50-56
- **CHAHINE, M.** ; **CHATELIER, A.** ; **BABICH, O.** ; **KRUPP, J. J.** Voltage-gated sodium channels in neurological disorders.. *CNS Neurol. Disord. Drug Targets*, 2008, vol. 7 (2), 144-58
- **SODERPALM, B.** Anticonvulsants: aspects of their mechanisms of action.. *Eur. J. Pain*, 2002, vol. 6, 3-9
- **WANG, G. K.** ; **MITCHELL, J.** ; **WANG, S. Y.** Block of persistent late Na+ currents by antidepressant sertraline and paroxetine.. *J. Membr. Biol.*, 2008, vol. 222 (2), 79-90
- **CATTERALL, W. A.** ; **GOLDIN, A. L.** ; **WAXMAN, S. G.** International Union of Pharmacology. XLVII. Nomenclature and structure-function relationships of voltage-gated sodium channels.. *Pharmacol. Rev.*, 2005, vol. 57 (4), 397
- **AKOPIAN, A.N.** ; **L. SIVILOTTI** ; **J.N. WOOD**. A tetrodotoxin-resistant voltage-gated sodium channel expressed by sensory neurons.. *Nature*, 1996, vol. 379 (6562), 257-62
- **BLAIR, N.T.** ; **B.P. BEAN**. Roles of tetrodotoxin (TTX)-sensitive Na+ current, TTX-resistant Na+ current, and Ca2+ current in the action potentials of nociceptive sensory neurons.. *J. Neurosci.*, 2002, vol. 22 (23), 10277-90
- **ROZA, C. et al.** The tetrodotoxin-resistant Na+ channel NaV1.8 is essential for the expression of spontaneous activity in damaged sensory axons of mice.. *J. Physiol.*, 2003, vol. 550 (3), 921-6
- **JARVIS, M.F. et al.** A-803467, a potent and selective NaV1.8 sodium channel blocker, attenuates neuropathic and inflammatory pain in the rat.. *Proc. Natl. Acad. Sci. U S A*, 2007, vol. 104 (20), 8520-5
- **JOSHI, S.K. et al.** Involvement of the TTX-resistant sodium channel NaV1.8 in inflammatory and neuropathic, but not post-operative, pain states.. *Pain*, 2006, vol. 123 (1-2), 75-82
- **LAI, J. et al.** Inhibition of neuropathic pain by decreased expression of the tetrodotoxin-resistant sodium channel, NaV1.8.. *Pain*, 2002, vol. 95 (1-2), 143-52
- **DONG, X.W. et al.** Small interfering RNA-mediated selective knockdown of Na(V) 1.8 tetrodotoxin-resistant sodium channel reverses mechanical allodynia in neuropathic rats.. *Neuroscience*, 2007, vol. 146 (2), 812-21
- **HUANG, H.L. et al.** Proteomic profiling of neuromas reveals alterations in protein composition and local protein synthesis in hyper-excitable nerves.. *Mol. Pain*, 2008, vol. 4, 33
- **BLACK, J.A. et al.** Multiple sodium channel isoforms and mitogen-activated protein kinases are present in painful human neuromas.. *Ann. Neurol.*, 2008, vol. 64 (6), 644-53
- **COWARD, K. et al.** Immunolocalization of SNS/PN3 and NaN/SNS2 sodium channels in human pain states.. *Pain*, 2000, vol. 85 (1-2), 41-50
- **YIANGOU, Y. et al.** SNS/PN3 and SNS2/NaN sodium channel-like immunoreactivity in human adult and neonate injured sensory nerves.. *FEBS Lett.*, 2000, vol. 467 (2-3), 249-52
- **RUANGSRI, S. et al.** Relationship of axonal voltage-gated sodium channel 1.8 (NaV1.8) mRNA accumulation to sciatic nerve injury-induced painful neuropathy in rats.. *J. Biol. Chem.*, vol. 286 (46), 39836-47
- **CHOI, J.S.** ; **S.G. WAXMAN**. Physiological interactions between NaV1.7 and NaV1.8 sodium channels: a computer simulation study.. *J. Neurophysiol.*, vol. 106 (6), 3173-84
- **RENGANATHAN, M.** ; **T.R. CUMMINS** ; **S.G. WAXMAN**. Contribution of Na(V)1.8 sodium channels to action potential electrogenesis in DRG neurons.. *J. Neurophysiol.*, 2001, vol. 86 (2), 629-40
- **RUSH, A.M. et al.** A single sodium channel mutation produces hyper- or hypoexcitability in different types of neurons.. *Proc. Natl. Acad. Sci. USA*, 2006, vol. 103 (21), 8245-50
- **SUN, W. et al.** Reduced conduction failure of the main axon of polymodal nociceptive C-fibers contributes to painful diabetic neuropathy in rats.. *Brain*, vol. 135 (2), 359-75
- **STRICKLAND, I.T. et al.** Changes in the expression of NaV1.7, NaV1.8 and NaV1.9 in a distinct population of dorsal root ganglia innervating the rat knee joint in a model of chronic inflammatory joint pain.. *Eur. J. Pain*, 2008, vol. 12 (5), 564-72
- **QIU, F. et al.** Increased expression of tetrodotoxin-resistant sodium channels NaV1.8 and NaV1.9 within dorsal root ganglia in a rat model of bone cancer pain.. *Neurosci. Lett.*, vol. 512 (2), 61-6
- **JARVIS et al.** *Proc. Nat. Acad. Sci.*, 2007, vol. 104 (20), 8520

- Periodic Table of the Elements, CAS version. Handbook of Chemistry and Physics
- **THOMAS SORRELL**. Organic Chemistry. University Science Books, 1999
- **SMITH, M.B.** ; **MARCH, J.** March's Advanced Organic Chemistry. John Wiley & Sons, 2001
- **V.S. SHIRLEY** ; **C.M. LEDERER**. Isotopes Project, Nuclear Science Division, Lawrence Berkeley Laboratory. *Table of Nuclides*, January 1980
- **S. M. BERGE et al.** *J. Pharmaceutical Sciences*, 1977, vol. 66, 1-19
- **E. W. MARTIN**. Remington's Pharmaceutical Sciences. Mack Publishing Co., 1980
- Perry's Chemical Engineering Handbook. McGraw-Hill book co., 1984
- **MARSHALL**. Atomization and Spray-Drying. *Chem. Eng. Prog. Monogr. Series 2*, 1954
- The Merck Manual. Merck & Co., Inc., 2011
- *Food and Drug Administration*, www.fda.gov.
- **T.G.M. WUTS et al.** Greene's Protective Groups in Organic Synthesis. 2006
- **C.-J. HUANG et al.** Characterization of voltage-gated sodium channel blockers by electrical stimulation and fluorescence detection of membrane potential. *24 Nature Biotech.*, 2006, 439-46
- **J. A. FORNWALD et al.** Gene Expression in Mammalian Cells Using BacMam, a Modified Baculovirus System. *1350 Methods in Molecular Biology*, 2016, 95-116
- *CHEMICAL ABSTRACTS*, 169211-44-3
- **GONZALEZ, J.E.** ; **TSIEN, R.Y.** Voltage Sensing by Fluorescence Resonance Energy Transfer in Single Cells. *Biophys. J.*, 1995, vol. 69, 1272-1280
- *CHEMICAL ABSTRACTS*, 393782-57-5
- **GONZALEZ, J.E.** ; **TSIEN, R.Y.** Improved indicators of cell membrane potential that use fluorescence resonance energy transfer. *Chem. Biol.*, 1997, vol. 4, 269-277